(19) Europäisches Patentamt
European Patent Office
Office européen des brevets

(11) Numéro de publication: **0 487 408 B1**

(12) **FASCICULE DE BREVET EUROPEEN**

(45) Date de publication de fascicule du brevet: **21.06.95**

(51) Int. Cl.[6]: **C07D 498/04**, C07D 213/74, A61K 31/435, //(C07D498/04, 263:00,221:00)

(21) Numéro de dépôt: **91403116.6**

(22) Date de dépôt: **20.11.91**

(54) **Dérivés d'oxazolopyridines leurs procédés de préparation et les compositions pharmaceutiques qui les contiennent.**

(30) Priorité: **20.11.90 FR 9014381**

(43) Date de publication de la demande: **27.05.92 Bulletin 92/22**

(45) Mention de la délivrance du brevet: **21.06.95 Bulletin 95/25**

(84) Etats contractants désignés: **AT BE CH DE DK ES FR GB GR IT LI LU NL SE**

(56) Documents cités:
**EP-A- 0 232 740**
**EP-A- 0 412 899**

**EUROPEAN JOURNAL OF MEDICINAL CHEMISTRY.CHIMICA THERAPEUTICA. vol. 17, no. 1,Janvier 1982, CHATENAY-MALABRY FR pages 85 - 88; M. DEBAERT et al.: "Pharmacomodulation du modèle benzoxazoline par la structure aryl-pipérazinique"**

(73) Titulaire: **ADIR ET COMPAGNIE**
**1 rue Carle Hébert**
**F-92415 Courbevoie Cédex (FR)**

(72) Inventeur: **Guillaumet, Gérald**
**2 rue Auguste Renoir**
**F-45100 Orleans (FR)**
Inventeur: **Flouzat, Christine**
**9 rue Barrière de Jaude**
**F-63000 Clermont Ferrand (FR)**
Inventeur: **Devissaguet, Michelle**
**14 boulevard d'Inkermann**
**F-92200 Neuilly sur Seine (FR)**
Inventeur: **Renard, Pierre**
**50 avenue de Villeneuve l'Etang**
**F-78000 Versailles (FR)**
Inventeur: **Caignard, Daniel Henri**
**69 bis rue Brancion**
**F-75015 Paris (FR)**
Inventeur: **Adam, Gérard**
**9 clos du Mesnil,**
**Route du Pecq**
**F-78600 Le Mesnil le Roi (FR)**

EP 0 487 408 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

| | |
|---|---|
| HELVETICA CHIMICA ACTA, vol. 59, no. 5, 1976, BASEL CH pages 1593-1612; R. RÜFE-NACHT et al : "Aza-analogie II: Derivate vo-nOxazolo[4,5-b]pyridin-2(3H)-on, einem Aza-Analogen von Benzoxazol-2(3H)-on" | |

## Description

La présente invention concerne de nouveaux composés d'oxazolo[5,4-b] pyridines, leurs procédés de préparation et les compositions pharmaceutiques qui les contiennent.

On connait déjà les propriétés à la fois analgésiques et antiinflammatoires des phényl-2 oxazolo-[5,4] ou [4,5] pyridines (Brevets US 4038396, FR 2328471, FR 2319354, GB 1421619, US 232740).

EP-A-0232 740 décrit des composés utiles dans le traitement de la thrombose, des inflammations, des allergies, ou comme analgésiques.

Toutefois, ces produits possèdent un profil essentiellement antiinflammatoire comme le confirment les indications thérapeutiques mentionnées dans les brevets cités ci-dessus ou bien présentent l'inconvénient de ne pas dissocier les deux types d'activités : analgésique d'une part, anti-pyrétique et antiinflammatoire d'autre part.

Les nouveaux composés découverts par la demanderesse possèdent non seulement un niveau d'activité analgésique au moins équivalent à celui des phényl-2 [3H] oxazolo [4,5-b] ou [5,4-b] pyridines déjà connus mais présentent également la caractéristique très intéressante d'être pratiquement dépourvus d'effets antiinflammatoires.

La plupart des substances analgésiques non morphiniques connues à ce jour possèdent également une activité antiinflammatoire et interviennent donc sur les processus liés aux phénomènes d'inflammation (c'est le cas par exemple des dérivés salicyclés comme l'aspirine, les pyrazolés comme la phényl butazone, les acides arylacétiques ou hétéroarylacétiques comme l'indométhacine...). Etant antiinflammatoires, ces substances inhibent la cyclooxygénase ce qui provoque un bloquage de la biosynthèse de nombreux médiateurs chimiques (prostaglandines, prostacycline, thromboxane A2...). Il s'ensuit donc de multiples effets secondaires parmi lesquels l'inhibition de l'agrégation plaquettaire associée à des troubles de la coagulation et une toxicité gastro intestinale avec possibilité d'ulcérations et d'hémorragie due à une diminution de la biosynthèse des prostaglandines PG $E_2$ et PC $F_1$ $\alpha$ qui sont cytoprotectrices de la muqueuse gastrique.

Outre les désagrements qu'ils occasionnent, ces effets secondaires peuvent, chez de nombreux sujets qui y sont particulièrement sensibles, rendre impossible la prescription de substances dotées de propriétés antiinflammatoires.

Les composés de la présente invention n'intervenant pas sur les médiateurs de l'inflammation sont donc dépourvus des effets secondaires mentionnés précedemment.

Cette caractéristique associée à leur absence de toxicité et à leur haut niveau d'activité rend les composés de la présente invention utilisables en tant qu'analgésiques sans les restrictions d'usage habituellement en vigueur pour la majorité des produits de cette classe.

Plus spécifiquement l'invention concerne les composés de formule générale (I) :

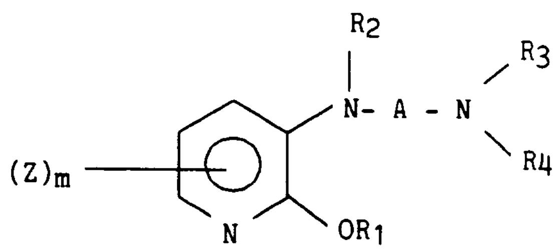

(I)

dans laquelle :

- $R_1$ et $R_2$ représentent chacun un atome d'hydrogène ou forment avec l'oxygène et l'azote qui les portent un enchainement - O - CO - N - ce qui correspond aux oxazolo [5,4 b] pyridin-2-ones,
- Z représente un atome d'halogène, un groupement alkyle inférieur linéaire ou ramifié comprenant de 1 à 6 atomes de carbone, un groupement alcoxy inférieur linéaire ou ramifié comprenant de 1 à 6 atomes de carbone, un groupement trifluorométhyle,
- m nombre entier peut prendre les valeurs 0, 1, 2, 3,
- A est un radical alkyl linéaire ou ramifié comprenant de 1 à 6 atomes de carbone,

  $R_3$ et $R_4$ identiques ou différents représentent :

  - un atome d'hydrogène,
  - un groupement alkyle inférieur linéaire ou ramifié, comprenant de 1 à 6 atomes de carbone,
  - un groupement alkényle inférieur linéaire ou ramifié, comprenant de 1 à 6 atomes de carbone,
  - un groupement aryle ou hétéroaryle éventuellement substitué,

- un groupement arylalkyle ou hétéroarylalkyle éventuellement substitué dont la chaine alkyle comprend de 1 à 3 atomes de carbone,
- un groupement cycloalkyle mono ou bicyclique de 3 à 10 atomes de carbone ,

ou bien :

$R_3$ et $R_4$ constituent avec l'atome d'azote auquel ils sont liés un système hétérocyclique azoté, mono ou bicyclique, saturé ou insaturé, comprenant un maximum de 12 atomes non compris les atomes d'hydrogène parmi lesquels on peut compter de un à trois hétéroatomes choisis parmi azote, oxygène ou soufre et éventuellement substitué par un ou des :

- groupement hydroxy,
- groupement oxo,
- groupement alkyle inférieur, linéaire ou ramifié, comprenant de 1 à 6 atomes de carbone,
- groupement aryle éventuellement substitué,
- groupement arylalkyle éventuellement substitué ou diarylalkyle éventuellement substitué dont la chaine alkyle comporte de 1 à 3 atomes de carbone,
- groupement -CO - $OR_5$
- groupement

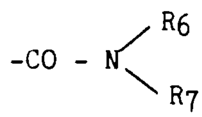

$R_5$ représente :

- un atome d'hydrogène,
- un groupement alkyle inférieur linéaire ou ramifié comprenant de 1 à 6 atomes de carbone,
- un groupement aryle éventuellement substitué,
- un groupement aralkyle éventuellement substitué dont la chaine alkyle comprend de 1 à 3 atomes de carbone,

$R_6$ et $R_7$ identiques ou différents ont la même signification que $R_5$,

- leurs isomères, épimères, diastéréoisomères,
- leurs sels d'addition à un acide pharmaceutiquement acceptable et/ou lorsque $R_1$ et $R_2$ représentent chacun un atome d'hydrogène, leurs sels d'addition à une base pharmaceutiquement acceptable,
- par groupement aryle on entend un groupement mono ou bicyclique insaturé ou aromatique comprenant de 5 à 12 atomes de carbone,
- par groupement hétéroaryle on entend un groupement mono ou bicyclique, insaturé ou aromatique, comprenant de 5 à 12 atomes non compris les atomes d'hydrogène et intégrant dans son squelette carboné un, deux ou trois hétéroatomes choisis parmi azote, oxygène ou soufre,
- le terme substitué associé aux expressions aryle, arylalkyle, diarylalkyle, hétéroaryle, hétéroarylalkyle signifie que le, ou les, noyaux aryle ou hétéroaryle peuvent être substitués par un ou plusieurs groupement alkyle inférieur de 1 à 6 atomes de carbone linéaire ou ramifié, alcoxy inférieur de 1 à 6 atomes de carbone linéaire ou ramifié, hydroxy, nitro, halogène, trifluorométhyle.

L'invention s'étend également au procédé d'obtention des composés de formule générale (I) caractérisé en ce que l'on fait réagir une 3-amino 2-pyridinone de formule générale (II) :

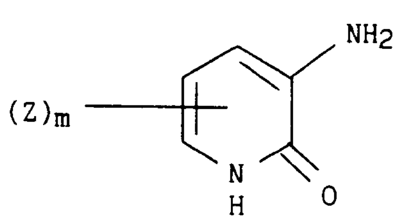

(II)

dans laquelle Z et m ont la même signification que dans les composés de formule générale (I), en solution à -78° C (température du mélange acétone carboglace) dans un solvant ou un mélange de solvants aprotiques avec du bis (trichlorométhyl) carbonate (triphosgène) en présence d'une amine basique comme par exemple la triéthylamine de manière à obtenir une [1H] oxazolo [5,4-b] pyridin-2-one de formule

générale (III) :

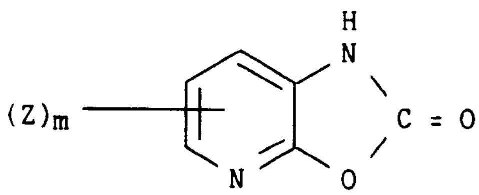

(III)

dans laquelle Z et m ont la même signification que dans les composés de formule générale (I) que l'on fait réagir avec un alcoolate ou un hydrure de métal alcalin en milieu organique aprotique de manière à obtenir le composé de formule générale (IV) :

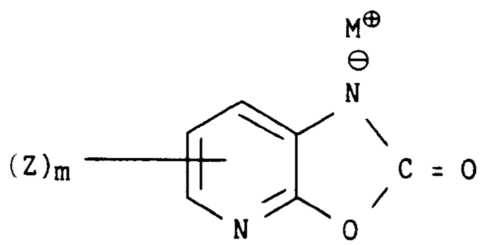

(IV)

dans laquelle Z et m ont la même signification que dans les composés de formule générale (I) et M représente un métal alcalin, que l'on fait réagir en milieu organique et à une température comprise entre la température ambiante et le reflux du solvant (ou mélange de solvants) choisi :

a) soit avec un composé électrophile (préférentiellement en excès) de formule générale (V) :

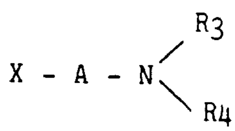

(V)

dans laquelle X représente un atome d'halogène et A, $R_3$, $R_4$ ont la même signification que dans les composés de formule générale (I) de manière à obtenir après refroidissement extraction et éventuelle purification, les composés de formule générale ($I_A$) :

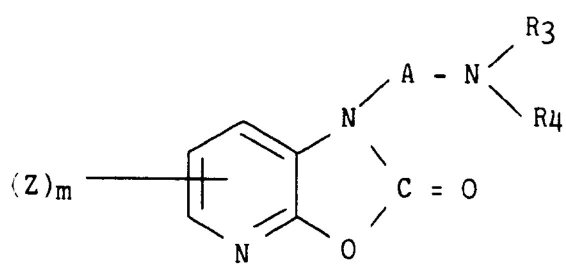

($I_A$)

cas particulier des composés de formule générale (I) pour lesquels $R_1$ et $R_2$ forment avec l'oxygène et l'azote qui les portent un chainon

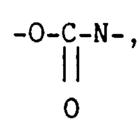

Z, m, A, $R_3$, $R_4$ ayant la même signification que dans les dérivés de formule générale (I),

b) soit avec un composé électrophile (préférentiellement en excès) de formule générale (VI) :

X - A - X'     (VI)

dans laquelle X et X' identiques ou différents représentent chacun un atome d'halogène de manière à obtenir le composé halogéné de formule générale (VII) :

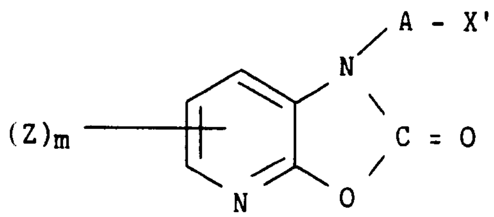

(VII)

dans laquelle X', A, Z et m ont la même signification que précedemment que l'on fait finalement réagir avec une amine (préférentiellement en excès) de formule générale (VIII) :

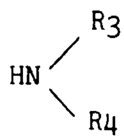

(VIII)

dans laquelle $R_3$ et $R_4$ ont la même signification que dans les composés de formule (I) en milieu organique, éventuellement en présence d'une amine basique comme par exemple la diisopropylamine et à une température comprise entre la température ambiante et le reflux du solvant choisi de manière à obtenir après refroidissement, extraction et éventuelle purification les composés de formule générale ($I_A$),
c) soit, dans le cas particulier des composés de formule générale ($I_A$) où A est un chainon méthylène -$CH_2$ -, avec du chlorométhyl phényl sulfure de manière à obtenir les composés de formule générale (IX) :

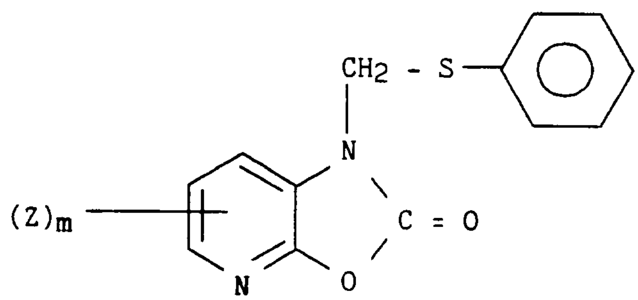

(IX)

dans laquelle Z et m ont la même signification que dans les composés de formule générale (I) que l'on fait ensuite réagir en milieu organique avec du chlorure de sulfuryle pour obtenir après purification le composé chloré de formule générale (X) :

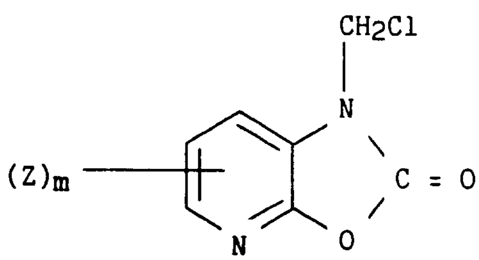

(X)

dans laquelle Z et m ont la même signification que dans les composés de formule générale (I) que l'on fait ensuite réagir comme précédemment avec des composés de formule générale (VIII) de manière à obtenir les composés de formule générale ($I_A$) avec A étant un chainon méthylène - $CH_2$ -.

Les composés de formule générale ($I_A$) pour lesquels A est un chainon méthylène - $CH_2$ - peuvent également être obtenus en une seule étape par condensation en milieu d'alcool aliphatique inférieur d'un composé de formule générale (III), d'une amine de formule générale (VIII) en léger excès et d'un excès de formol à une température comprise entre la température ambiante et le reflux du milieu réactionnel suivie après refroidissement et isolement d'une éventuelle purification par chromatographie sur colonne de silice.

Les composés ($I_A$) obtenus par les méthodes évoquées précédemment peuvent si on le désire être séparés en leurs isomères et/ou être salifiés par un acide pharmaceutiquement acceptable.

Les composés ($I_A$) peuvent également si on le désire être traités par un agent alcalin comme par exemple l'hydroxyde de sodium en solution aqueuse à une température comprise entre la température ambiante et la température de reflux du milieu réactionnel pour conduire après éventuelle acidification et/ou neutralisation du milieu réactionnel aux composés de formule générale ($I_B$) :

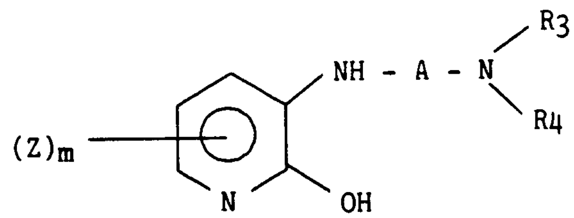

($I_B$)

cas particulier des composés de formule générale (I) pour lesquels $R_1 = R_2 =$ H, A, $R_3$, $R_4$, Z et m ayant la même signification que dans les dérivés de formule générale (I).

L'étude pharmacologique des composés de l'invention a montré qu'ils sont peu toxiques, doués d'une haute activité analgésique pure et donc dépourvus des inconvénients, inhérents à la composante antiinflammatoire des composés non morphiniques présentant ce type d'activité (action ulcérigène, perturbation des processus de coagulation...)

Cette activité analgésique pure rend les composés de la présente invention très intéressants dans nombres d'indications telles que : algies rhumatismales, névralgies lombosciatiques, névralgies cervico-brachiales, algies traumatiques telles que entorses, fractures, luxations, douleurs post-traumatiques, douleurs post-opératoires, douleurs dentaires, douleurs neurologiques telles que névralgies faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant les produits de formule (I) ou un de leurs sels d'addition à un acide pharmaceutiquement acceptable seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire et notamment les préparations injectables, les aérosols, les gouttes occulaires ou nasales, les comprimés simples ou dragéifiés, les comprimés sublinguaux, les sachets, les paquets, les gélules, les glossettes, les tablettes, les suppositoires, les crèmes, les pommades, gels dermiques etc...

La posologie utile varie selon l'âge et le poids du patient, la voie d'administration, la nature de l'indication thérapeutique et des traitements éventuellement associés et se situe entre 10 mg et 4 grammes par 24 heures.

Les exemples suivants illustrent l'invention.

**EXEMPLE 1 : 1-[2-(4-PHENYL PIPERAZIN-1-YL) ETHYL] [1H] OXAZOLO [5,4-b] PYRIDIN-2-ONE**

**STADE A** : [1H] OXAZOLO [5,4-b] PYRIDIN-2-ONE

Refroidir à - 78° C (température du mélange acétone/carboglace) sous atmosphère inerte (argon) une solution de 0,33 g (3 mmoles) de 3-amino 2-hydroxy pyridine dans un mélange 50/50 de dichlorométhane et de tétrahydrofurane anhydre. Ajouter 2,2 $cm^3$ (15 mmoles) de triéthylamine puis couler goutte à goutte 0,98 g (3,3 mmoles) de bis (trichlorométhyl) carbonate (Triphosgène) en solution dans 10 $cm^3$ d'un mélange

50/50 de dichlorométhane et de tétrahydrofurane.

Après 30 minutes d'agitation, rajouter 2,2 $cm^3$ (15 mmoles) de triéthylamine et maintenir l'agitation pendant six heures.

Après évaporation du solvant sous pression réduite, les cristaux obtenus sont purifiés par flash chromatographie sur colonne de silice (Eluant : éther éthylique/tétrahydrofurane 50/50).

On obtient finalement la [1H] oxazolo [5,4-b] pyridin-2-one avec un rendement de 78 %.

Point de fusion : 252° C

IR (pastille KBr) 3220 $cm^{-1}$ (faible) ν NH

2900 - 3000 $cm^{-1}$ ν CH

1775 $cm^{-1}$ ν C = 0

RMN $^1H$ ($CDCl_3$, TMS) :

δ :11,8 ppm, 1H, singulet NH

δ :7,92 ppm, 1H, doublet dédoublé (J = 5 Hz et J = 1,5 Hz) H (5)

δ :7,46 ppm, 1H, doublet dédoublé (J = 8 Hz et J = 1,5 Hz) H (7)

δ :7,17 ppm, 1H, doublet dédoublé (J = 8 Hz et J = 5 Hz) H (6)

METHODE A

**STADE B** : 1-(2-BROMO ETHYL) [1H] OXAZOLO [5,4-B] PYRIDIN-2-ONE

Dissoudre sous atmosphère inerte (argon) 1,36 g (10 mmoles) de [1H] oxazolo [5,4-b] pyridin-2-one dans 80 $cm^3$ de diméthyl formamide. Ajouter ensuite à température ambiante et par petites portions 15 mmoles d'hydrure de sodium préalablement lavé par du tétrahydrofurane.

Chauffer à 50° C pendant 40 minutes puis refroidir à température ambiante et ajouter 5,17 $cm^3$ (60 mmoles) de dibromoéthane dilué dans 20 $cm^3$ de diméthylformamide.

Chauffer à 110° C pendant une heure puis éliminer la diméthyl formamide par distillation sous pression réduite.

Reprendre le résidu par de l'eau, extraire la phase aqueuse par du chlorure de méthylène, sécher les phases organiques sur sulfate de magnésium puis mettre à sec et purifier le produit brut par flash chromatographie sur colonne de silice (silice 230 - 240 Mesh, éluant acétonitrile/chlorure de méthylène 5/95)

On obtient finalement la 1-(2-bromoéthyl) [1H] oxazolo [5,4-b] pyridin-2-one avec un rendement de 66 %.

Point de fusion : 109 - 110° C

IR (pastille KBr) 2900 - 3000 $cm^{-1}$ ν CH

1760 $cm^{-1}$ ν C = 0

RMN $^1H$ ($CDCl_3$, TMS) :

δ :8,7 ppm, 1H, doublet dédoublé (J = 5,1 Hz et J = 1,5 Hz) H (5)

δ :7,37 ppm, 1H, doublet dédoublé (J = 7,7 Hz et J = 1,5 Hz) H (7)

δ :7,17 ppm, 1H, doublet dédoublé (J = 5,1 Hz et J = 7,7 Hz) H (6)

δ :4,27 ppm, 2H, triplet (J = 6,3 Hz ) $CH_2$ - N

δ :3,7 ppm, 2H, triplet (J = 6,3 Hz) $CH_2$

**STADE C** : 1-[2-(4-PHENYL PIPERAZIN-1-YL)ETHYL] [1H] OXAZOLO [5,4-b] PYRIDIN-2-ONE

Dans un ballon sous atmosphère d'argon surmonté d'une réfrigérant additionner 2,43 g (15 mmoles) de 1-phényl pipérazine puis 2,61 $cm^3$ (15 mmoles) de diisopropyléthylamine à une solution de 2,43 g (10 mmoles) de 1-(2-bromoéthyl) [1H] oxazolo [5,4-b] pyridin-2-one dans l'acétonitrile.

Porter à 80° C pendant 12 heures puis laisser refroidir et évaporer l'acétonitrile sous pression réduite.

Reprendre le résidu dans de l'eau, vérifier l'alcalinité du milieu et extraire la phase aqueuse au chlorure de méthylène, sécher les phases chlorométhyléniques sur sulfate de magnésium et mettre à sec.

Le produit brut obtenu est ensuite purifié par chromatographie sur colonne de silice (silice 230 - 240 Mesh, éluant chlorure de méthylène/méthanol 95/5)

On obtient finalement la 1-[2-(4-phenyl piperazin-1-yl)éthyl] [1H] oxazolo [5,4-b] pyridin-2-one avec un rendement de 90 %.

Point de fusion : 182 - 183° C

IR (pastille KBr) 2900 - 3100 $cm^{-1}$ ν CH

1760 $cm^{-1}$ ν C = 0

RMN $^1H$ ($CDCl_3$, TMS) :
δ :8,03 ppm, 1H, doublet dédoublé (J = 5 Hz et J = 1,3 Hz) H (5)
δ :7,3 ppm, 1H, doublet dédoublé (J = 7,6 Hz et J = 1,3 Hz) H (7)
δ :7,25 ppm, 2H, doublet H aromatique
δ :7,13 ppm, 1H, doublet dédoublé (J = 5 Hz et J = 7,6 Hz) H (6)
δ :4 ppm, 2H, triplet (J = 6 Hz) $CH_2$ - N
δ :3,15 ppm, 4H, multiplet H pipérazinique
δ :2,77 ppm, 2H, triplet (J = 6 Hz) $CH_2$
δ :2,68 ppm, 4H, multiplet H pipérazinique

METHODE B

**STADE B** : 4-PHENYL 1-(2-CHLORO ETHYL) PIPERAZINE

Dissoudre sous argon 6,49 g (40 mmoles) de 1-phényl pipérazine dans 40 $cm^3$ de diméthyl formamide, ajouter 6,63 g (48 mmoles) de carbonate de potassium anhydre puis 6,88 g (48 mmoles) de 1-bromo 2-chloro éthane.

Agiter à température ambiante pendant 22 heures puis éliminer l'insoluble minéral par filtration.

Acidifier le filtrat par de l'éthanol saturé en acide chlorhydrique sec jusqu'à pH de 1.

Ajouter 400 $cm^3$ d'éther éthylique anhydre et isoler par mise à sec le chlorhydrate de 4-phényl 1-(2-chloroéthyl) pipérazine qui précipite dans le milieu.

Reprendre le chlorhydrate dans une solution aqueuse à 10 % de carbonate de sodium puis extraire la phase aqueuse au chlorure de méthylène.

Sécher la phase organique sur sulfate de magnésium, filtrer puis mettre à sec sous pression réduite.

La 4-phényl 1-(2-chloroéthyl) pipérazine obtenue est engagée telle quelle dans l'étape suivante.

**STADE C** : 1-[2-(4-PHENYLPIPERAZIN-1-YL)ETHYL][1H] OXAZOLO [5,4-b] PYRIDIN-2-ONE

Ajouter par petites portions 15 mmoles d'hydrure de sodium préalablement lavé au THF à une solution sous atmosphère d'argon de 1,36 g (10 mmoles) de [1H] oxazolo [5,4-b] pyridin-2-one dans 80 $cm^3$ de diméthyl formamide.

Chauffer à 50° C pendant 40 minutes puis refroidir et ajouter à température ambiante 12 mmoles de 4-phényl 1-(2-chloroéthyl) pipérazine en solution dans 20 $cm^3$ de diméthyl formamide.

Porter à reflux (≃ 153° C) pendant 90 minutes. Reprendre le résidu par de l'eau, extraire la phase aqueuse au chlorure de méthylène , sécher les phases chlorométhyléniques sur sulfate de magnésium et mettre à sec.

Le produit brut obtenu est ensuite purifié sur colonne de silice (silice 230 - 240 Mesh, éluant, chlorure de méthylène/méthanol 95/5)

On obtient la 1-[2-(4-phénylpipérazin-1-yl)éthyl] [1H] oxazolo [5,4-b] pyridin-2-one avec un rendement de 64 %.

**EXEMPLE 2 : 1-[2-(4-(3-TRIFLUOROMETHYL PHENYL)PIPERAZIN-1-YL) ETHYL] [1H] OXAZOLO [5,4-b] PYRIDIN-2-ONE**

Les deux méthodes de synthèse décrites dans l'exemple 1 sont utilisables en remplaçant la 1-phényl pipérazine par la 1-(3-trifluorométhyl phényl) pipérazine.

Point de fusion : 99 - 100° C
IR (pastille KBr) 2700 - 3000 $cm^{-1}$ ν CH
1765 $cm^{-1}$ ν C = 0
RMN $^1H$ ($CDCl_3$, TMS) :
δ :8,03 ppm, 1H, doublet dédoublé (J = 5,4 Hz et J = 1,3 Hz) H (5)
δ :7,3 ppm, 3H, multiplet H (7) + 2H aromatiques
δ :7,14 ppm, 1H, doublet dédoublé (J = 7,3 Hz et J = 5,4 Hz) H (6)
δ :7,0 - 7,08 ppm, 2H, multiplet 2H aromatiques
δ :3,98 ppm, 2H, triplet (J = 6,3 Hz) N - $CH_2$
δ :3,16 ppm, 4H, multiplet 2 $CH_2$ pipéraziniques
δ :2,78 ppm, 2H, triplet (J = 6,3 Hz) $CH_2$
δ :2,67 ppm, 4H, multiplet 2 $CH_2$ pipéraziniques

**EXEMPLE 3 : 1-(2-MORPHOLINO ETHYL) [1H] OXAZOLO [5,4-b] PYRIDIN-2-ONE**

Les deux méthodes de synthèse décrites dans l'exemple 1 sont utilisables en remplaçant la 1-phényl pipérazine par la morpholine.
Point de fusion : 102 - 103° C
IR (pastille KBr) 2700 - 3000 $cm^{-1}$ ν CH
1765 $cm^{-1}$ ν C = 0
RMN $^1H$ ($CDCl_3$, TMS) :
δ :8,06 ppm, 1H, doublet dédoublé (J = 5 Hz et J = 1,4 Hz) H (5) δ :7,27 ppm, 1H, doublet dédoublé (J = 7,9 Hz et J = 1,4 Hz) H (7)
δ :7,14 ppm, 1H, doublet dédoublé (J = 7,9 Hz et J = 5 Hz) H (6)
δ :3,93 ppm, 2H, triplet (J = 6,2 Hz) $CH_2$
δ :3,63 ppm, 4H, multiplet 2 $CH_2$ morpholiniques
δ :2,49 ppm, 4H, multiplet 2 $CH_2$ morpholiniques

**EXEMPLE 4 : 1-[2-(4-(4-FLUOROPHENYL) PIPERAZIN-1-YL)ETHYL] [1H] OXAZOLO [5,4-b] PYRIDIN-2-ONE**

Les deux méthodes de synthèse décrites dans l'exemple 1 sont utilisables en remplaçant la 1-phényl pipérazine par la 1-(4-fluoro phényl) pipérazine.
Point de fusion : 135° C
IR (pastille KBr) 3100 - 2800 $cm^{-1}$ ν CH
1760 $cm^{-1}$ ν C = 0
RMN $^1H$ ($CDCl_3$, TMS) :
δ :8,04 ppm, 1H, doublet dédoublé (J = 5,1 Hz et J = 1,6 Hz) H (5)
δ :7,3 ppm, 1H, doublet dédoublé (J = 7,5 Hz et J = 1,6 Hz) H (7)
δ :7,14 ppm, 1H, doublet dédoublé (J = 7,5 Hz et J = 5,1 Hz) H (6)
δ :6,8-6,99 ppm, 4H, multiplet, protons aromatiques
δ :3,98 ppm, 2H, triplet (J = 6,1 Hz) $CH_2$
δ :3,03-3,08 ppm, 4H, multiplet, $CH_2$ pipérazine
δ :2,78 ppm, 2H, triplet, (J = 6,1 Hz) $CH_2$
δ :2,66-2,72 ppm, 4H, multiplet $CH_2$ pipérazine

**EXEMPLE 5 : 1-[2-(4-(2-METHOXY PHENYL) PIPERAZIN-1-YL) ETHYL] [1H] OXAZOLO [5,4-b] PYRIDIN-2-ONE**

Les deux méthodes de synthèse décrites dans l'exemple 1 sont urilisable en remplaçant la 1-phényl pipérazine par la 1-(2-méthoxy phényl) pipérazine.
Point de fusion : 146° C
IR (pastille KBr) 3100 - 2725 $cm^{-1}$ ν CH
1770 $cm^{-1}$ ν C = 0
RMN $^1H$ ($CDCl_3$, TMS) :
δ :8,03 ppm, 1H, doublet dédoublé (J = 5,1 Hz et J = 1,2 Hz) H (5)
δ :7,32 ppm, 1H, doublet dédoublé (J = 7,5 Hz et J = 1,2 Hz) H (7)
δ :7,14 ppm, 1H, doublet dédoublé (J = 7,5 Hz et J = 5,3 Hz) H (6)
δ :6,84-7,03 ppm, 5H, multiplet, protons aromatiques
δ :3,99 ppm, 2H, triplet (J = 6,3 Hz) $CH_2$
δ :3,86 ppm, 3H, singulet $OCH_3$
δ :2,99-3,08 ppm, 4H, multiplet, $CH_2$ pipérazine
δ :2,79 ppm, 2H, triplet, (J = 6,3 Hz) $CH_2$
δ :2,69-2,75 ppm, 4H, multiplet $CH_2$ pipérazine

**EXEMPLE 6 : 1-[2-(4-PHENYL PIPERIDIN-1-YL)ETHYL] [1H] OXAZOLO [5,4-b] PYRIDIN-2-ONE**

Les deux méthodes de synthèse décrites dans l'exemple 1 sont utilisables en remplaçant la 1-phényl pipérazine par la 4-phényl pipéridine.
Point de fusion : 128° C
IR (pastille KBr) 3100 - 2725 $cm^{-1}$ ν CH

1760 $cm^{-1}$ ν C = 0
RMN $^1H$ ($CDCl_3$, TMS) :
δ :8,04 ppm, 1H, doublet dédoublé (J = 5,1 Hz et J = 1,6 Hz) H (5)
δ :7,10-7,34 ppm, 7H, multiplet H (6), H(7) et 5 protons aromatiques
δ :3,98 ppm, 2H, triplet (J = 6,3 Hz) $CH_2$
δ :3,01-3,08 ppm, 2H, multiplet, $CH_2$ pipéridine
δ :2,75 ppm, 2H, triplet, (J = 6,3 Hz) $CH_2$
δ :2,44-2,55 ppm, 1H, multiplet, CH pipéridine
δ :2,13-2,26 ppm, 2H, multiplet, $CH_2$ pipéridine
δ :1,60-1,88 ppm, 4H, multiplet $CH_2$ pipéridine

**EXEMPLE 7 : 1-[2-(PYRROLIDIN-1-YL) ETHYL] [1H] OXAZOLO [5,4-b] PYRIDIN-2-ONE**

Les deux méthodes de synthèse décrites dans l'exemple 1 sont utilisables en remplaçant la 1-phényl pipérazine par la pyrrolidine.
Point de fusion : 90-91° C
IR (pastille KBr) 3080 - 2700 $cm^{-1}$ ν CH
1750 $cm^{-1}$ ν C = 0
RMN $^1H$ ($CDCl_3$, TMS) :
δ :8,02 ppm, 1H, doublet dédoublé (J = 5,1 Hz et J = 1,4 Hz) H (5)
δ :7,29 ppm, 1H, doublet dédoublé (J = 7,5 Hz et J = 1,4 Hz) H(7)
δ :7,12 ppm, 1H, doublet dédoublé (J = 7,5 Hz et J = 5,1 Hz) H(6)
δ :3,96 ppm, 2H, triplet (J = 6,3 Hz) $CH_2$
δ :2,84 ppm, 2H, triplet, (J = 6,3 Hz) $CH_2$
δ :2,54-2,62 ppm, 4H, multiplet, $CH_2$ pyrrolidine
δ :1,73-1,81 ppm, 4H, multiplet, $CH_2$ pyrrolidine

**EXEMPLE 8 : 1-[2-(HEXAMETHYLENEIMINO) ETHYL] [1H] OXAZOLO [5,4-b] PYRIDIN-2-ONE**

Les deux méthodes de synthèse décrites dans l'exemple 1 sont utilisables en remplaçant la 1-phényl pipérazine par l'hexaméthylène imine.
Point de fusion : 86° C
IR (pastille KBr) 3080 - 2700 $cm^{-1}$ ν CH
1750 $cm^{-1}$ ν C = 0
RMN $^1H$ ($CDCl_3$, TMS) :
δ :8,02 ppm, 1H, doublet dédoublé (J = 5,2 Hz et J = 1,3 Hz) H (5)
δ :7,31 ppm, 1H, doublet dédoublé (J = 7,8 Hz et J = 1,3 Hz) H (7)
δ :7,14 ppm, 1H, doublet dédoublé (J = 7,8 Hz et J = 5,2 Hz) H (6)
δ :3,89 ppm, 2H, triplet (J = 6,5 Hz) $CH_2$
δ :2,87 ppm, 2H, triplet, (J = 6,5 Hz) $CH_2$
δ :2,64-2,72 ppm, 4H, multiplet $CH_2$
δ :1,50-1,65 ppm, 8H, multiplet $CH_2$

**EXEMPLE 9 : 1-(3-MORPHOLINO PROP-1-YL) [1H] OXAZOLO [5,4-b] PYRIDIN-2-ONE**

**STADE A** : 1-(3-BROMOPROPYL) [1H] OXAZOLO [5,4-b] PYRIDIN-2-ONE

On procède comme pour la 1-(2 bromoéthyl) [1H] oxazolo [5,4-b] pyridin-2-one (Exemple 1 Méthode A stade B) en remplaçant le 1,2-dibromoéthane par du 1,3-dibromopropane.
On obtient la 1-[3-bromopropyl] [1H] oxazolo [5,4-b] pyridin-2-one avec un rendement de 57 %
Point de fusion : 62 - 63° C
IR (pastille KBr) 2900 - 3100 $cm^{-1}$ ν CH
1760 $cm^{-1}$ ν C = 0
RMN $^1H$ ($CDCl_3$, TMS) :
δ :8 ppm, 1H, doublet dédoublé (J = 5,2 Hz et J = 1,4 Hz) H (5)
δ :7,38 ppm, 1H, doublet dédoublé (J = 8 Hz et J = 1,4 Hz) H (7)
δ :7,17 ppm, 1H, doublet dédoublé (J = 5,2 Hz et J = 8 Hz) H (6)
δ :4 ppm, 2H, triplet (J = 6,3 Hz) $CH_2N$

δ :3,45 ppm, 2H, triplet (J = 6,3 Hz) $CH_2$
δ :2,37 ppm, 2H, multiplet $CH_2$

**STADE B** : 1-(3-MORPHOLINO PROP-1-YL) [1H] OXAZOLO [5,4-b] PYRIDIN-2-ONE

On procède comme pour la 1-[2-(4-phényl pipérazin-1-yl) éthyl] [1H] oxazolo [5,4-b] pyridin-2-one (Exemple 1 Méthode A stade C) en remplaçant la 1-(2-bromoéthyl) [1H] oxazolo [5,4-b] pyridin-2-one par la 1-(3-bromopropyl) [1H] oxazolo [5,4-b] pyridin-2-one et la 1-phénylpipérazine par la morpholine
Rendement : 72 %
Point de fusion : 170 - 171° C
IR (pastille KBr) 2700 - 3100 $cm^{-1}$ ν CH
1760 $cm^{-1}$ ν C = 0
RMN $^1H$ ($CDCl_3$, TMS) :
δ :8,04 ppm, 1H, doublet dédoublé (J = 5,2 Hz et J = 1,5 Hz) H (5)
δ :7,32 ppm, 1H, doublet dédoublé (J = 7,4 Hz et J = 1,5 Hz) H (7)
δ :7,14 ppm, 1H, doublet dédoublé (J = 7,4 Hz et J = 5,2 Hz) H (6)
δ :3,95 ppm, 2H, triplet (J = 6,6 Hz) $CH_2$
δ :3,62 ppm, 4H, multiplet 2 $CH_2$ morpholiniques
δ :2,4 ppm, 2H, triplet (J = 6,6 Hz) $CH_2$
δ :2,35 ppm, 4H, multiplet 2 $CH_2$ morpholiniques
δ :1,96 ppm, 2H, multiplet $CH_2$

**EXEMPLE 10 : 1-[3-(4-PHENYL PIPERAZIN-1-YL) PROP-1-YL][1H] OXAZOLO [5,4-b] PYRIDIN-2-ONE**

On procède comme pour la 1-(3-morpholino propyl) [1H] oxazolo [5,4-b] pyridin-2-one en remplaçant dans le stade B la morpholine par la 1-phényl pipérazine.
Point de fusion : 119° C
IR (pastille KBr) 3100 - 2760 $cm^{-1}$ ν CH
1760 $cm^{-1}$ ν C = 0
RMN $^1H$ ($CDCl_3$, TMS) :
δ :8,02 ppm, 1H, doublet dédoublé (J = 5,1 Hz et J = 1,6 Hz) H (5)
δ :7,26 ppm, 2H, triplet (J = 7,9 Hz) protons aromatiques
δ :7,12 ppm, 1H, doublet dédoublé (J = 7,9 Hz et J = 5,1 Hz) H (6)
δ :6,91 ppm, 2H, doublet (J = 7,9 Hz) protons aromatiques
δ :6,85 ppm, 1H, triplet (J = 7,9 Hz) protons aromatiques
δ :3,96 ppm, 2H, triplet (J = 6,7 Hz) $CH_2$
δ :3,09-3,16 ppm, 4H, multiplet $CH_2$ pipérazine
δ :2,5-2,55 ppm, 4H, multiplet $CH_2$ pipérazine
δ :2,45 ppm, 2H, triplet (J = 6,7 Hz) $CH_2$
δ :2,00 ppm, 2H, quadruplet (J = 6,7 Hz) $CH_2$

**EXEMPLE 11 : 1-(4-MORPHOLINO nBUT-1-YL)[1H] OXAZOLO [5,4-b] PYRIDIN-2-ONE**

**STADE A** : 1-(4-BROMOBUTYL) [1H] OXAZOLO [5,4-b] PYRIDIN-2-ONE

On procède comme pour la 1-(2 bromoéthyl) [1H] oxazolo [5,4-b] pyridin-2-one (Exemple 1 Méthode A stade B) en remplaçant le 1,2-dibromoéthane par du 1,4-dibromobutane.
Rendement : 62 %
Point de fusion : 60 - 61° C
IR (pastille KBr) 2900 - 3100 $cm^{-1}$ ν CH
1760 $cm^{-1}$ ν C = 0
RMN $^1H$ ($CDCl_3$, TMS) :
δ :8,05 ppm, 1H, doublet dédoublé (J = 5,1 Hz et J = 1,5 Hz) H (5)
δ :7,28 ppm, 1H, doublet dédoublé (J = 7,9 Hz et J = 1,5 Hz) H (7)
δ :7,16 ppm, 1H, doublet dédoublé (J = 7,9 Hz et J = 5,1 Hz) H (6)
δ :3,9 ppm, 2H, triplet (J = 6,5 Hz) $CH_2$
δ :3,46 ppm, 2H, triplet (J = 5,9 Hz) $CH_2$

**STADE B** : 1-[4-MORPHOLINO nBUT-1-YL] [1H] OXAZOLO [5,4-b] PYRIDIN-2-ONE

On procède comme pour la 1-[2-(4-phényl pipérazin-1-yl)éthyl] [1H] oxazolo [5,4-b] pyridin-2-one (Exemple 1 Méthode A stade C) en remplaçant la 1-(2-bromoéthyl) [1H] oxazolo [5,4-b] pyridin-2-one par la 1-(4-bromobutyl) [1H] oxazolo [5,4-b] pyridin-2-one et la 1-phényl pipérazine par la morpholine.
Rendement : 76 %
Point de fusion : 108 - 109° C
IR (pastille KBr) 2700 - 3000 $cm^{-1}$ ν CH
1760 $cm^{-1}$ ν C = 0
RMN $^1H$ ($CDCl_3$, TMS) :
δ :8,02 ppm, 1H, doublet dédoublé (J = 5,2 Hz et J = 1,5 Hz) H (5)
δ :7,25 ppm, 1H, doublet dédoublé (J = 7,4 Hz et J = 1,5 Hz) H (7)
δ :7,14 ppm, 1H, doublet dédoublé (J = 7,4 Hz et J = 5,2 Hz) H (6)
δ :3,88 ppm, 2H, triplet (J = 7,1 Hz) $CH_2$
δ :3,7 ppm, 2H, multiplet 2H morpholiniques
δ :2,43-2,46 ppm, 4H, multiplet $CH_2$ + 2H morpholiniques
δ :1,84 ppm, 2H, multiplet 2H morpholiniques

**EXEMPLE 12 : 1-[4-(4-PHENYL PIPERAZIN-1-YL) nBUT-1-YL][1H] OXAZOLO [5,4-b] PYRIDIN-2-ONE**

On procède comme pour la 1-(4-morpholino nbut-1-yl) [1H] oxazolo [5,4-b] pyridin-2-one en remplaçant dans le stade B la morpholine par la 1-phényl pipérazine.
Point de fusion : 94° C
IR (pastille KBr) 2960 - 2760 $cm^{-1}$ ν CH
1765 $cm^{-1}$ ν C = 0
RMN $^1H$ ($CDCl_3$, TMS) :
δ :8,03 ppm, 1H, doublet dédoublé (J = 5,1 Hz et J = 1,4 Hz) H (5)
δ :7,21 ppm, 3H, multiplet H (7) et 2 protons aromatiques
δ :7,15 ppm, 1H, doublet dédoublé (J = 7,9 Hz et J = 5,1 Hz) H (6)
δ :6,92 ppm, 2H, doublet (J = 7,5 Hz) protons aromatiques
δ :6,84 ppm, 1H, triplet (J = 7,5 Hz) protons aromatiques
δ :3,88 ppm, 2H, triplet (J = 7,5 Hz) $CH_2$
δ :3,16-3,22 ppm, 4H, multiplet $CH_2$ pipérazine
δ :2,54-2,61 ppm, 4H, multiplet $CH_2$ pipérazine
δ :2,46 ppm, 2H, triplet (J = 7,5 Hz) $CH_2$
δ :1,80-1,92 ppm, 2H, multiplet $CH_2$
δ :1,56-1,69 ppm, 2H, multiplet $CH_2$

**EXEMPLE 13 : 1-[2-(4-(N,N-DIETHYL AMINOCARBONYL) PIPERAZIN-1-YL)ETHYL] [1H] OXAZOLO [5,4-b] PYRIDIN-2-ONE**

On procède comme pour l'exemple 1 en remplaçant la 1-phényl pipérazine par la 1-(N,N-diéthyl aminocarbonyl) pipérazine.
Point de fusion : 137° C
IR (pastille KBr) 3100 - 2800 $cm^{-1}$ ν CH
1765 $cm^{-1}$ ν C = 0
RMN $^1H$ ($CDCl_3$, TMS) :
δ :8,02 ppm, 1H, doublet dédoublé (J = 5,2 Hz et J = 1,3 Hz) H (5)
δ :7,27 ppm, 1H, doublet dédoublé (J = 7,8 Hz et J = 1,3 Hz) H (7)
δ :7,12 ppm, 1H, doublet dédoublé (J = 7,8 Hz et J = 5,2 Hz) H (6)
δ :3,94 ppm, 2H, triplet (J = 2,3 Hz) $CH_2$
δ :3,12 et 3,33 ppm, 8H, multiplet $CH_2$
δ :2,71 ppm, 2H, triplet (J = 2,3 Hz) $CH_2$
δ :2,5 ppm, 4H, triplet (J = 1,7 Hz) $CH_2$ pipérazine
δ :1,10 ppm, 6H, triplet (J = 5,3Hz) $CH_3$

**EXEMPLE 14 : 3-(2-(4-PHENYL PIPERAZIN-1-YL) ETHYLAMINO) 2-HYDROXY PYRIDINE**

Chauffer à reflux pendant 4 heures une solution de 1 mole de 1-[2-(4-phényl pipérazin -1-yl) éthyl] [1H] oxazolo [5,4-b] pyridin-2-one (obtenue dans l'exemple 1) dans 50 cm3 d'une solution aqueuse à 10 % d'hydroxyde de sodium.

Après refroidissement la solution aqueuse est légèrement acidifiée par addition d'acide chlorhydrique aqueux à 30 % puis neutralisée jusqu'à Ph = 7 avec une solution aqueuse saturée de bicarbonate de sodium.

Le précipité obtenu est filtré, lavé trois fois à l'eau puis séché.

**ETUDE PHARMACOLOGIOUE DES DERIVES DE L' INVENTION**

**A RECHERCHE DE L'ACTIVITE ANALGESIOUE**

1) Crampes induites à l'acide acétique "Acetic Acid Writhing"

Le potentiel analgésique de ces produits a été recherché selon le test dit "Acetic Acid writhing" (ou encore test de "KOSTER") qui est basé sur le comptage des crampes abdominales induites chez le rat par injection intrapéritonéale d'acide acétique.

Des rats mâles Wistar randomisés en lot de 5 (poids 150 ± 10 g) reçoivent les produits à tester per os 30 mn avant l'injection intrapéritonéale de 1 $cm^3$ d'acide acétique à 1 %.

Le nombre de crampes est compté durant les 25 minutes qui suivent l'injection.

Le pourcentage d'activité a été évalué pour chaque composé (% de diminution du nombre de crampes chez les traités par rapport aux témoins).

2) Crampes induites à la phényl benzoquinone "PBQ Writhing"

Le potentiel analgésique de ces produits a été recherché selon le test dit "PBQ Writhing" (ou encore test de SIGMUND") qui est basé sur le comptage des crampes induites chez la souris par injection intrapéritonéale de phényl benzoquinone.

Des souris mâles CD-1 randomisés en lot de 5 reçoivent les produits à tester per os 30 mn avec l'injection intrapéritonéale de 0,25 cm3 d'une solution à 0,01 % de phényl benzoquinone dans un mélange eau-éthanol 95-5

Le nombre de crampes est compté entre la 5ème et la 15ème minutes après l'injection de phényl benzoquinone.

Le pourcentage d'activité a été évalué pour chaque composé (% de diminution du nombre de crampes chez les traités par rapport aux témoins)

3) Résultats

| PRODUIT | DOSE | Acetic Acid Writhing % d'inhibition | PBQ Whrithing % d'inhibition |
|---|---|---|---|
| Aspirine | 50 mg/kg | 69 % | 70 % |
| 1-[3-(4-phényl pipérazin-1-yl) prop-1-yl] [1H] oxazolo [5,4-b] pyridin-2-one | 50 mg/kg | 92 % | 98 % |
| 1-[4-(4-phényl pipérazin-1-yl) but-1-yl ] [1H] oxazolo [5,4-b] pyridin-2-one | 50 mg/kg | 96 % | 91 % |
| 1-[2-(4-(4-fluorophényl) pipérazin-1-yl) éthyl] [1H] oxazolo [5,4-b] pyridin-2-one | 50 mg/kg | 84 % | 83 % |

Il apparait que les composés de l'invention possèdent une activité antalgique très intéressante, très significativement supérieure à celle de l'aspirine.

## B RECHERCHE D'UNE ACTIVITE ANTIINFLAMMATOIRE

Le potentiel antiinflammatoire des composés a été recherché sur un modèle d'inflammation aigüe provoquée par injection sous cutanée d'une suspension colloïdale de carragénine au niveau de la face plantaire de la patte postérieure de rat, selon une technique inspirée de la méthode de WINTER, RISLEY et NUSS Proc. Soc. Exp. Biol. Med. 111, 554 (1962) et WINEGAR et Coll. J. Pharmacol. Exp. Ther 166, 96 (1969)

Les rats mâles WISTAR EOPS de 250 ± 10 g sont randomisés par lot de 10 et reçoivent les substances à tester per os 1 heure après l'injection au niveau de la patte arrière gauche de 0,15 $cm^3$ d'une suspension à 1 % de carraghénate. L'inflammation est mesurée 5 heures plus tard par pesée des pattes et sont calculés les pourcentages d'inflammation et d'activité antiinflammatoire (AAI).

$$\text{Inflammation \%} = \frac{\text{poids de la patte inflammée} - \text{poids de la patte témoin}}{\text{poids de la patte témoin}} \times 100$$

$$\text{AAI \%} = \frac{\text{inflammation moyenne du lot témoin} - \text{inflammation moyenne du lot traité}}{\text{inflammation moyenne du lot témoin}} \times 100$$

| PRODUIT | DOSE | AAI |
|---|---|---|
| 1-[2-(4-phényl pipérazin-1-yl) éthyl] [1H] oxazolo [5,4-b] pyridin-2-one | 15 mg/kg<br>150 mg/kg<br>250 mg/kg | 0 %<br>12 %<br>2 % |
| 1-[2-(4-(3-trifluorométhyl phényl) pipérazin-1-yl) éthyl] [1H] oxazolo [5,4-b] pyridin-2-one | 75 mg/kg<br>200 mg/kg | 0 %<br>0 % |
| 1-[2-morpholino éthyl] [1H] oxazolo [5,4-b] pyridin-2-one | 100 mg/kg<br>200 mg/kg | 0 %<br>8 % |
| glafénine | 50 mg/kg<br>150 mg/kg | 24 %<br>28 % |
| aspirine | 150 mg/kg | 12 % |

Comme on peut le constater l'activité antiinflammatoire des composés de l'invention est très inférieure à celle de composés de références dont la glafénine.

## C ETUDE DE LA TOLERANCE GASTRIOUE

La tolérance gastrique de ces composés a été étudiée par recherche d'une gastroagressivité chez le rat selon une méthode inspirée de LAMBLING (LAMBLING et Coll. Arch. Mal. Appareil digestif et nutrition (1953), 42 p 430)

Les produits à tester sont administrés per os à des rats mâles WISTAR EOPS randomisés par lot de 5 et préalablement soumis à une diète hydrique de 24 heures .

Après stabulation des animaux dans des cages de contrainte pendant six heures avec privation d'eau de boisson on procède à la détermination au niveau gastrique des indices d'ulcération **(U)**, d'hyperhémie **(H)** et d'agressivité gastrique **(i)** par la cotation modifiée de LWOFF (LWOFF J. M. J. Pharmacol. Paris (1971) 2 (1) p 81-83).

$$U \text{ ou } H = \frac{\text{somme des cotations (U ou H)} \times \text{pourcentage d'estomacs lésés}}{\text{nombre d'animaux expérimentés}}$$

i = 3U + H

| PRODUIT | DOSE | INDICE D'AGRESSIVITE GASTRIQUE |
|---|---|---|
| 1-[2-(4-phényl pipérazin-1-yl) éthyl] [1H] oxazolo [5,4-b] pyridin-2-one | 15 mg/kg<br>75 mg/kg | 8<br>8 |
| 1-[2-(4-(3-trifluorométhyl phényl) pipérazin-1-yl) éthyl] [1H] oxazolo [5,4-b] pyridin-2-one | 75 mg/kg<br>250 mg/kg | 8<br>8 |
| 1-[2-morpholino éthyl] [1H] oxazolo [5,4-b] pyridin-2-one | 100 mg/kg<br>250 mg/kg | 0<br>2 |
| glafénine | 50 mg/kg<br>250 mg/kg | 20<br>240 |
| aspirine | 250 mg/kg | 146 |
| Indométhacin | 5 mg/kg<br>10 mg/kg | 516<br>570 |

Les composés de l'invention présentent donc une très bonne tolérance gastrique chez le rat.

**EXEMPLE 15 : COMPRIMES DOSES A 30 MG DE 1-[2-(4-(3-TRIFLUOROMETHYL PHENYL) PIPERAZIN-1-YL) ETHYL][1H] OXAZOLO [5,4-b] PYRIDIN-2-ONE**

Formule de préparation pour 1000 comprimés :

| | |
|---|---|
| 1-[2-(4-(3-trifluoromethyl phenyl) piperazin-1-yl)ethyl] [1H] oxazolo [5,4-b]pyridin-2-one | 30 G |
| Amidon de blé | 15 G |
| Amidon de maïs | 15 G |
| Lactose | 65 G |
| Stéarate de magnésium | 1 G |
| Silice | 1 G |
| Hydroxy propyl cellulose | 2 G |

**Revendications**

**Revendications pour les Etats contractants suivants : AT, BE, CH, DE, DK, FR, GB, IT, LI, LU, NL, SE**

**1.** Composés de formule générale (I) :

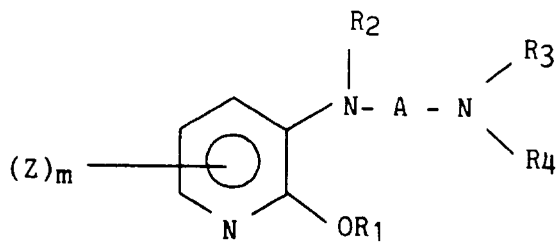

(I)

dans laquelle :

- $R_1$ et $R_2$ représentent chacun un atome d'hydrogène ou forment avec l'oxygène et l'azote qui les portent un enchainement - O - CO - N - ce qui correspond aux oxazolo [5,4 b] pyridin-2-ones,
- Z représente un atome d'halogène, un groupement alkyle inférieur linéaire ou ramifié comprenant de 1 à 6 atomes de carbone, un groupement alcoxy inférieur linéaire ou ramifié comprenant de 1 à 6 atomes de carbone, un groupement trifluorométhyle,
- m nombre entier peut prendre les valeurs 0, 1, 2, 3,
- A est un radical alkyl linéaire ou ramifié comprenant de 1 à 6 atomes de carbone,
- $R_3$ et $R_4$ identiques ou différents représentent :
  - un atome d'hydrogène,
  - un groupement alkyle inférieur linéaire ou ramifié, comprenant de 1 à 6 atomes de carbone,
  - un groupement alkényle inférieur linéaire ou ramifié, comprenant de 1 à 6 atomes de carbone,
  - un groupement aryle ou hétéroaryle éventuellement substitué,
  - un groupement arylalkyle ou hétéroarylalkyle éventuellement substitué dont la chaine alkyle comprend de 1 à 3 atomes de carbone,
  - un groupement cycloalkyle mono ou bicyclique de 3 à 10 atomes de carbone ,

  ou bien :

  $R_3$ et $R_4$ constituent avec l'atome d'azote auquel ils sont liés un système hétérocyclique azoté, mono ou bicyclique, saturé ou insaturé, comprenant un maximum de 12 atomes non compris les atomes d'hydrogène parmi lesquels on peut compter de un à trois hétéroatomes choisis parmi azote> oxygène ou soufre et éventuellement substitué par un ou des :
  - groupement hydroxy,
  - groupement oxo,
  - groupement alkyle inférieur, linéaire ou ramifié, comprenant de 1 à 6 atomes de carbone,
  - groupement aryle éventuellement substitué,
  - groupement arylalkyle éventuellement substitué ou diarylalkyle éventuellement substitué dont la chaine alkyle comporte de 1 à 3 atomes de carbone,
  - groupement -CO - $OR_5$
  - groupement

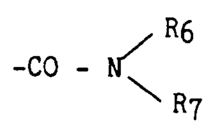

- $R_5$ représente :
  - un atome d'hydrogène,
  - un groupement alkyle inférieur linéaire ou ramifié comprenant de 1 à 6 atomes de carbone,
  - un groupement aryle éventuellement substitué,
  - un groupement aralkyle éventuellement substitué dont la chaine alkyle comprend de 1 à 3 atomes de carbone,

  $R_6$ et $R_7$ identiques ou différents ont la même signification que $R_5$,

- leurs isomères, épimères, diastéréoisomères,
- leurs sels d'addition à un acide pharmaceutiquement acceptable et/ou lorsque $R_1$ et $R_2$ représentent chacun un atome d'hydrogène, leurs sels d'addition à une base pharmaceutiquement acceptable,
- par groupement aryle on entend un groupement mono ou bicyclique insaturé ou aromatique comprenant de 5 à 12 atomes de carbone,
- par groupement hétéroaryle on entend un groupement mono ou bicyclique, insaturé ou aromatique, comprenant de 5 à 12 atomes non compris les atomes d'hydrogène et intégrant dans son squelette carboné un, deux ou trois hétéroatomes choisis parmi azote, oxygène ou soufre,
- le terme substitué associé aux expressions aryle, arylalkyle, diarylalkyle, hétéroaryle, hétéroarylalkyle signifie que le, ou les, noyaux aryle ou hétéroaryle peuvent être substitués par un ou plusieurs groupement alkyle inférieur de 1 à 6 atomes de carbone linéaire ou ramifié, alcoxy inférieur de 1 à 6 atomes de carbone linéaire ou ramifié, hydroxy, nitro, halogène, trifluorométhyle.

**2.** Composés selon la revendication 1, pour lesquels $R_1$ et $R_2$ forment avec l'oxygène et l'azote qui les portent un chainon - O - CO - N -, de formule générale ($I_A$) :

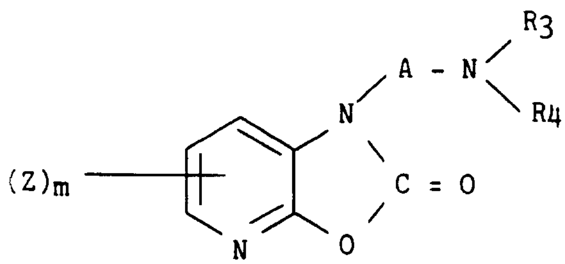

**(IA)**

Z, m, A, $R_3$, $R_4$ ayant la même signification que dans la revendication 1, leurs isomères, épimères, diastéréoisomères, ainsi que leurs sels d'addition à un acide pharmaceutiquement acceptable.

**3.** Composés selon la revendication 1, pour lesquels $R_1$ et $R_2$ représentent chacun un atome d'hydrogène, de formule générale ($I_B$) :

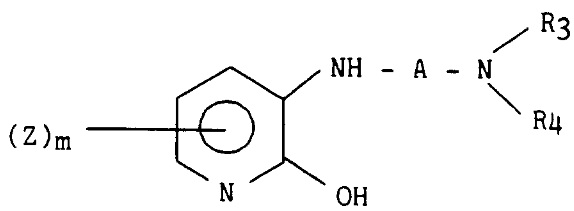

**(IB)**

Z, m, A, $R_3$, $R_4$ ayant la même signification que dans la revendication 1, leurs isomères, épimères, diastéréoisomères, ainsi que leurs sels d'addition à un acide ou à une base pharmaceutiquement acceptable.

**4.** Composés selon la revendication 1 pour lesquels m = 0 et A est un radical alkyl linéaire de 2 à 6 atomes de carbone.

**5.** Composé selon la revendication 1 qui est la 1-[2-(4-phényl pipérazin-1-yl) éthyl] [1H] oxazolo [5,4-b] pyridin-2-one dont la formule est représentée ci dessous, ainsi que ses sels d'addition à un acide pharmaceutiquement acceptable.

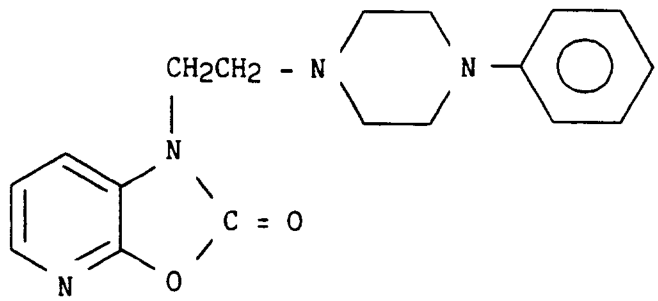

**6.** Composé selon la revendication 1 qui est la 1-[2-(4-(3-trifluorométhyl phényl) pipérazin-1-yl)éthyl] [1H] oxazolo [5,4-b] pyridin-2-one dont la formule est représentée ci dessous, ainsi que ses sels d'addition à un acide pharmaceutiquement acceptable.

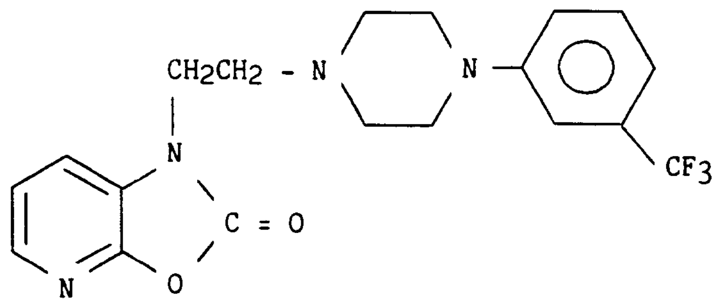

**7.** Composé selon la revendication 1 qui est la 1-[2-morpholino éthyl] [1H] oxazolo [5,4-b] pyridin-2-one dont la formule est représentée ci dessous, ainsi que ses sels d'addition à un acide pharmaceutiquement acceptable.

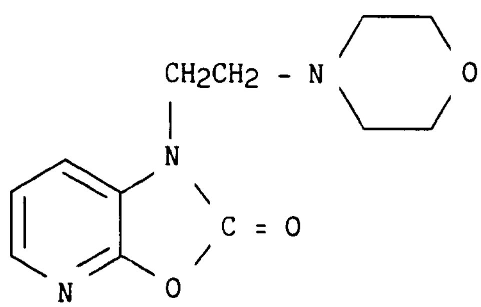

**8.** Composé selon la revendication 1 qui est la 1-[3-morpholino prop-1-yl] [1H] oxazolo [5,4-b] pyridin-2-one dont la formule est représentée ci dessous, ainsi que ses sels d'addition à un acide pharmaceutiquement acceptable.

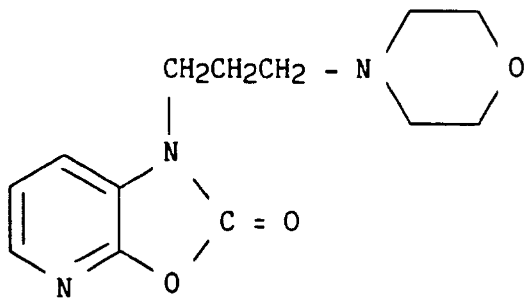

9. Composé selon la revendication 1 qui est la 1-[4-morpholino but-1-yl] [1H] oxazolo [5,4-b] pyridin-2-one dont la formule est représentée ci dessous, ainsi que ses sels d'addition à un acide pharmaceutiquement acceptable.

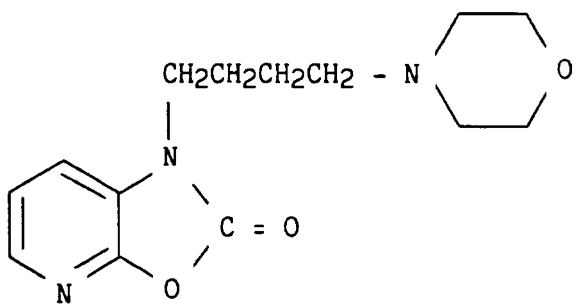

10. Composé selon la revendication 1 qui est la 1-[3-(4-phényl pipérazin-1-yl) prop-1-yl] [1H] oxazolo [5,4-b] pyridin-2-one dont la formule est représentée ci dessous, ainsi que ses sels d'addition à un acide pharmaceutiquement acceptable.

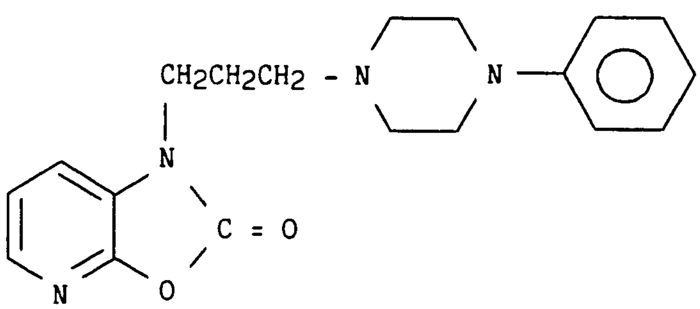

11. Composé selon la revendication 1 qui est la 1-[4-(4-phényl pipérazin-1-yl) but-1-yl] [1H] oxazolo [5,4-b] pyridin-2-one dont la formule est représentée ci dessous, ainsi que ses sels d'addition à un acide pharmaceutiquement acceptable.

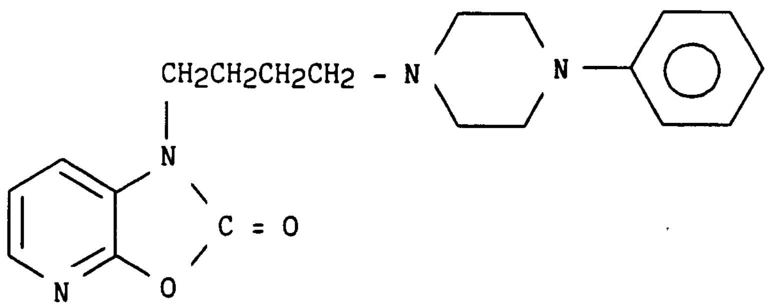

**12.** Composé selon la revendication 1 qui est la 1-[2-(4-(4-fluorophényl) pipérazin-1-yl) éthyl] [1H] oxazolo [5,4-b] pyridin-2-one dont la formule est représentée ci dessous, ainsi que ses sels d'addition à un acide pharmaceutiquement acceptable.

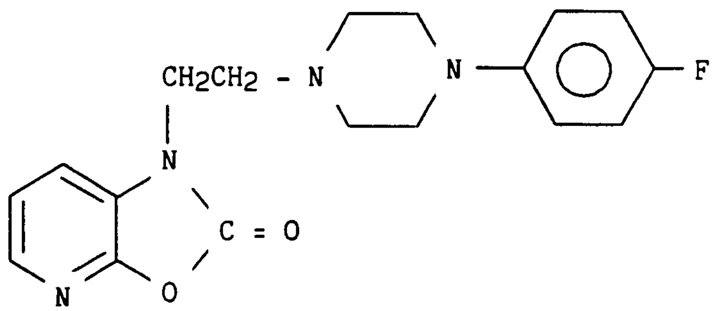

**13.** Composé selon la revendication 1 qui est la 1-[2-(pyrrolidin-1-yl) éthyl] [1H] oxazolo [5,4-b] pyridin-2-one dont la formule est représentée ci dessous, ainsi que ses sels d'addition à un acide pharmaceutiquement acceptable.

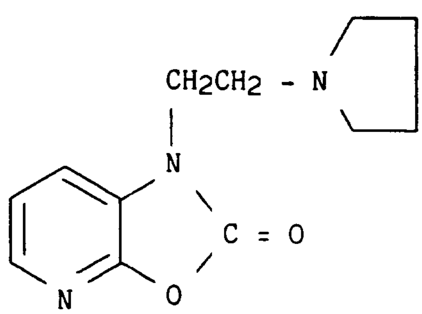

**14.** Composé selon la revendication 1 qui est la 1-[2-(4-phényl pipéridin-1-yl) éthyl] [1H] oxazolo [5,4-b] pyridin-2-one dont la formule est représentée ci dessous, ainsi que ses sels d'addition à un acide pharmaceutiquement acceptable.

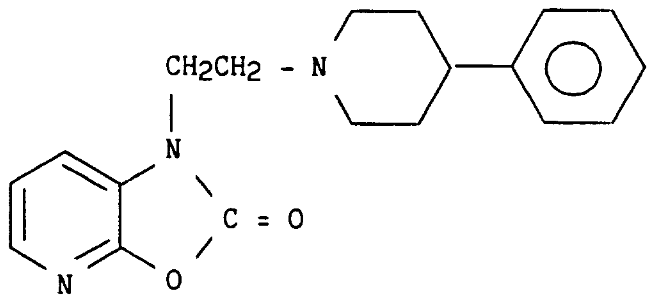

**15.** Composé selon la revendication 1 qui est la 1-[2-(4-N,N-diéthylaminocarbonyl)pipérazin-1-yl) éthyl] [1H] oxazolo [5,4-b] pyridin-2-one dont la formule est représentée ci dessous, ainsi que ses sels d'addition à un acide ou à une base pharmaceutiquement acceptable.

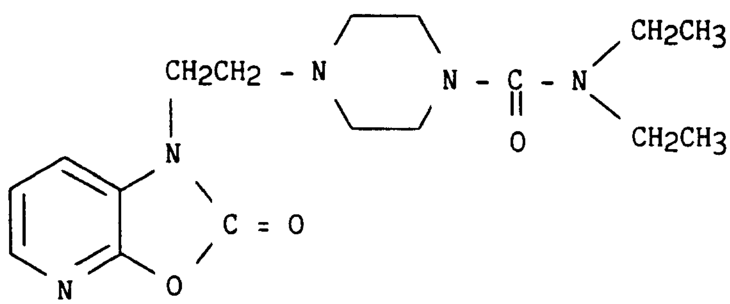

**16.** Composé selon la revendication 1 qui est la 3-[2-(4-phényl pipérazin-1-yl) éthylamino] 2-hydroxy pyridine dont la formule est représentée ci dessous, ainsi que ses sels d'addition à un acide ou à une base pharmaceutiquement acceptable.

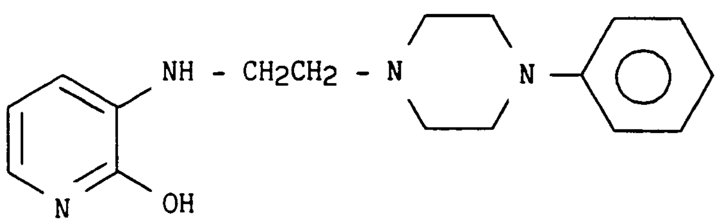

**17.** Procédé de préparation des composés de formule générale ($I_A$) selon la revendication 2 caractérisé en ce que l'on fait réagir une 3-amino 2-pyridinone de formule générale (II) :

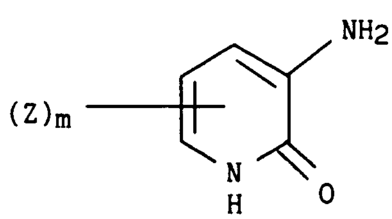

**(II)**

dans laquelle Z et m ont la même signification que dans les composés de formule générale (I), en

solution à -78° C (température du mélange acétone carboglace) dans un solvant ou un mélange de solvants aprotiques avec du bis (trichlorométhyl) carbonate (triphosgène) en présence d'une amine basique comme par exemple la triéthylamine de manière à obtenir une [1H] oxazolo [5,4-b] pyridine-2-one de formule générale (III) :

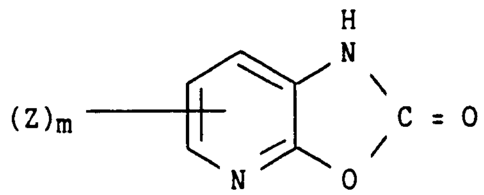

(III)

dans laquelle Z et m ont la même signification que dans les composés de formule générale (I) que l'on fait réagir avec un alcoolate ou un hydrure de métal alcalin en milieu organique aprotique de manière à obtenir le composé de formule générale (IV) :

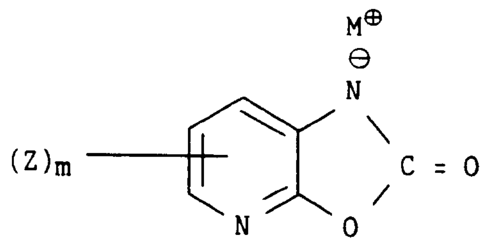

(IV)

dans laquelle Z et m ont la même signification que dans les composés de formule générale (I) et M représente un métal alcalin, que l'on fait réagir en milieu organique et à une température comprise entre la température ambiante et le reflux du solvant (ou mélange de solvants) choisi avec un composé électrophile (préférentiellement en excès) de formule générale (V) :

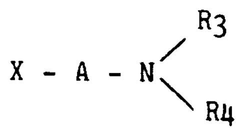

(V)

dans laquelle X représente un atome d'halogène et A, $R_3$, $R_4$ ont la même signification que dans les composés de formule générale (I) de manière à obtenir après refroidissement extraction et éventuelle purification, les composés de formule générale ($I_A$) :

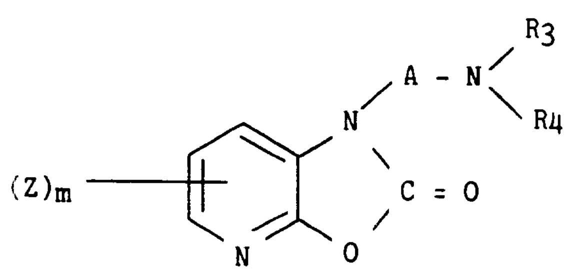

($I_A$)

tels que définis dans la revendication 2,
étant entendu que les composés de formule générale ($I_A$) ainsi obtenus peuvent si on le désire être séparés en leurs isomères et/ou être salifiés par un acide pharmaceutiquement acceptable.

**18.** Procédé de préparation des composés de formule ($I_A$) selon la revendication 2 caractérisé en ce que l'on fait réagir un composé de formule générale (IV)

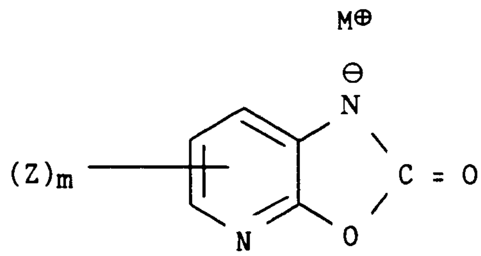

(IV)

dans laquelle Z et m ont la même signification que dans les composés de formule générale (I) et M représente un métal alcalin, en milieu organique et à une température comprise entre la température ambiante et le reflux du solvant (ou mélange de solvants) choisi avec un composé électrophile (préférentiellement en excès) de formule générale (VI)

X - A - X' (VI)

dans laquelle X et X' identiques ou différents représentent chacun un atome d'halogène et A a la même définition que dans les composés de formule générale (I) de manière à obtenir le composé halogéné de formule générale (VII) :

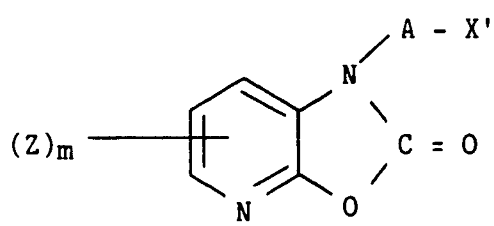

(VII)

dans laquelle X', A, Z et m ont la même signification que précedemment que l'on fait finalement réagir avec une amine (préférentiellement en excès) de formule générale (VIII) :

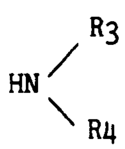

(VIII)

dans laquelle $R_3$ et $R_4$ ont la même signification que dans les composés de formule (I), en milieu organique, éventuellement en présence d'une amine basique comme par exemple la diisopropylamine et à une température comprise entre la température ambiante et le reflux du solvant choisi de manière à obtenir après refroidissement, extraction et éventuelle purification les composés de formule générale ($I_A$) tels que définis dans la revendication 2,
étant entendu que les composés de formule ($I_A$) ainsi obtenus peuvent, si on le désire, être séparés en leurs isomères et/ou être salifiés par un acide pharmaceutiquement acceptable.

**19.** Procédé de préparation des composés de formule générale ($I_A$) selon la revendication 2 pour lesquels A est un chainon méthylène - $CH_2$ - caractérisé en ce que l'on fait réagit un composé de formule générale (IV)

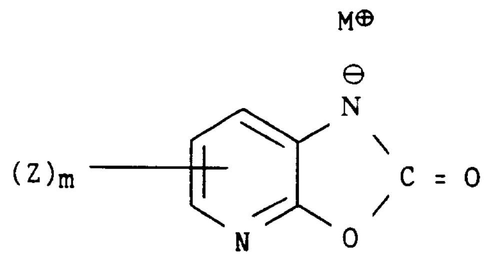

(IV)

dans laquelle Z et m ont la même signification que dans les composés de formule générale (I) et M représente un métal alcalin, en milieu organique et à une température comprise entre la température ambiante et le reflux du solvant (ou mélange de solvants) choisi avec du chlorométhyl phényl sulfure de manière à obtenir les composés de formule générale (IX) :

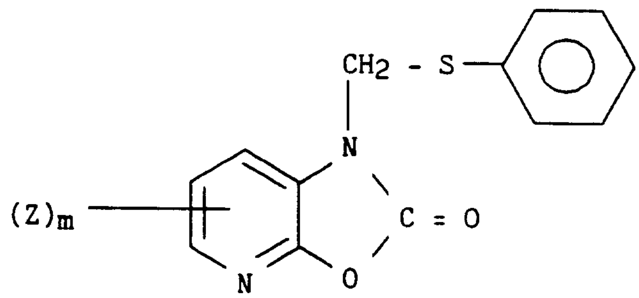

(IX)

dans laquelle Z et m ont la même signification que dans les composés de formule générale (I) que l'on fait ensuite réagir en milieu organique avec du chlorure de sulfuryle pour obtenir après purification le composé chloré de formule générale (X) :

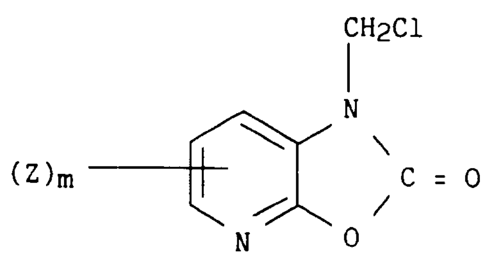

(X)

dans laquelle Z et m ont la même signification que dans les composés de formule générale (I) que l'on fait ensuite réagir comme dans la revendication 18 avec des composés de formule générale (VIII) de manière à obtenir les composés de formule générale ($I_A$) tels que définis dans la revendication 2 pour lesquels A est un chainon méthylène - $CH_2$ -, étant entendu que les composés de formule ($I_A$) ainsi obtenus peuvent si on le désire être séparés en leurs isomères et/ou être salifiés par un acide pharmaceutiquement acceptable.

**20.** Procédé de préparation des composés de formule générale ($I_A$) selon la revendication 2 pour lesquels A est un chainon méthylène - CH2 - caractérisé en ce que l'on condense en milieu d'alcool aliphatique inférieur, un composé de formule générale (III) tel que défini dans la revendication 17 avec une amine de formule générale (VIII) telle que définie dans la revendication 18 en léger excès et en présence d'un excès de formol à une température comprise entre la température ambiante et le reflux du milieu réactionnel de manière à obtenir après refroidissement, isolement et éventuelle purification par chromatographie sur colonne de silice les composés de formule générale ($I_A$) tels que définis dans la revendication 2 pour lesquels A est un chainon méthylène - $CH_2$ -, étant entendu que les composés de formule ($I_A$) ainsi obtenus peuvent, si on le désire, être séparés en leurs isomères et/ou être salifiés par un acide pharmaceutiquement acceptable.

**21.** Procédé de préparation de composés de formule générale ($I_B$) selon la revendication 3 caractérisé en ce que l'on traite les composés de formule générale ($I_A$) par un agent alcalin comme par exemple l'hydroxyde de sodium en solution aqueuse à une température comprise entre la température ambiante et la température de reflux du milieu réactionnel pour conduire après éventuelle acidification et/ou neutralisation du milieu réactionnel aux composés de formule générale ($I_B$) :

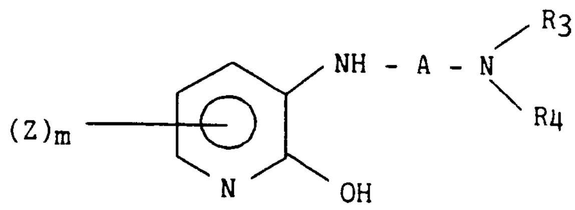

($I_B$)

cas particulier des dérivés de formule générale (I) pour lesquels $R_1 = R_2 = H$ et A, $R_3$, $R_4$, Z et m ont la même signification que dans les composés de formule générale (I).

**22.** Composés de formule générale (VII)

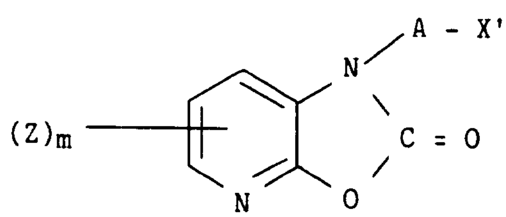

(VII)

dans laquelle X' représente un atome d'halogène, A est un radical alkyl linéaire ou ramifié comprenant de 1 à 6 atomes de carbone, Z représente un atome d'halogène, un groupement alkyle ou alkoxy inférieur comprenant de 1 à 6 atomes de carbone ou un groupement trifluorométhyle, et m un nombre entier compris entre 0 et 3 inclusivement, utilisables comme intermédiaires de synthèse dans la préparation des composés de formule générale (I) selon la revendication 1.

**23.** Compositions pharmaceutiques contenant comme principe actif au moins un composé selon la revendication 1 en combinaison avec un ou plusieurs excipients ou véhicules inertes, non toxiques pharmaceutiquement acceptables

**24.** Compositions pharmaceutiques selon la revendication 23 utilisables dans le traitement d'algies

**Revendications pour l'Etat contractant suivant : GR**

**1.** Procédé de préparation de nouveaux composés de formule générale (I) :

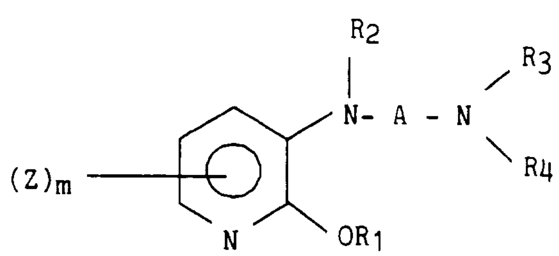

(I)

dans laquelle :

- $R_1$ et $R_2$ représentent chacun un atome d'hydrogène ou forment avec l'oxygène et l'azote qui les portent un enchainement - O - CO - N - ce qui correspond aux oxazolo [5,4 b] pyridin-2-ones,
- Z représente un atome d'halogène, un groupement alkyle inférieur linéaire ou ramifié comprenant de 1 à 6 atomes de carbone, un groupement alcoxy inférieur linéaire ou ramifié comprenant de 1 à 6 atomes de carbone, un groupement trifluorométhyle,
- m nombre entier peut prendre les valeurs 0, 1, 2, 3,
- A est un radical alkyl linéaire ou ramifié comprenant de 1 à 6 atomes de carbone,
- $R_3$ et $R_4$ identiques ou différents représentent :
  - un atome d'hydrogène,
  - un groupement alkyle inférieur linéaire ou ramifié, comprenant de 1 à 6 atomes de carbone,
  - un groupement alkényle inférieur linéaire ou ramifié, comprenant de 1 à 6 atomes de carbone,
  - un groupement aryle ou hétéroaryle éventuellement substitué,
  - un groupement arylalkyle ou hétéroarylalkyle éventuellement substitué dont la chaine alkyle comprend de 1 à 3 atomes de carbone,
  - un groupement cycloalkyle mono ou bicyclique de 3 à 10 atomes de carbone ,

  ou bien :

  $R_3$ et $R_4$ constituent avec l'atome d'azote auquel ils sont liés un système hétérocyclique azoté, mono ou bicyclique, saturé ou insaturé, comprenant un maximum de 12 atomes non compris les atomes d'hydrogène parmi lesquels on peut compter de un à trois hétéroatomes choisis parmi azote, oxygène ou soufre et éventuellement substitué par un ou des :
  - groupement hydroxy,
  - groupement oxo,
  - groupement alkyle inférieur, linéaire ou ramifié, comprenant de 1 à 6 atomes de carbone,
  - groupement aryle éventuellement substitué,
  - groupement arylalkyle éventuellement substitué ou diarylalkyle éventuellement substitué dont la chaine alkyle comporte de 1 à 3 atomes de carbone,
  - groupement -CO - $OR_5$
  - groupement

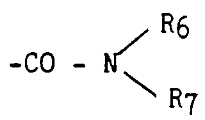

- $R_5$ représente :
  - un atome d'hydrogène,
  - un groupement alkyle inférieur linéaire ou ramifié comprenant de 1 à 6 atomes de carbone,
  - un groupement aryle éventuellement substitué,
  - un groupement aralkyle éventuellement substitué dont la chaine alkyle comprend de 1 à 3 atomes de carbone,

  $R_6$ et $R_7$ identiques ou différents ont la même signification que $R_5$, étant entendu que :
- par groupement aryle on entend un groupement mono ou bicyclique insaturé ou aromatique comprenant de 5 à 12 atomes de carbone,
- par groupement hétéroaryle on entend un groupement mono ou bicyclique, insaturé ou aromatique, comprenant de 5 à 12 atomes non compris les atomes d'hydrogène et intégrant dans son squelette carboné un, deux ou trois hétéroatomes choisis parmi azote, oxygène ou soufre,
- le terme substitué associé aux expressions aryle, arylalkyle, diarylalkyle, hétéroaryle, hétéroarylalkyle signifie que le, ou les, noyaux aryle ou hétéroaryle peuvent être substitués par un ou plusieurs groupement alkyle inférieur de 1 à 6 atomes de carbone linéaire ou ramifié, alcoxy inférieur de 1 à 6 atomes de carbone linéaire ou ramifié, hydroxy, nitro, halogène, trifluorométhyle,

ainsi que de leurs isomères, épimères, diastéréoisomères et de leurs sels d'addition à un acide pharmaceutiquement acceptable et/ou lorsque $R_1$ et $R_2$ représentent chacun un atome d'hydrogène, leurs sels d'addition à une base pharmaceutiquement acceptable,

caractérisé en ce que :

A) Soit l'on fait réagir une 3-amino 2-pyridinone de formule générale (II) :

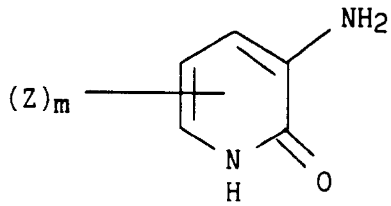

(II)

dans laquelle Z et m ont la même signification que dans les composés de formule générale (I), en solution à -78° C (température du mélange acétone carboglace) dans un solvant ou un mélange de solvants aprotiques avec du bis (trichlorométhyl) carbonate (triphosgène) en présence d'une amine basique comme par exemple la triéthylamine de manière à obtenir une [1H] oxazolo [5,4-b] pyridin-2-one de formule générale (III) :

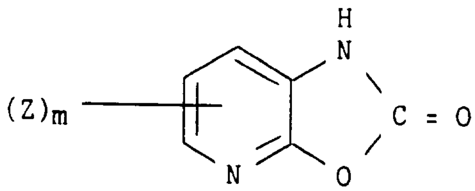

(III)

dans laquelle Z et m ont la même signification que dans les composés de formule générale (I), que l'on fait réagir avec un alcoolate ou un hydrure de métal alcalin en milieu organique aprotique de manière à obtenir le composé de formule générale (IV) :

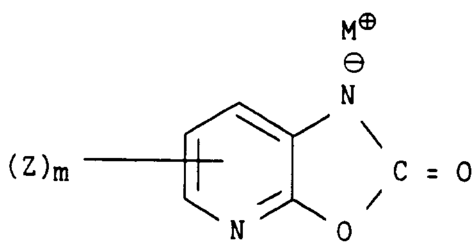

(IV)

dans laquelle Z et m ont la même signification que dans les composés de formule générale (I) et M représente un métal alcalin, que l'on fait réagir :

a) soit en milieu organique et à une température comprise entre la température ambiante et le reflux du solvant (ou mélange de solvants) choisi avec un composé électrophile (préférentiellement en excès) de formule générale (V):

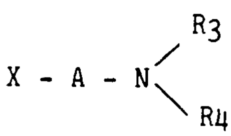

(V)

dans laquelle X représente un atome d'halogène et A, $R_3$, $R_4$ ont la même signification que dans les composés de formule générale (I), de manière à obtenir après refroidissement extraction et éventuelle purification, les composés de formule générale ($I_A$) :

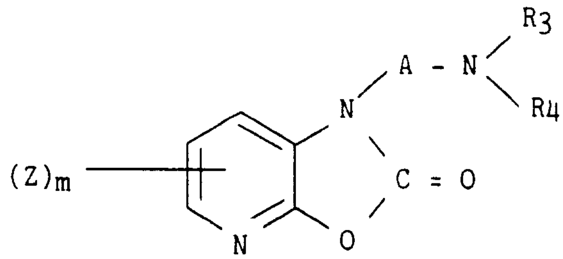

(IA)

dans laquelle A, $R_3$, $R_4$, Z et m ont la même signification que dans les composés de formule générale (I),

b) soit en milieu organique et à une température comprise entre la température ambiante et le reflux du solvant (ou mélange de solvants) choisi avec un composé électrophile (préférentiellement en excès) de formule générale (VI) :

X - A - X' (VI)

dans laquelle X et X' identiques ou différents représentent chacun un atome d'halogène et A a la même définition que dans les composés de formule générale (I), de manière à obtenir le composé halogéné de formule générale (VII) :

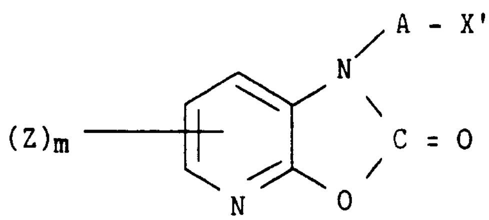

(VII)

dans laquelle X', A, Z et m ont la même signification que précedemment, que l'on fait finalement réagir avec une amine (préférentiellement en excès) de formule générale (VIII) :

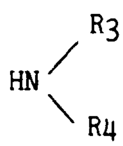

(VIII)

dans laquelle $R_3$ et $R_4$ ont la même signification que dans les composés de formule (I) en milieu organique, éventuellement en présence d'une amine basique comme par exemple la diisopropylamine et à une température comprise entre la température ambiante et le reflux du solvant choisi de manière à obtenir après refroidissement, extraction et éventuelle purification les composés de formule générale ($I_A$) tels que définis précédemment,

c) soit dans le cas des composés de formule générale ($I_A$) pour lesquel A est un chainon méthylène - $CH_2$ -, en milieu organique et à une température amgiante et le reflux du solvant (ou mélange de solvants) choisi avec du chlorométhyl phényl sulfure de manière à obtenir les composés de formule générale (IX) :

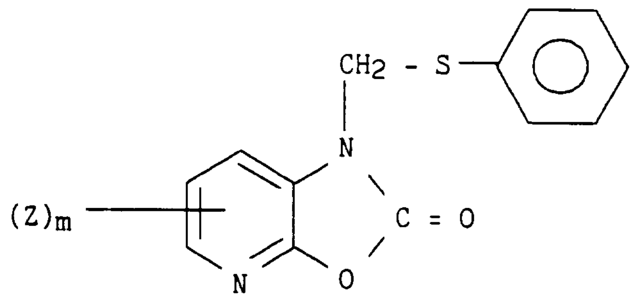

(IX)

dans laquelle Z et m ont la même significaiton que dans les composés de formule générale (I) que l'on fait ensuite réagir en milieu organique avec du chlorure de sulfuryle pour obtenir après purification le composé chloré de formule générale (X) :

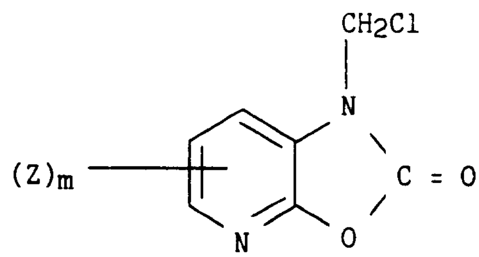

(X)

dans laquelle Z et m ont la même signification que dans les composés de formule générale (I) que l'on fait ensuite réagir comme dans le cas des composés de formule générale (VII) avec des composés de formule générale (VIII) de manière à obtenir les composés de formule générale ($I_A$) tels que définis précedemment pour lesquels A est un chainon méthylène - $CH_2$ -,

B) soit dans le cas des composés de formule générale ($I_A$) pour lesquels A est un chainon méthylène - $CH_2$ -, on condense en milieu d'alcool aliphatique inférieur un composé de formule générale (III), avec une amine de formule générale (VIII) en léger excès et un excès de formol à une température comprise entre la température ambiante et le reflux du milieu réactionnel, les produits obtenus de formule générale ($I_A$) pouvant éventuellement, après isolement, être purifiés par chromatographie sur colonne de silice,

C) soit dans le cas des composés de formule générale (I) pour lesquels $R_1$ et $R_2$ représentent chacun un atome d'hydrogène, on traite les composés ($I_A$) par un agent alcalin comme par exemple l'hydroxyde de sodium en solution aqueuse à une température comprise entre la température ambiante et la température de reflux du milieu réactionnel de manière à obtenir après éventuelle acidification et/ou neutralisation du milieu réactionnel les composés de formule générale ($I_B$) :

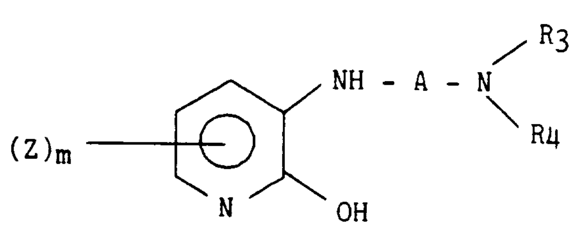

($I_B$)

cas particulier des composés de formule générale (I) pour lesquels $R_1$ = $R_2$ = H, A, $R_3$, $R_4$, Z et m ont la même signification que dans les dérivés de formule générale (I),

étant entendu que les composés de formule ($I_A$) et ($I_B$) ainsi obtenus peuvent si on le désire être séparés en leurs isomères et/ou être salifiés par un acide ou une base pharmaceutiquement acceptable.

2. Procédé de préparation, selon la revendication 1, des composés pour lesquels $R_1$ et $R_2$ forment avec l'oxygène et l'azote qui les portent un chainon - O - CO - N -, de formule générale ($I_A$) :

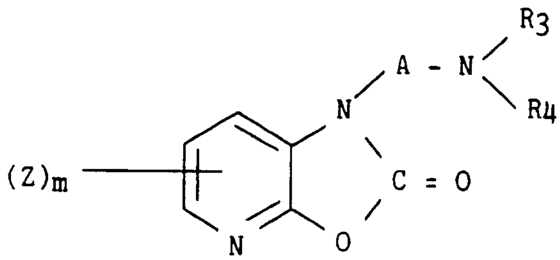

($I_A$)

Z, m, A, $R_3$, $R_4$ ayant la même signification que dans la revendication 1, ainsi que de leurs isomères, épimères, diastéréoisomères, et de leurs sels d'addition à un acide pharmaceutiquement acceptable.

3. Procédé de préparation, selon la revendication 1, des composés pour lesquels $R_1$ et $R_2$ représentent chacun un atome d'hydrogène, de formule générale ($I_B$) :

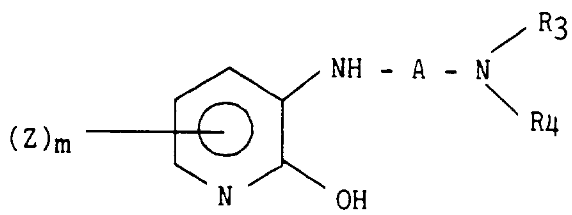

($I_B$)

Z, m, A, $R_3$, $R_4$ ayant la même signification que dans la revendication 1, ainsi que de leurs isomères, épimères, diastéréoisomères, et de leurs sels d'addition à un acide ou à une base pharmaceutiquement acceptable.

4. Procédé de préparation selon la revendication 1 des composés, de formule générale (I), pour lesquels m = 0 et A est un radical alkyl linéaire de 2 à 6 atomes de carbone.

5. Procédé de préparation selon la revendication 1 du composé qui est la 1-[2-(4-phényl pipérazin-1-yl) éthyl] [1H] oxazolo [5,4-b] pyridin-2-one dont la formule est représentée ci dessous, ainsi que de ses sels d'addition à un acide pharmaceutiquement acceptable.

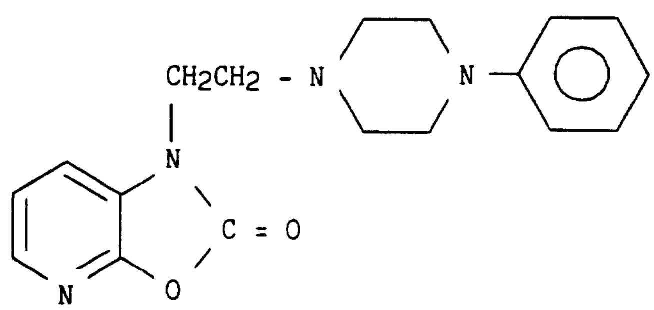

6. Procédé de préparation selon la revendication 1 du composé qui est la 1-[2-(4-(3-trifluorométhyl phényl) pipérazin-1-yl)éthyl] [1H] oxazolo [5,4-b] pyridin-2-one dont la formule est représentée ci dessous, ainsi que de ses sels d'addition à un acide pharmaceutiquement acceptable.

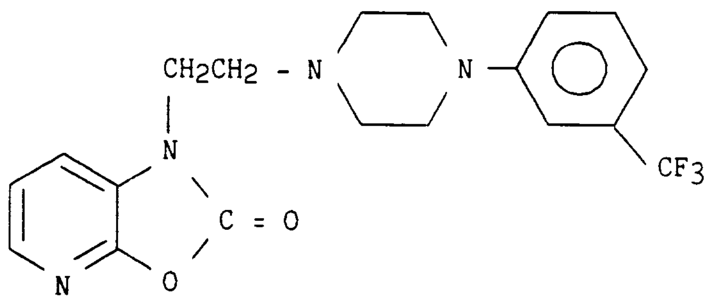

**7.** Procédé de préparation selon la revendication 1 du composé qui est la 1-[2-morpholino éthyl] [1H] oxazolo [5,4-b] pyridin-2-one dont la formule est représentée ci dessous, ainsi que de ses sels d'addition à un acide pharmaceutiquement acceptable.

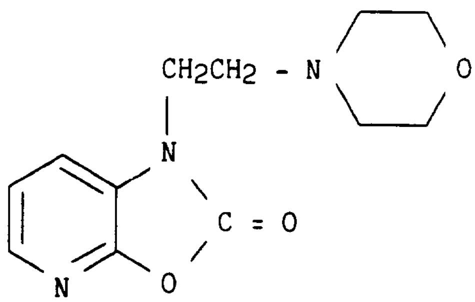

**8.** Procédé de préparation selon la revendication 1 du composé qui est la 1-[3-morpholino prop-1-yl] [1H] oxazolo [5,4-b] pyridin-2-one dont la formule est représentée ci dessous, ainsi que de ses sels d'addition à un acide pharmaceutiquement acceptable.

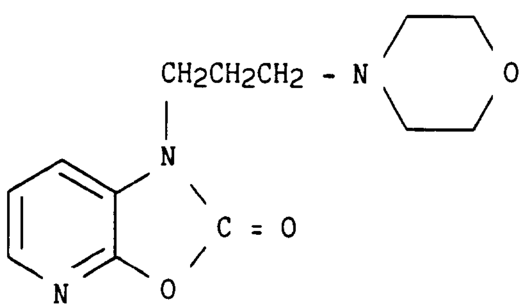

**9.** Procédé de préparation selon la revendication 1 du composé qui est la 1-[4-morpholino but-1-yl] [1H] oxazolo [5,4-b] pyridin-2-one dont la formule est représentée ci dessous, ainsi que de ses sels d'addition à un acide pharmaceutiquement acceptable.

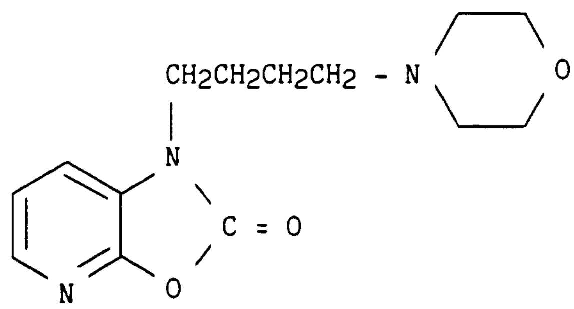

10. Procédé de préparation selon la revendication 1 du composé qui est la 1-[3-(4-phényl pipérazin-1-yl) prop-1-yl] [1H] oxazolo [5,4-b] pyridin-2-one dont la formule est représentée ci dessous, ainsi que de ses sels d'addition à un acide pharmaceutiquement acceptable.

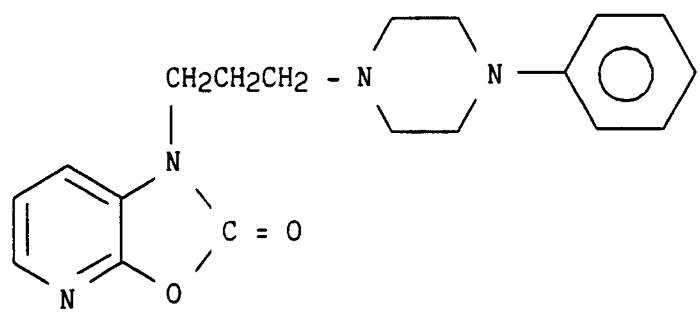

11. Procédé de préparation selon la revendication 1 du composé qui est la 1-[4-(4-phényl pipérazin-1-yl) but-1-yl] [1H] oxazolo [5,4-b] pyridin-2-one dont la formule est représentée ci dessous, ainsi que de ses sels d'addition à un acide pharmaceutiquement acceptable.

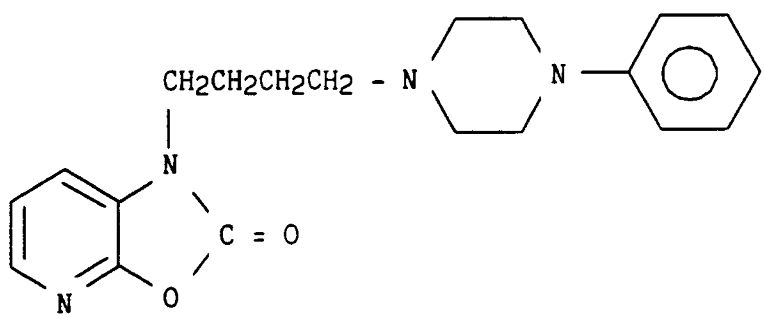

12. Procédé de préparation selon la revendication 1 du composé qui est la 1-[2-(4-(4-fluorophényl) pipérazin-1-yl) éthyl] [1H] oxazolo [5,4-b] pyridin-2-one dont la formule est représentée ci dessous, ainsi que de ses sels d'addition à un acide pharmaceutiquement acceptable.

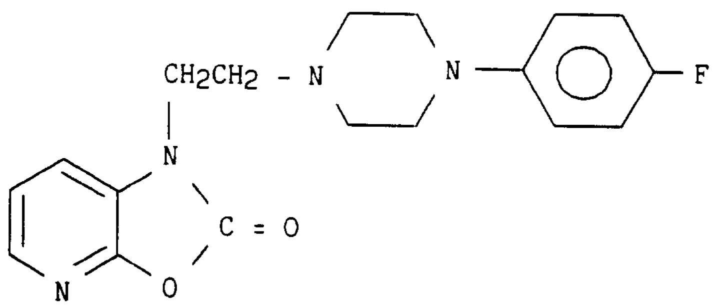

**13.** Procédé de préparation selon la revendication 1 du composé qui est la 1-[2-(pyrrolidin-1-yl) éthyl] [1H] oxazolo [5,4-b] pyridin-2-one dont la formule est représentée ci dessous, ainsi que de ses sels d'addition à un acide pharmaceutiquement acceptable.

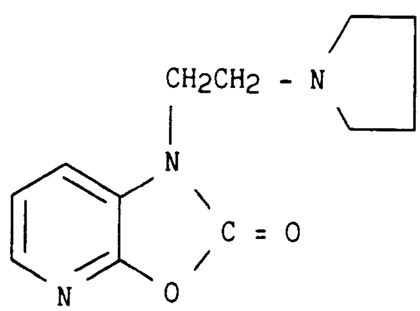

**14.** Procédé de préparation selon la revendication 1 du composé qui est la 1-[2-(4-phényl pipéridin-1-yl) éthyl] [1H] oxazolo [5,4-b] pyridin-2-one dont la formule est représentée ci dessous, ainsi que de ses sels d'addition à un acide pharmaceutiquement acceptable.

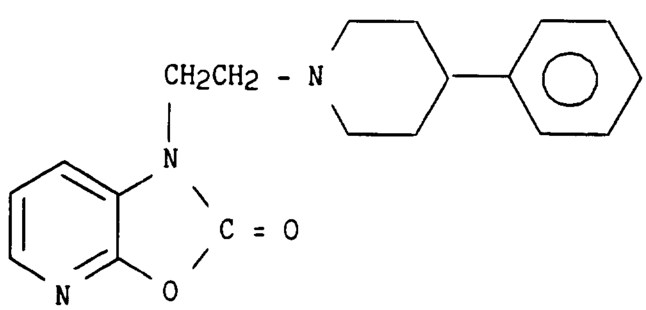

**15.** Procédé de préparation selon la revendication 1 du composé qui est la 1-[2-(4-N,N-diéthyl aminocarbonyl)pipérazin-1-yl) éthyl] [1H] oxazolo [5,4-b] pyridin-2-one dont la formule est représentée ci dessous, ainsi que de ses sels d'addition à un acide ou à une base pharmaceutiquement acceptable.

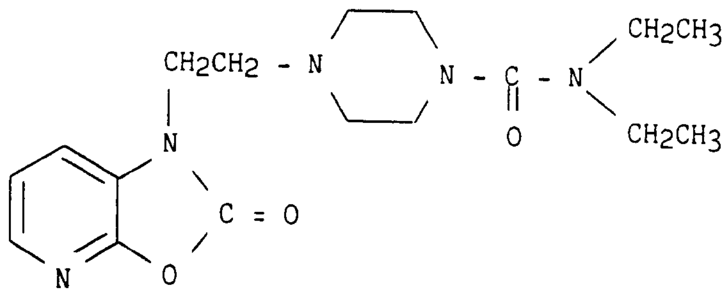

**16.** Procédé de préparation selon la revendication 1 du composé qui est la 3-[2-(4-phényl pipérazin-1-yl) éthylamino] 2-hydroxy pyridine dont la formule est représentée ci dessous, ainsi que de ses sels d'addition à un acide ou à une base pharmaceutiquement acceptable.

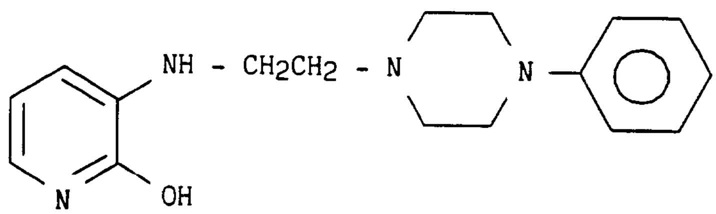

**17.** Procédé de préparation de compositions pharmaceutiques contenant comme principe actif au moins un composé préparé selon la revendication 1 en combinaison avec un ou plusieurs excipients ou véhicules inertes, non toxiques pharmaceutiquement acceptables

**18.** Procédé de préparation de compositions pharmaceutiques selon la revendication 17 utilisables dans le traitement d'algies.

**Revendications pour l'Etat contractant suivant : ES**

**1.** Procédé de préparation de nouveaux composés de formule générale (I) :

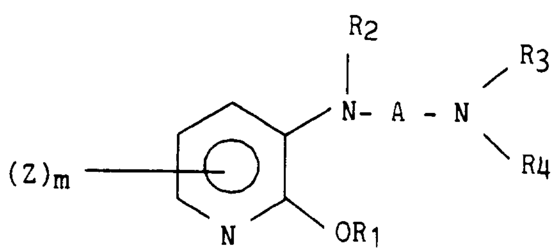

(I)

dans laquelle :

- $R_1$ et $R_2$ représentent chacun un atome d'hydrogène ou forment avec l'oxygène et l'azote qui les portent un enchainement - O - CO - N - ce qui correspond aux oxazolo [5,4 b] pyridin-2-ones,
- Z représente un atome d'halogène, un groupement alkyle inférieur linéaire ou ramifié comprenant de 1 à 6 atomes de carbone, un groupement alcoxy inférieur linéaire ou ramifié comprenant de 1 à 6 atomes de carbone, un groupement trifluorométhyle,
- m nombre entier peut prendre les valeurs 0, 1, 2, 3,
- A est un radical alkyl linéaire ou ramifié comprenant de 1 à 6 atomes de carbone,

- $R_3$ et $R_4$ identiques ou différents représentent :
  - un atome d'hydrogène,
  - un groupement alkyle inférieur linéaire ou ramifié, comprenant de 1 à 6 atomes de carbone,
  - un groupement alkényle inférieur linéaire ou ramifié, comprenant de 1 à 6 atomes de carbone,
  - un groupement aryle ou hétéroaryle éventuellement substitué,
  - un groupement arylalkyle ou hétéroarylalkyle éventuellement substitué dont la chaine alkyle comprend de 1 à 3 atomes de carbone,
  - un groupement cycloalkyle mono ou bicyclique de 3 à 10 atomes de carbone ,

  ou bien :

  $R_3$ et $R_4$ constituent avec l'atome d'azote auquel ils sont liés un système hétérocyclique azoté, mono ou bicyclique, saturé ou insaturé, comprenant un maximum de 12 atomes non compris les atomes d'hydrogène parmi lesquels on peut compter de un à trois hétéroatomes choisis parmi azote, oxygène ou soufre et éventuellement substitué par un ou des :
  - groupement hydroxy,
  - groupement oxo,
  - groupement alkyle inférieur, linéaire ou ramifié, comprenant de 1 à 6 atomes de carbone,
  - groupement aryle éventuellement substitué,
  - groupement arylalkyle éventuellement substitué ou diarylalkyle éventuellement substitué dont la chaine alkyle comporte de 1 à 3 atomes de carbone,
  - groupement -CO - $OR_5$
  - groupement

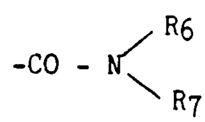

- $R_5$ représente :
  - un atome d'hydrogène,
  - un groupement alkyle inférieur linéaire ou ramifié comprenant de 1 à 6 atomes de carbone,
  - un groupement aryle éventuellement substitué,
  - un groupement aralkyle éventuellement substitué dont la chaine alkyle comprend de 1 à 3 atomes de carbone,

  $R_6$ et $R_7$ identiques ou différents ont la même signification que $R_5$, étant entendu que :
- par groupement aryle on entend un groupement mono ou bicyclique insaturé ou aromatique comprenant de 5 à 12 atomes de carbone,
- par groupement hétéroaryle on entend un groupement mono ou bicyclique, insaturé ou aromatique, comprenant de 5 à 12 atomes non compris les atomes d'hydrogène et intégrant dans son squelette carboné un, deux ou trois hétéroatomes choisis parmi azote, oxygène ou soufre,
- le terme substitué associé aux expressions aryle, arylalkyle, diarylalkyle, hétéroaryle, hétéroarylalkyle signifie que le, ou les, noyaux aryle ou hétéroaryle peuvent être substitués par un ou plusieurs groupement alkyle inférieur de 1 à 6 atomes de carbone linéaire ou ramifié, alcoxy inférieur de 1 à 6 atomes de carbone linéaire ou ramifié, hydroxy, nitro, halogène, trifluorométhyle,

ainsi que de leurs isomères, épimères, diastéréoisomères et de leurs sels d'addition à un acide pharmaceutiquement acceptable et/ou lorsque $R_1$ et $R_2$ représentent chacun un atome d'hydrogène, leurs sels d'addition à une base pharmaceutiquement acceptable,

caractérisé en ce que :

A) Soit l'on fait réagir une 3-amino 2-pyridinone de formule générale (II) :

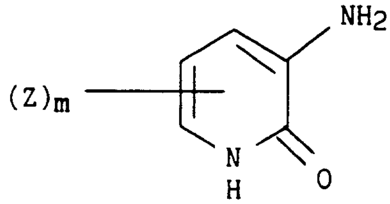

(II)

dans laquelle Z et m ont la même signification que dans les composés de formule générale (I), en solution à -78° C (température du mélange acétone carboglace) dans un solvant ou un mélange de solvants aprotiques avec du bis (trichlorométhyl) carbonate (triphosgène) en présence d'une amine basique comme par exemple la triéthylamine de manière à obtenir une [1H] oxazolo [5,4-b] pyridin-2-one de formule générale (III) :

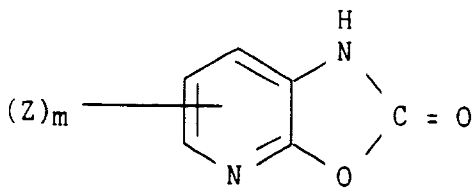

(III)

dans laquelle Z et m ont la même signification que dans les composés de formule générale (I), que l'on fait réagir avec un alcoolate ou un hydrure de métal alcalin en milieu organique aprotique de manière à obtenir le composé de formule générale (IV) :

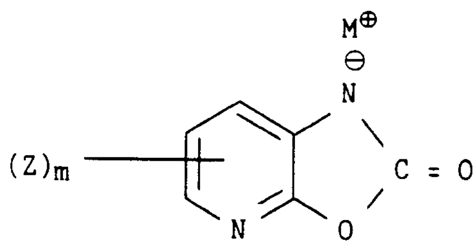

(IV)

dans laquelle Z et m ont la même signification que dans les composés de formule générale (I) et M représente un métal alcalin, que l'on fait réagir :

a) soit en milieu organique et à une température comprise entre la température ambiante et le reflux du solvant (ou mélange de solvants) choisi avec un composé électrophile (préférentiellement en excès) de formule générale (V):

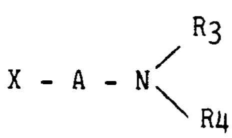

(V)

dans laquelle X représente un atome d'halogène et A, $R_3$, $R_4$ ont la même signification que dans les composés de formule générale (I), de manière à obtenir après refroidissement extraction et éventuelle purification, les composés de formule générale ($I_A$) :

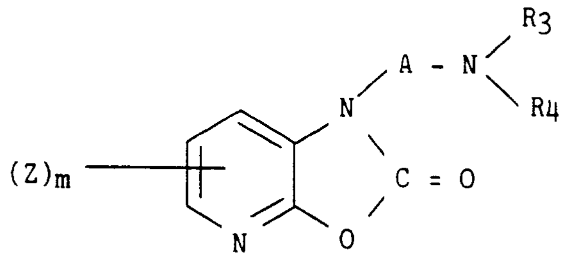

(IA)

dans laquelle A, $R_3$, $R_4$, Z et m ont la même signification que dans les composés de formule générale (I),

b) soit en milieu organique et à une température comprise entre la température ambiante et le reflux du solvant (ou mélange de solvants) choisi avec un composé électrophile (préférentiellement en excès) de formule générale (VI) :

X - A - X' (VI)

dans laquelle X et X' identiques ou différents représentent chacun un atome d'halogène et A a la même définition que dans les composés de formule générale (I), de manière à obtenir le composé halogéné de formule générale (VII) :

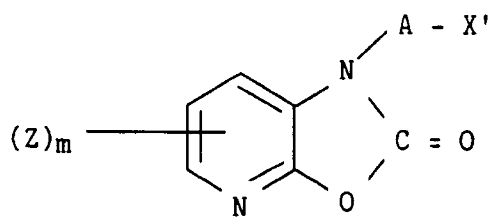

(VII)

dans laquelle X', A, Z et m ont la même signification que précedemment, que l'on fait finalement réagir avec une amine (préférentiellement en excès) de formule générale (VIII) :

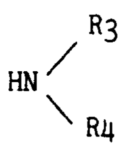

(VIII)

dans laquelle $R_3$ et $R_4$ ont la même signification que dans les composés de formule (I) en milieu organique, éventuellement en présence d'une amine basique comme par exemple la diisopropylamine et à une température comprise entre la température ambiante et le reflux du solvant choisi de manière à obtenir après refroidissement, extraction et éventuelle purification les composés de formule générale ($I_A$) tels que définis précédemment,

c) soit dans le cas des composés de formule générale ($I_A$) pour lesquel A est un chainon méthylène - $CH_2$ -, en milieu organique et à une température amgiante et le reflux du solvant (ou mélange de solvants) choisi avec du chlorométhyl phényl sulfure de manière à obtenir les composés de formule générale (IX) :

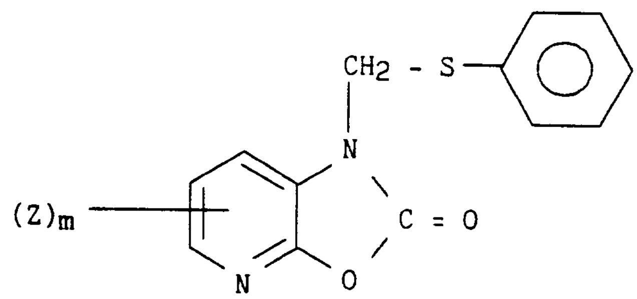

(IX)

dans laquelle Z et m ont la même significaiton que dans les composés de formule générale (I) que l'on fait ensuite réagir en milieu organique avec du chlorure de sulfuryle pour obtenir après purification le composé chloré de formule générale (X) :

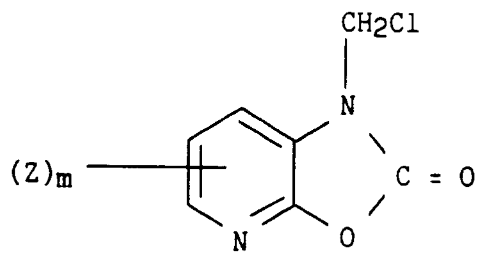

(X)

dans laquelle Z et m ont la même signification que dans les composés de formule générale (I) que l'on fait ensuite réagir comme dans le cas des composés de formule générale (VII) avec des composés de formule générale (VIII) de manière à obtenir les composés de formule générale ($I_A$) tels que définis précedemment pour lesquels A est un chainon méthylène - $CH_2$ -,

B) soit dans le cas des composés de formule générale ($I_A$) pour lesquels A est un chainon méthylène - $CH_2$ -, on condense en milieu d'alcool aliphatique inférieur un composé de formule générale (III), avec une amine de formule générale (VIII) en léger excès et un excès de formol à une température comprise entre la température ambiante et le reflux du milieu réactionnel, les produits obtenus de formule générale ($I_A$) pouvant éventuellement, après isolement, être purifiés par chromatographie sur colonne de silice,

C) soit dans le cas des composés de formule générale (I) pour lesquels $R_1$ et $R_2$ représentent chacun un atome d'hydrogène, on traite les composés ($I_A$) par un agent alcalin comme par exemple l'hydroxyde de sodium en solution aqueuse à une température comprise entre la température ambiante et la température de reflux du milieu réactionnel de manière à obtenir après éventuelle acidification et/ou neutralisation du milieu réactionnel les composés de formule générale ($I_B$) :

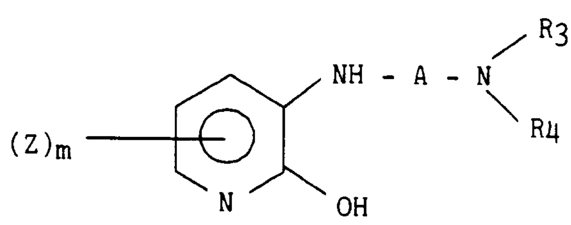

($I_B$)

cas particulier des composés de formule générale (I) pour lesquels $R_1 = R_2 = H$, A, $R_3$, $R_4$, Z et m ont la même signification que dans les dérivés de formule générale (I),

étant entendu que les composés de formule ($I_A$) et ($I_B$) ainsi obtenus peuvent si on le désire être séparés en leurs isomères et/ou être salifiés par un acide ou une base pharmaceutiquement acceptable.

**2.** Procédé de préparation, selon la revendication 1, des composés pour lesquels $R_1$ et $R_2$ forment avec l'oxygène et l'azote qui les portent un chainon - O - CO - N -, de formule générale ($I_A$) :

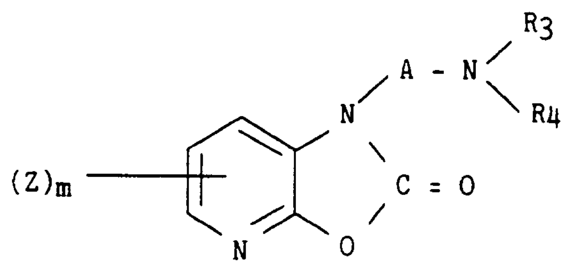

($I_A$)

Z, m, A, $R_3$, $R_4$ ayant la même signification que dans la revendication 1, ainsi que de leurs isomères, épimères, diastéréoisomères, et de leurs sels d'addition à un acide pharmaceutiquement acceptable.

3. Procédé de préparation, selon la revendication 1, des composés pour lesquels $R_1$ et $R_2$ représentent chacun un atome d'hydrogène, de formule générale ($I_B$) :

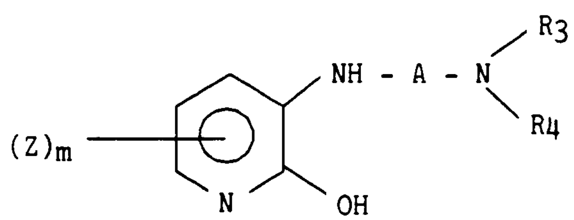

($I_B$)

Z, m, A, $R_3$, $R_4$ ayant la même signification que dans la revendication 1, ainsi que de leurs isomères, épimères, diastéréoisomères, et de leurs sels d'addition à un acide ou à une base pharmaceutiquement acceptable.

4. Procédé de préparation selon la revendication 1 des composés, de formule générale (I), pour lesquels m = 0 et A est un radical alkyl linéaire de 2 à 6 atomes de carbone.

5. Procédé de préparation selon la revendication 1 du composé qui est la 1-[2-(4-phényl pipérazin-1-yl) éthyl] [1H] oxazolo [5,4-b] pyridin-2-one dont la formule est représentée ci dessous, ainsi que de ses sels d'addition à un acide pharmaceutiquement acceptable.

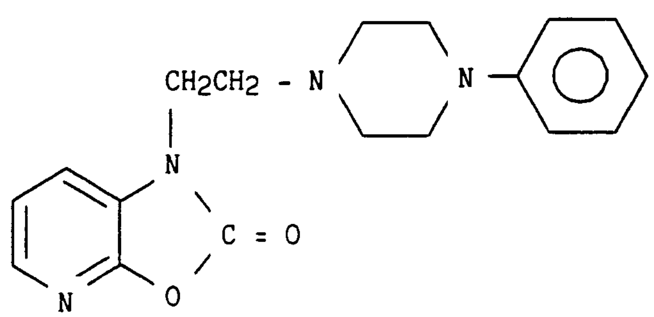

6. Procédé de préparation selon la revendication 1 du composé qui est la 1-[2-(4-(3-trifluorométhyl phényl) pipérazin-1-yl)éthyl] [1H] oxazolo [5,4-b] pyridin-2-one dont la formule est représentée ci dessous, ainsi que de ses sels d'addition à un acide pharmaceutiquement acceptable.

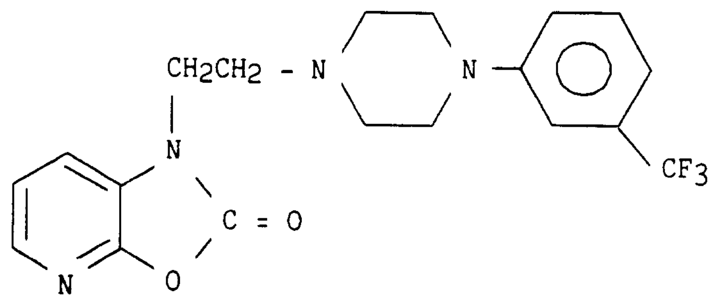

7. Procédé de préparation selon la revendication 1 du composé qui est la 1-[2-morpholino éthyl] [1H] oxazolo [5,4-b] pyridin-2-one dont la formule est représentée ci dessous, ainsi que de ses sels d'addition à un acide pharmaceutiquement acceptable.

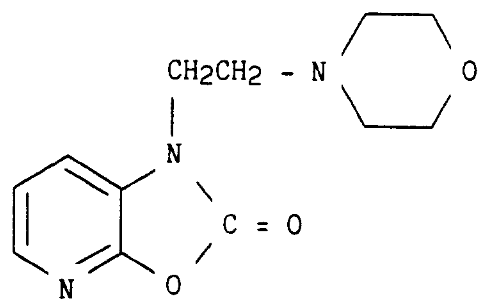

8. Procédé de préparation selon la revendication 1 du composé qui est la 1-[3-morpholino prop-1-yl] [1H] oxazolo [5,4-b] pyridin-2-one dont la formule est représentée ci dessous, ainsi que de ses sels d'addition à un acide pharmaceutiquement acceptable.

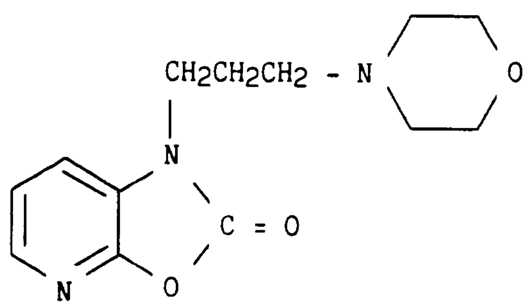

9. Procédé de préparation selon la revendication 1 du composé qui est la 1-[4-morpholino but-1-yl] [1H] oxazolo [5,4-b] pyridin-2-one dont la formule est représentée ci dessous, ainsi que de ses sels d'addition à un acide pharmaceutiquement acceptable.

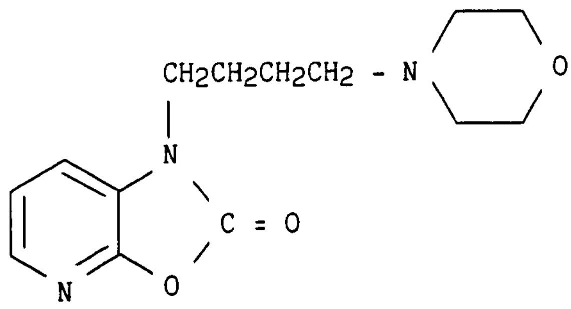

**10.** Procédé de préparation selon la revendication 1 du composé qui est la 1-[3-(4-phényl pipérazin-1-yl) prop-1-yl] [1H] oxazolo [5,4-b] pyridin-2-one dont la formule est représentée ci dessous, ainsi que de ses sels d'addition à un acide pharmaceutiquement acceptable.

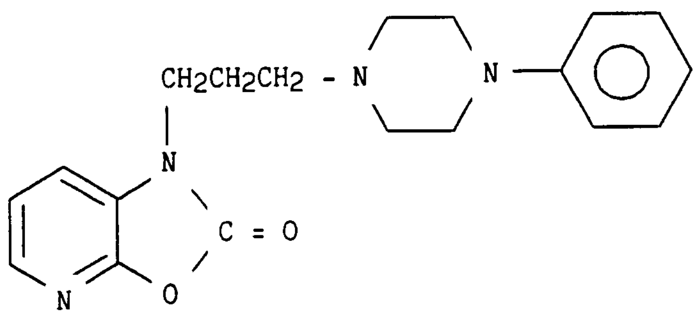

**11.** Procédé de préparation selon la revendication 1 du composé qui est la 1-[4-(4-phényl pipérazin-1-yl) but-1-yl] [1H] oxazolo [5,4-b] pyridin-2-one dont la formule est représentée ci dessous, ainsi que de ses sels d'addition à un acide pharmaceutiquement acceptable.

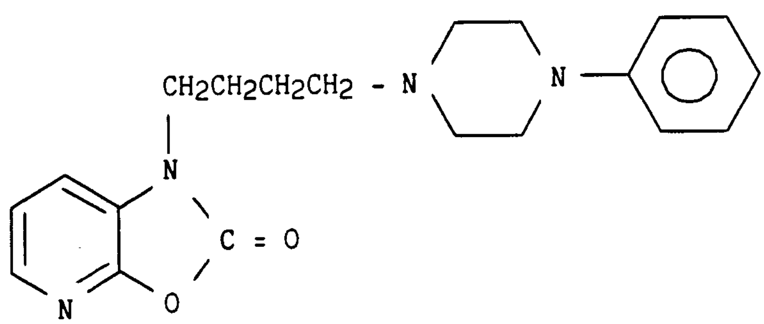

**12.** Procédé de préparation selon la revendication 1 du composé qui est la 1-[2-(4-(4-fluorophényl) pipérazin-1-yl) éthyl] [1H] oxazolo [5,4-b] pyridin-2-one dont la formule est représentée ci dessous, ainsi que de ses sels d'addition à un acide pharmaceutiquement acceptable.

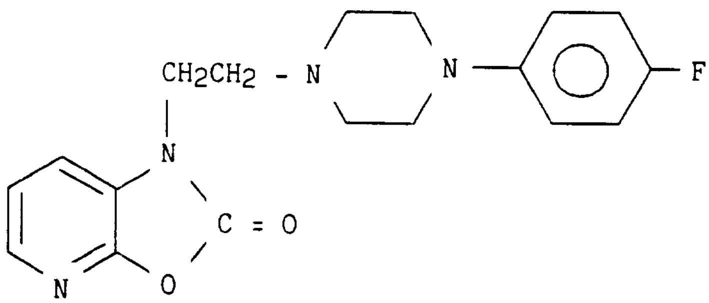

13. Procédé de préparation selon la revendication 1 du composé qui est la 1-[2-(pyrrolidin-1-yl) éthyl] [1H] oxazolo [5,4-b] pyridin-2-one dont la formule est représentée ci dessous, ainsi que de ses sels d'addition à un acide pharmaceutiquement acceptable.

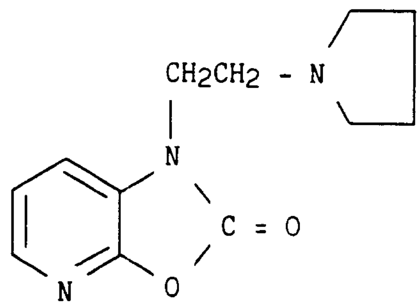

14. Procédé de préparation selon la revendication 1 du composé qui est la 1-[2-(4-phényl pipéridin-1-yl) éthyl] [1H] oxazolo [5,4-b] pyridin-2-one dont la formule est représentée ci dessous, ainsi que de ses sels d'addition à un acide pharmaceutiquement acceptable.

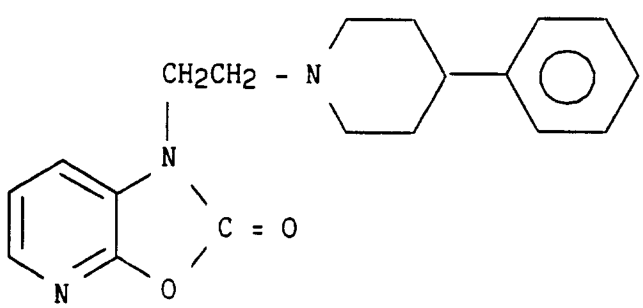

15. Procédé de préparation selon la revendication 1 du composé qui est la 1-[2-(4-N,N-diéthyl aminocarbonyl)pipérazin-1-yl) éthyl] [1H] oxazolo [5,4-b] pyridin-2-one dont la formule est représentée ci dessous, ainsi que de ses sels d'addition à un acide ou à une base pharmaceutiquement acceptable.

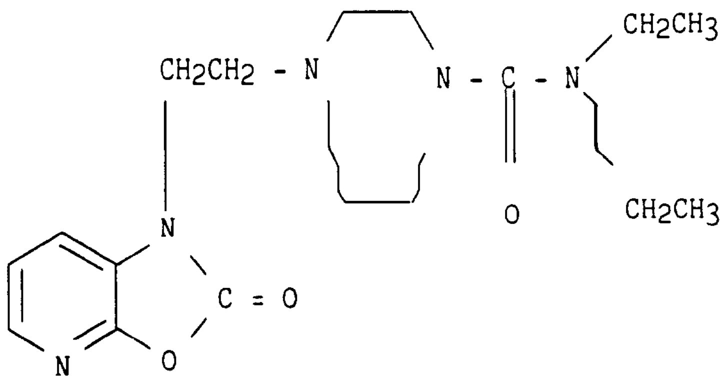

16. Procédé de préparation selon la revendication 1 du composé qui est la 3-[2-(4-phényl pipérazin-1-yl) éthylamino] 2-hydroxy pyridine dont la formule est représentée ci dessous, ainsi que de ses sels d'addition à un acide ou à une base pharmaceutiquement acceptable.

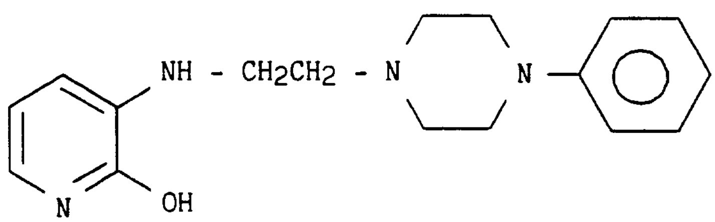

17. Procédé de préparation de compositions pharmaceutiques contenant comme principe actif au moins un composé préparé selon la revendication 1 en combinaison avec un ou plusieurs excipients ou véhicules inertes, non toxiques pharmaceutiquement acceptables

18. Procédé de préparation de compositions pharmaceutiques selon la revendication 17 utilisables dans le traitement d'algies.

**Claims**

**Claims for the following Contracting States : AT, BE, CH, DE, DK, FR, GB, IT, LI, LU, NL, SE**

1. Compounds of the general formula (I):

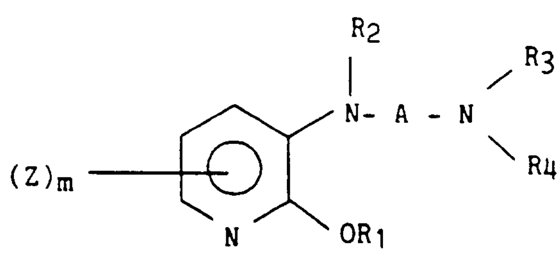

(I)

in which:

- $R_1$ and $R_2$ each represent a hydrogen atom or, with the oxygen and the nitrogen carrying them, form a chain -O-CO-N-, which corresponds to the oxazolo[5,4-b]pyridin-2-ones,
- Z represents a halogen atom, a linear or branched lower alkyl group containing from 1 to 6 carbon atoms, a linear or branched lower alkoxy group containing from 1 to 6 carbon atoms, or a trifluoromethyl group,

- m, which is an integer, may be 0, 1, 2 or 3,
- A is a linear or branched alkyl radical containing from 1 to 6 carbon atoms,
- $R_3$ and $R_4$, which may be the same or different, represent:
  - a hydrogen atom,
  - a linear or branched lower alkyl group containing from 1 to 6 carbon atoms,
  - a linear or branched lower alkenyl group containing from 1 to 6 carbon atoms,
  - an optionally substituted aryl or heteroaryl group,
  - an optionally substituted arylalkyl or heteroarylalkyl group, the alkyl chain of which contains from 1 to 3 carbon atoms, or
  - a mono- or bi-cyclic cycloalkyl group having from 3 to 10 carbon atoms,

  or:
- $R_3$ and $R_4$, with the nitrogen atom to which they are bonded, form a mono- or bi-cyclic, saturated or unsaturated, nitrogen-containing heterocyclic system that contains not more than 12 atoms, not including the hydrogen atoms, which may include from one to three hetero atoms selected from nitrogen, oxygen and sulphur, and that is optionally substituted by one or more:
  - hydroxy groups,
  - oxo groups,
  - linear or branched lower alkyl groups containing from 1 to 6 carbon atoms,
  - optionally substituted aryl groups,
  - optionally substituted arylalkyl or optionally substituted diarylalkyl groups, the alkyl chain of which contains from 1 to 3 carbon atoms,
  - -CO-$OR_5$ groups,
  -

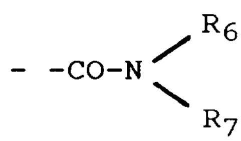

groups,
- $R_5$ represents:
  - a hydrogen atom,
  - a linear or branched lower alkyl group containing from 1 to 6 carbon atoms,
  - an optionally substituted aryl group, or
  - an optionally substituted arylalkyl group, the alkyl chain of which contains from 1 to 3 carbon atoms,
  - $R_6$ and $R_7$, which may be the same or different, have the same meaning as $R_5$,
- their isomers, epimers and diastereoisomers,
- their addition salts with a pharmaceutically acceptable acid and/or, when $R_1$ and $R_2$ each represent a hydrogen atom, their addition salts with a pharmaceutically acceptable base,
- "aryl group" denotes a mono- or bi-cyclic, unsaturated or aromatic group containing from 5 to 12 carbon atoms,
- "heteroaryl group" denotes a mono- or bi-cyclic, unsaturated or aromatic group that contains from 5 to 12 atoms, not including the hydrogen atoms, and that includes in its carbon skeleton one, two or three hetero atoms selected from nitrogen, oxygen and sulphur,
- the term "substituted" associated with the expressions aryl, arylalkyl, diarylalkyl, heteroaryl and heteroarylalkyl indicates that the aryl or heteroaryl ring, or rings, may be substituted by one or more groups linear or branched lower alkyl having from 1 to 6 carbon atoms, linear or branched lower alkoxy having from 1 to 6 carbon atoms, hydroxy, nitro, halogen, trifluoromethyl.

**2.** Compounds according to claim 1, in which $R_1$ and $R_2$, with the oxygen and the nitrogen carrying them, form a chain -O-CO-N-, of the general formula ($I_A$):

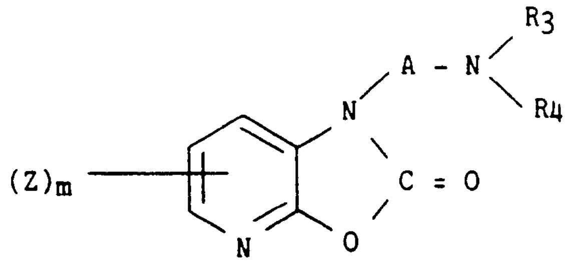

$(I_A)$,

Z, m, A, $R_3$ and $R_4$ having the same meaning as in claim 1, their isomers, epimers and diastereoisomers, and also their addition salts with a pharmaceutically acceptable acid.

3. Compounds according to claim 1, in which $R_1$ and $R_2$ each represent a hydrogen atom, of the general formula ($I_B$):

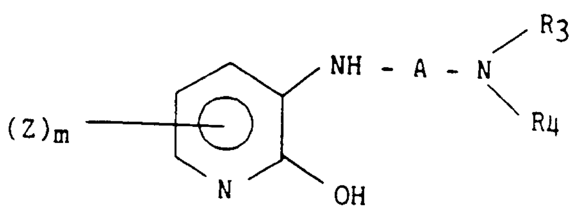

$(I_B)$,

Z, m, A, $R_3$ and $R_4$ having the same meaning as in claim 1, their isomers, epimers and diastereoisomers, and also their addition salts with a pharmaceutically acceptable acid or base.

4. Compounds according to claim 1, in which m = 0 and A is a linear alkyl radical having from 2 to 6 carbon atoms.

5. Compound according to claim 1 which is 1-[2-(4-phenylpiperazin-1-yl)ethyl]-[1H]-oxazolo[5,4-b]pyridin-2-one, the formula of which is shown below, and its addition salts with a pharmaceutically acceptable acid

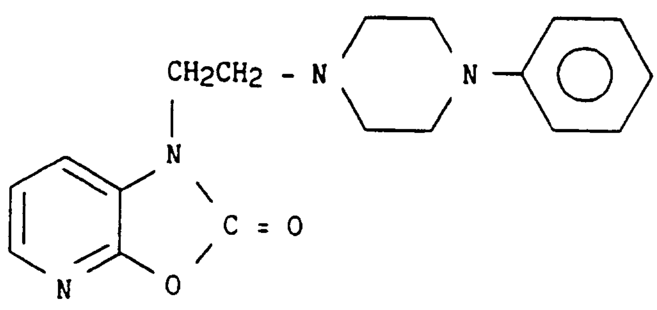

.

6. Compound according to claim 1 which is 1-[2-(4-(3-trifluoromethylphenyl)piperazin-1-yl)ethyl]-[1H]-oxazolo[5,4-b]pyridin-2-one, the formula of which is shown below, and its addition salts with a pharmaceutically acceptable acid

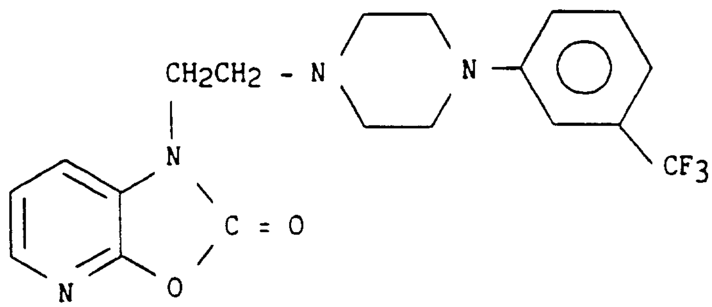

.

7. Compound according to claim 1 which is 1-[2-morpholinoethyl]-[1H]-oxazolo[5,4-b]pyridin-2-one, the formula of which is shown below, and its addition salts with a pharmaceutically acceptable acid

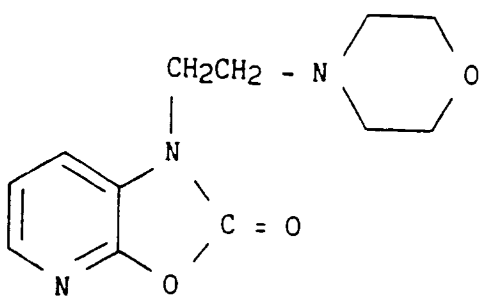

.

8. Compound according to claim 1 which is 1-[3-morpholinoprop-1-yl]-[1H]-oxazolo[5,4-b]pyridin-2-one, the formula of which is shown below, and its addition salts with a pharmaceutically acceptable acid

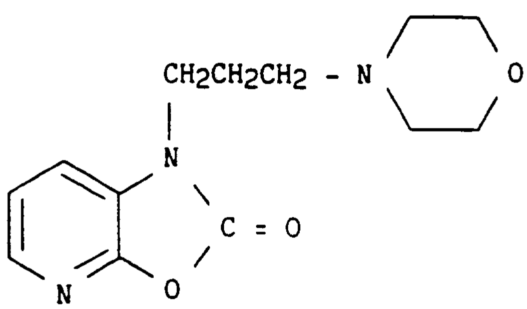

.

9. Compound according to claim 1 which is 1-[4-morpholinobut-1-yl]-[1H]-oxazolo[5,4-b]pyridin-2-one, the formula of which is shown below, and its addition salts with a pharmaceutically acceptable acid

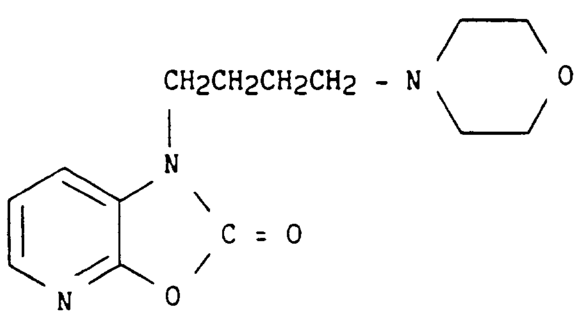

.

10. Compound according to claim 1 which is 1-[3-(4-phenylpiperazin-1-yl)prop-1-yl]-[1H]-oxazolo[5,4-b]-pyridin-2-one, the formula of which is shown below, and its addition salts with a pharmaceutically acceptable acid

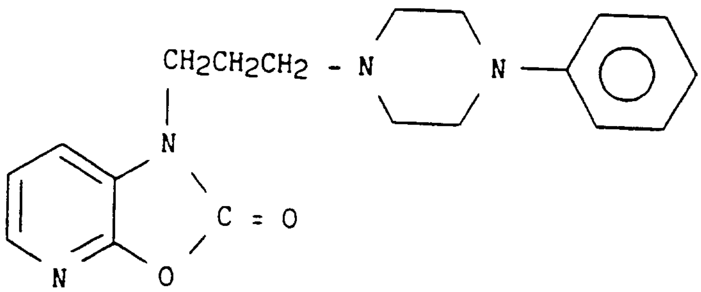

.

11. Compound according to claim 1 which is 1-[4-(4-phenylpiperazin-1-yl)but-1-yl]-[1H]-oxazolo[5,4-b]-pyridin-2-one, the formula of which is shown below, and its addition salts with a pharmaceutically acceptable acid

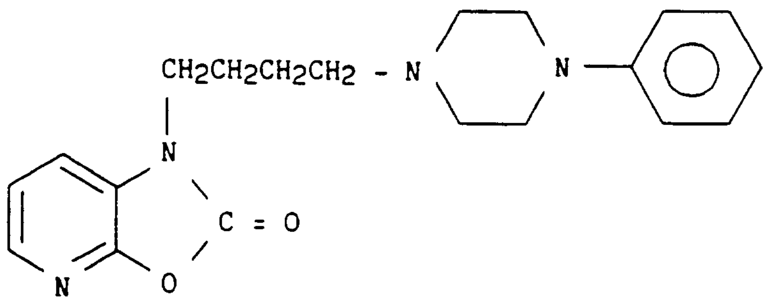

.

12. Compound according to claim 1 which is 1-[2-(4-(4-fluorophenyl)piperazin-1-yl)ethyl]-[1H]-oxazolo[5,4-b]pyridin-2-one, the formula of which is shown below, and its addition salts with a pharmaceutically acceptable acid

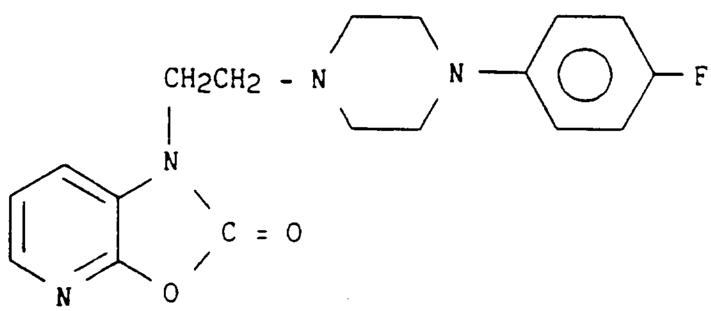

.

13. Compound according to claim 1 which is 1-[2-(pyrrolidin-1-yl)ethyl]-[1H]-oxazolo[5,4-b]pyridin-2-one, the formula of which is shown below, and its addition salts with a pharmaceutically acceptable acid

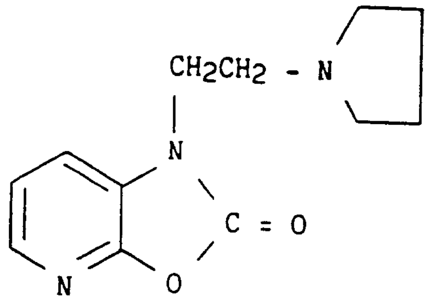 .

14. Compound according to claim 1 which is 1-[2-(4-phenylpiperidin-1-yl)ethyl]-[1H]-oxazolo[5,4-b]pyridin-2-one, the formula of which is shown below, and its addition salts with a pharmaceutically acceptable acid

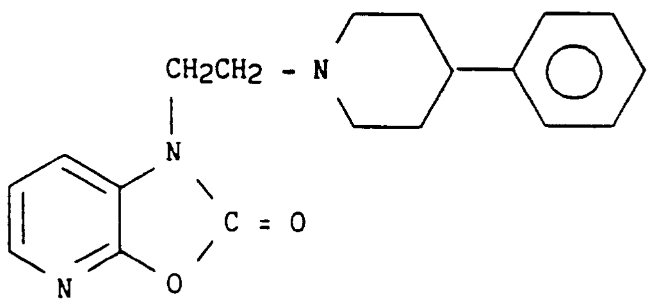 .

15. Compound according to claim 1 which is 1-[2-(4-(N,N-diethylaminocarbonyl)piperazin-1-yl)ethyl]-[1H]-oxazolo[5,4-b]pyridin-2-one, the formula of which is shown below, and its addition salts with a pharmaceutically acceptable acid or base

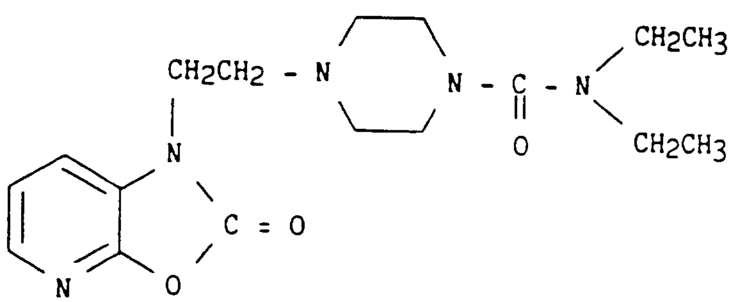 .

16. Compound according to claim 1 which is 3-[2-(4-phenylpiperazin-1-yl)ethylamino]-2-hydroxypyridine, the formula of which is shown below, and its addition salts with a pharmaceutically acceptable acid or base

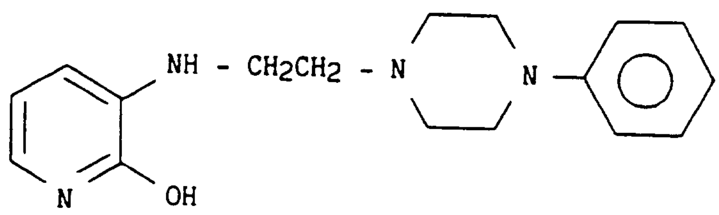

.

17. Process for the preparation of the compounds of the general formula ($I_A$) according to claim 2, characterised in that a 3-amino-2-pyridinone of the general formula (II):

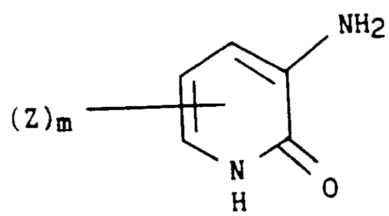

(II),

in which Z and m have the same meaning as in the compounds of the general formula (I), in solution at -78 ° C (temperature of the acetone/solid carbon dioxide mixture) in an aprotic solvent or a mixture of aprotic solvents, is reacted with bis(trichloromethyl) carbonate (triphosgene) in the presence of a basic amine, such as, for example, triethylamine, in such a manner as to obtain a [1H]-oxazolo[5,4-b]pyridin-2-one of the general formula (III):

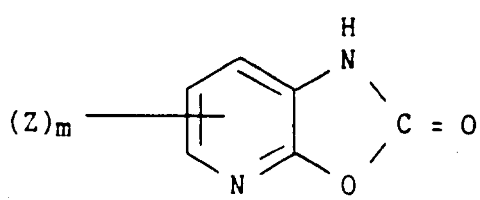

(III),

in which Z and m have the same meaning as in the compounds of the general formula (I), which is reacted with an alkali metal alcoholate or hydride in an aprotic organic medium in such a manner as to obtain the compound of the general formula (IV):

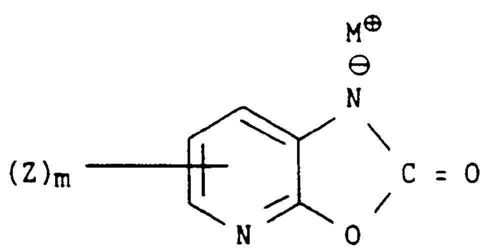

(IV),

in which Z and m have the same meaning as in the compounds of the general formula (I) and M represents an alkali metal, which is reacted, in an organic medium and at a temperature of from room temperature to the reflux temperature of the chosen solvent (or mixture of solvents), with an electrophilic compound (preferably in excess) of the general formula (V):

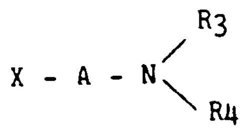 (V),

in which X represents a halogen atom and A, $R_3$ and $R_4$ have the same meaning as in the compounds of the general formula (I), in such a manner as to obtain, after cooling, extraction and, optionally, purification, the compounds of the general formula ($I_A$):

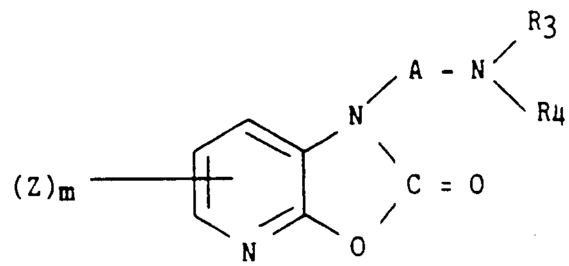 ($I_A$)

such as defined in claim 2, it being understood that the compounds of the general formula ($I_A$) so obtained may, if desired, be separated into their isomers and/or converted into a salt with a pharmaceutically acceptable acid.

**18.** Process for the preparation of the compounds of formula ($I_A$) according to claim 2, characterised in that a compound of the general formula (IV):

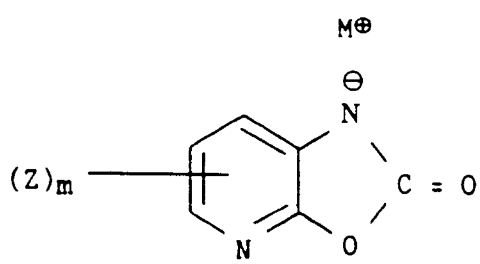 (IV),

in which Z and m have the same meaning as in the compounds of the general formula (I) and M represents an alkali metal, is reacted, in an organic medium and at a temperature of from room temperature to the reflux temperature of the chosen solvent (or mixture of solvents), with an electrophilic compound (preferably in excess) of the general formula (VI):

X - A - X'     (VI),

in which X and X', which may be the same or different, each represent a halogen atom and A has the same definition as in the compounds of the general formula (I), in such a manner as to obtain the halogenated compound of the general formula (VII):

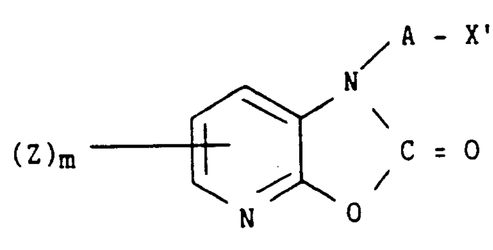 (VII),

in which X', A, Z and m have the same meaning as above, which, finally, is reacted with an amine (preferably in excess) of the general formula (VIII):

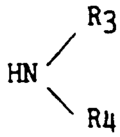

(VIII),

in which $R_3$ and $R_4$ have the same meaning as in the compounds of formula (I), in an organic medium, optionally in the presence of a basic amine, such as, for example, diisopropylamine, and at a temperature of from room temperature to the reflux temperature of the chosen solvent, in such a manner as to obtain, after cooling, extraction and, optionally, purification, the compounds of the general formula ($I_A$) such as defined in claim 2, it being understood that the compounds of formula ($I_A$) so obtained may, if desired, be separated into their isomers and/or converted into a salt with a pharmaceutically acceptable acid.

**19.** Process for the preparation of the compounds of the general formula ($I_A$) according to claim 2 in which A is a methylene chain $-CH_2-$, characterised in that a compound of the general formula (IV):

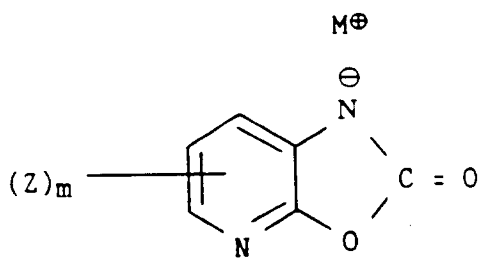

(IV),

in which Z and m have the same meaning as in the compounds of the general formula (I) and M represents an alkali metal, is reacted, in an organic medium and at a temperature of from room temperature to the reflux temperature of the chosen solvent (or mixture of solvents), with chloromethyl phenyl sulphide in such a manner as to obtain the compounds of the general formula (IX):

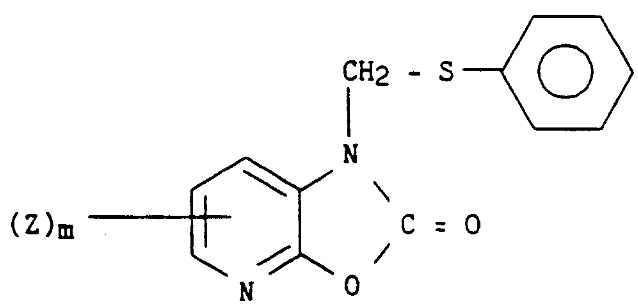

(IX),

in which Z and m have the same meaning as in the compounds of the general formula (I), which are then reacted, in an organic medium, with sulphuryl chloride to obtain, after purification, the chlorinated compound of the general formula (X):

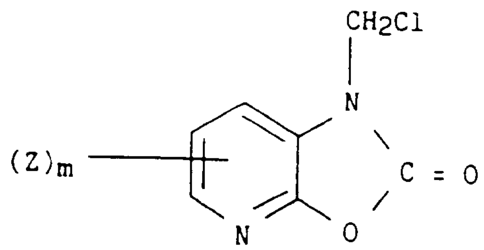

(X),

in which Z and m have the same meaning as in the compounds of the general formula (I), which is then reacted as in claim 18 with compounds of the general formula (VIII) in such a manner as to obtain the compounds of the general formula ($I_A$) such as defined in claim 2 in which A is a methylene chain $-CH_2-$, it being understood that the compounds of formula ($I_A$) so obtained may, if desired, be separated into their isomers and/or converted into a salt with a pharmaceutically acceptable acid.

**20.** Process for the preparation of the compounds of the general formula ($I_A$) according to claim 2 in which A is a methylene chain $-CH_2-$, characterised in that a compound of the general formula (III) such as defined in claim 17 is condensed, in a lower aliphatic alcohol medium, with an amine of the general formula (VIII) such as defined in claim 18 in a slight excess and in the presence of an excess of formaldehyde, at a temperature of from room temperature to the reflux temperature of the reaction medium, in such a manner as to obtain, after cooling, isolation and, optionally, purification by chromatography on a column of silica, the compounds of the general formula ($I_A$) such as defined in claim 2 in which A is a methylene chain $-CH_2-$, it being understood that the compounds of formula ($I_A$) so obtained may, if desired, be separated into their isomers and/or converted into a salt with a pharmaceutically acceptable acid.

**21.** Process for the preparation of the compounds of the general formula ($I_B$) according to claim 3, characterised in that the compounds of the general formula ($I_A$) are treated with an alkaline agent such as, for example, sodium hydroxide in aqueous solution, at a temperature of from room temperature to the reflux temperature of the reaction medium, to yield, after optional acidification and/or neutralisation of the reaction medium, the compounds of the general formula ($I_B$):

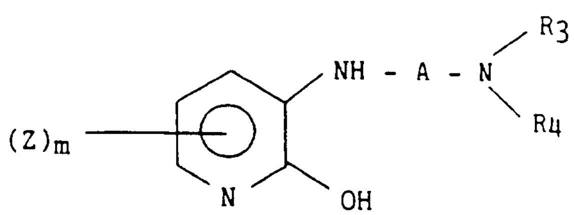

($I_B$),

which are a particular case of the compounds of the general formula (I) in which $R_1 = R_2 = H$ and A, $R_3$, $R_4$, Z and m have the same meaning as in the compounds of the general formula (I).

**22.** Compounds of the general formula (VII):

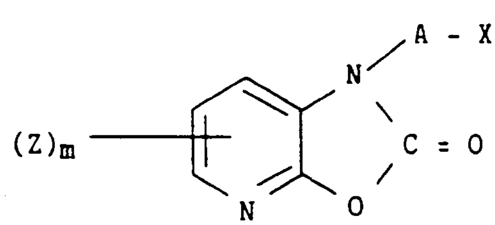

(VII),

in which X' represents a halogen atom, A is a linear or branched alkyl radical containing from 1 to 6

carbon atoms, Z represents a halogen atom, a lower alkyl or alkoxy group containing from 1 to 6 carbon atoms, or a trifluoromethyl group, and m represents an integer of from 0 to 3 inclusive, which can be used as synthesis intermediates in the preparation of the compounds of the general formula (I) according to claim 1.

**23.** Pharmaceutical compositions containing as active ingredient at least one compound according to claim 1, in combination with one or more inert, non-toxic, pharmaceutically acceptable excipients or carriers.

**24.** Pharmaceutical compositions according to claim 23 which can be used in the treatment of pain.

**Claims for the following Contracting State : GR**

**1.** Process for the preparation of new compounds of the general formula (I):

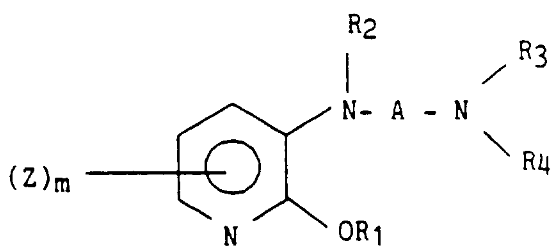

(I)

in which:

- $R_1$ and $R_2$ each represent a hydrogen atom or, with the oxygen and the nitrogen carrying them, form a chain -O-CO-N-, which corresponds to the oxazolo[5,4-b]pyridin-2-ones,
- Z represents a halogen atom, a linear or branched lower alkyl group containing from 1 to 6 carbon atoms, a linear or branched lower alkoxy group containing from 1 to 6 carbon atoms, or a trifluoromethyl group,
- m, which is an integer, may be 0, 1, 2 or 3,
- A is a linear or branched alkyl radical containing from 1 to 6 carbon atoms,
- $R_3$ and $R_4$, which may be the same or different, represent:
  - a hydrogen atom,
  - a linear or branched lower alkyl group containing from 1 to 6 carbon atoms,
  - a linear or branched lower alkenyl group containing from 1 to 6 carbon atoms,
  - an optionally substituted aryl or heteroaryl group,
  - an optionally substituted arylalkyl or heteroarylalkyl group, the alkyl chain of which contains from 1 to 3 carbon atoms, or
  - a mono- or bi-cyclic cycloalkyl group having from 3 to 10 carbon atoms,

  or:
- $R_3$ and $R_4$, with the nitrogen atom to which they are bonded, form a mono- or bi-cyclic, saturated or unsaturated, nitrogen-containing heterocyclic system that contains not more than 12 atoms, not including the hydrogen atoms, which may include from one to three hetero atoms selected from nitrogen, oxygen and sulphur, and that is optionally substituted by one or more:
  - hydroxy groups,
  - oxo groups,
  - linear or branched lower alkyl groups containing from 1 to 6 carbon atoms,
  - optionally substituted aryl groups,
  - optionally substituted arylalkyl or optionally substituted diarylalkyl groups, the alkyl chain of which contains from 1 to 3 carbon atoms,
  - -CO-$OR_5$ groups,

-

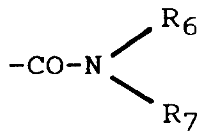

groups,

- $R_5$ represents:
  - a hydrogen atom,
  - a linear or branched lower alkyl group containing from 1 to 6 carbon atoms,
  - an optionally substituted aryl group, or
  - an optionally substituted arylalkyl group, the alkyl chain of which contains from 1 to 3 carbon atoms,
  - $R_6$ and $R_7$, which may be the same or different, have the same meaning as $R_5$,

  it being understood that:
- "aryl group" denotes a mono- or bi-cyclic, unsaturated or aromatic group containing from 5 to 12 carbon atoms,
- "heteroaryl group" denotes a mono- or bi-cyclic, unsaturated or aromatic group that contains from 5 to 12 atoms, not including the hydrogen atoms, and that includes in its carbon skeleton one, two or three hetero atoms selected from nitrogen, oxygen and sulphur,
- the term "substituted" associated with the expressions aryl, arylalkyl, diarylalkyl, heteroaryl and heteroarylalkyl indicates that the aryl or heteroaryl ring, or rings, may be substituted by one or more groups linear or branched lower alkyl having from 1 to 6 carbon atoms, linear or branched lower alkoxy having from 1 to 6 carbon atoms, hydroxy, nitro, halogen, trifluoromethyl,

and of their isomers, epimers and diastereoisomers, and also of their addition salts with a pharmaceutically acceptable acid and/or, when $R_1$ and $R_2$ each represent a hydrogen atom, their addition salts with a pharmaceutically acceptable base,

which process is characterised in that:

A) either a 3-amino-2-pyridinone of the general formula (II):

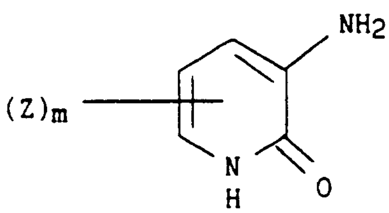

(II),

in which Z and m have the same meaning as in the compounds of the general formula (I), in solution at -78 °C (temperature of the acetone/solid carbon dioxide mixture) in an aprotic solvent or a mixture of aprotic solvents, is reacted with bis(trichloromethyl) carbonate (triphosgene) in the presence of a basic amine, such as, for example, triethylamine, in such a manner as to obtain a [1H]-oxazolo[5,4-b]pyridin-2-one of the general formula (III):

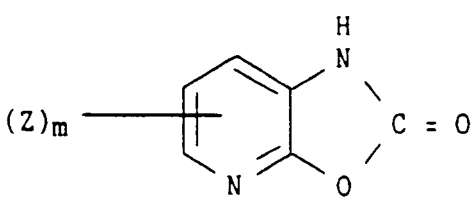

(III),

in which Z and m have the same meaning as in the compounds of the general formula (I), which is reacted with an alkali metal alcoholate or hydride in an aprotic organic medium in such a manner as to obtain the compound of the general formula (IV):

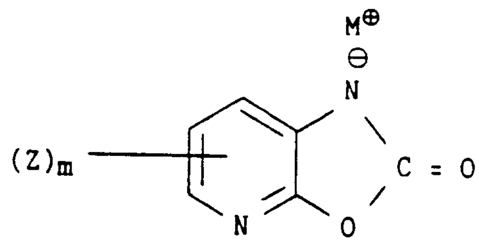

(IV),

in which Z and m have the same meaning as in the compounds of the general formula (I) and M represents an alkali metal, which is reacted:

a) either, in an organic medium and at a temperature of from room temperature to the reflux temperature of the chosen solvent (or mixture of solvents), with an electrophilic compound (preferably in excess) of the general formula (V):

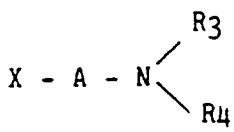

(V),

in which X represents a halogen atom and A, $R_3$ and $R_4$ have the same meaning as in the compounds of the general formula (I), in such a manner as to obtain, after cooling, extraction and, optionally, purification, the compounds of the general formula ($I_A$):

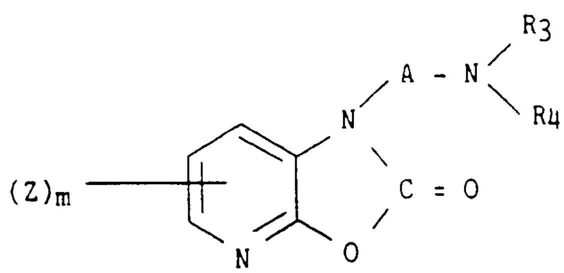

($I_A$)

in which A, $R_3$, $R_4$, Z and m have the same meaning as in the compounds of the general formula (I),

b) or, in an organic medium and at a temperature of from room temperature to the reflux temperature of the chosen solvent (or mixture of solvents), with an electrophilic compound (preferably in excess) of the general formula (VI):

X - A - X' (VI),

in which X and X', which may be the same or different, each represent a halogen atom and A has the same definition as in the compounds of the general formula (I), in such a manner as to obtain the halogenated compound of the general formula (VII):

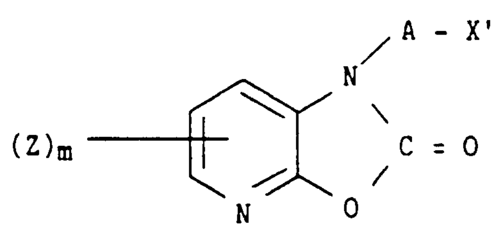

(VII),

in which X', A, Z and m have the same meaning as above, which, finally, is reacted with an amine (preferably in excess) of the general formula (VIII):

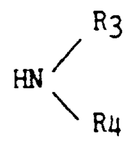

(VIII),

in which $R_3$ and $R_4$ have the same meaning as in the compounds of formula (I), in an organic medium, optionally in the presence of a basic amine, such as, for example, diisopropylamine, and at a temperature of from room temperature to the reflux temperature of the chosen solvent, in such a manner as to obtain, after cooling, extraction and, optionally, purification, the compounds of the general formula ($I_A$) such as defined above,

c) or, in the case of the compounds of general formula ($I_A$) in which A is a methylene chain $-CH_2-$, in an organic medium and at a temperature of from room temperature to the reflux temperature of the chosen solvent (or mixture of solvents), with chloromethyl phenyl sulphide in such a manner as to obtain the compounds of the general formula (IX):

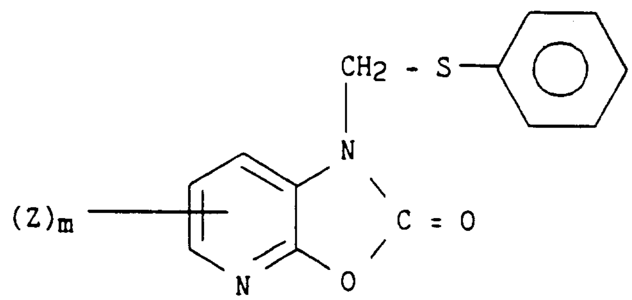

(IX),

in which Z and m have the same meaning as in the compounds of the general formula (I), which are then reacted, in an organic medium, with sulphuryl chloride to obtain, after purification, the chlorinated compound of the general formula (X):

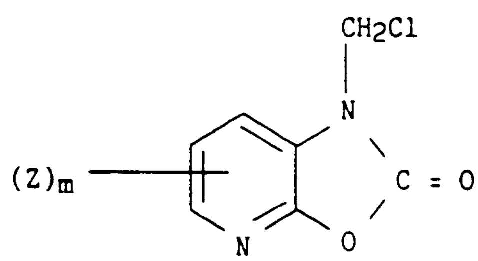

(X),

in which Z and m have the same meaning as in the compounds of the general formula (I), which is then reacted, as in the case of the compounds of the general formula (VII), with compounds of the general formula (VIII) in such a manner as to obtain the compounds of the general formula ($I_A$) such as defined above in which A is a methylene chain $-CH_2-$,

B) or, in the case of the compounds of the general formula ($I_A$) in which A is a methylene chain $-CH_2-$, a compound of the general formula (III) is condensed, in a lower aliphatic alcohol medium, with an amine of the general formula (VIII) in a slight excess and in an excess of formaldehyde, at a temperature of from room temperature to the reflux temperature of the reaction medium, it being possible for the resulting compounds of the general formula ($I_A$), after isolation, optionally to be purified by chromatography on a column of silica,

C) or, in the case of the compounds of the general formula (I) in which $R_1$ and $R_2$ each represent a hydrogen atom, the compounds ($I_A$) are treated with an alkaline agent such as, for example, sodium

hydroxide in aqueous solution, at a temperature of from room temperature to the reflux temperature of the reaction medium, in such a manner as to obtain, after optional acidification and/or neutralisation of the reaction medium, the compounds of the general formula ($I_B$):

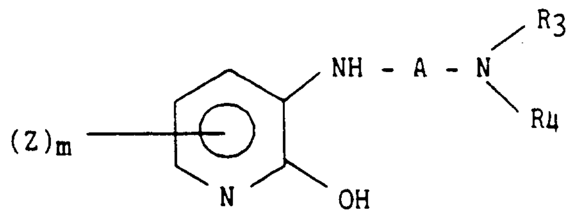

($I_B$),

which are a particular case of the compounds of the general formula (I) in which $R_1 = R_2 =$ H and A, $R_3$, $R_4$, Z and m have the same meaning as in the compounds of the general formula (I),

it being understood that the compounds of formulae ($I_A$) and ($I_B$) so obtained may, if desired, be separated into their isomers and/or converted into a salt with a pharmaceutically acceptable acid or base.

2. Process according to claim 1 for the preparation of compounds in which $R_1$ and $R_2$, with the oxygen and the nitrogen carrying them, form a chain -O-CO-N-, of the general formula ($I_A$):

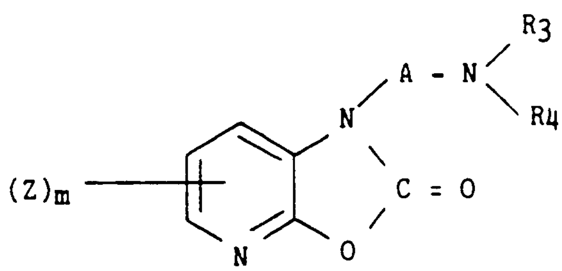

($I_A$),

Z, m, A, $R_3$ and $R_4$ having the same meaning as in claim 1, and of their isomers, epimers and diastereoisomers, and also of their addition salts with a pharmaceutically acceptable acid.

3. Process according to claim 1 for the preparation of compounds in which $R_1$ and $R_2$ each represent a hydrogen atom, of the general formula ($I_B$):

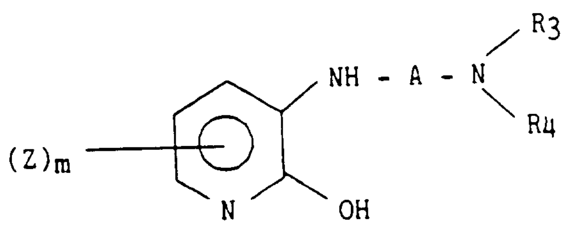

($I_B$),

Z, m, A, $R_3$ and $R_4$ having the same meaning as in claim 1, and of their isomers, epimers and diastereoisomers, and also of their addition salts with a pharmaceutically acceptable acid or base.

4. Process according to claim 1 for the preparation of compounds of the general formula (I) in which m = 0 and A is a linear alkyl radical having from 2 to 6 carbon atoms.

5. Process according to claim 1 for the preparation of the compound which is 1-[2-(4-phenylpiperazin-1-yl)ethyl]-[1H]-oxazolo[5,4-b]pyridin-2-one, the formula of which is shown below, and its addition salts with a pharmaceutically acceptable acid

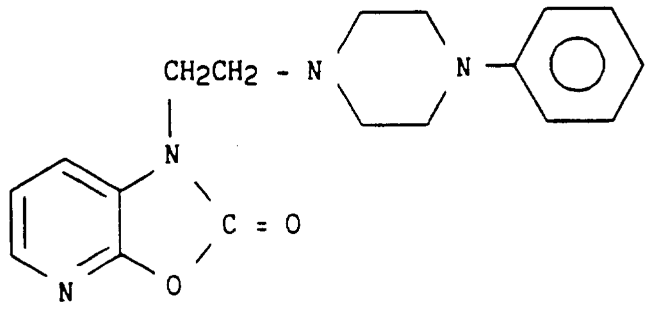

.

6. Process according to claim 1 for the preparation of the compound which is 1-[2-(4-(3-trifluoromethylphenyl)piperazin-1-yl)ethyl]-[1H]-oxazolo[5,4-b]pyridin-2-one, the formula of which is shown below, and of its addition salts with a pharmaceutically acceptable acid

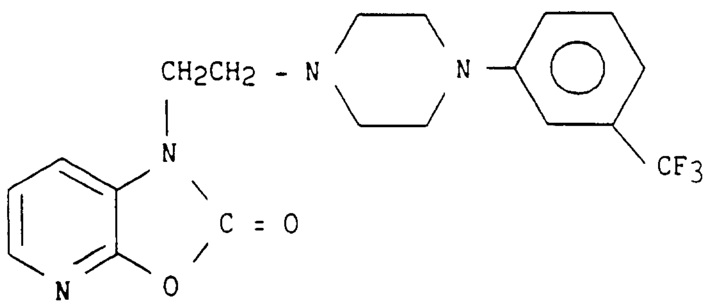

.

7. Process according to claim 1 for the preparation of the compound which is 1-[2-morpholinoethyl]-[1H]-oxazolo[5,4-b]pyridin-2-one, the formula of which is shown below, and of its addition salts with a pharmaceutically acceptable acid

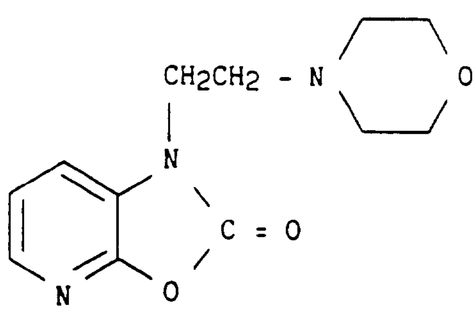

.

8. Process according to claim 1 for the preparation of the compound which is 1-[3-morpholinoprop-1-yl]-[1H]-oxazolo[5,4-b]pyridin-2-one, the formula of which is shown below, and of its addition salts with a pharmaceutically acceptable acid

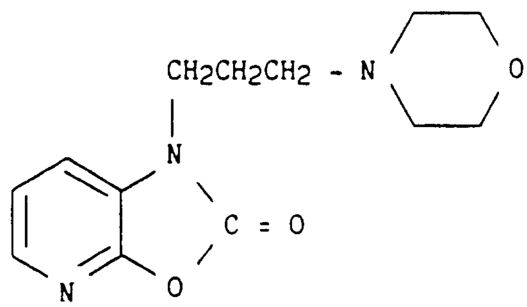

.

9. Process according to claim 1 for the preparation of the compound which is 1-[4-morpholinobut-1-yl]-[1H]-oxazolo[5,4-b]pyridin-2-one, the formula of which is shown below, and of its addition salts with a pharmaceutically acceptable acid

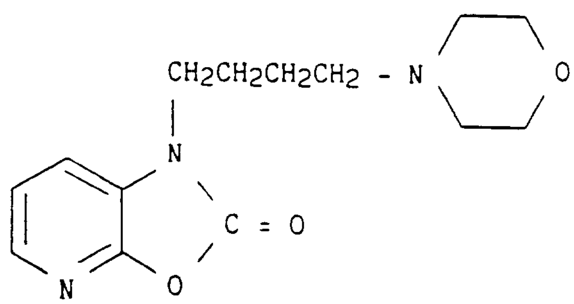

.

10. Process according to claim 1 for the preparation of the compound which is 1-[3-(4-phenylpiperazin-1-yl)prop-1-yl]-[1H]-oxazolo[5,4-b]pyridin-2-one, the formula of which is shown below, and of its addition salts with a pharmaceutically acceptable acid

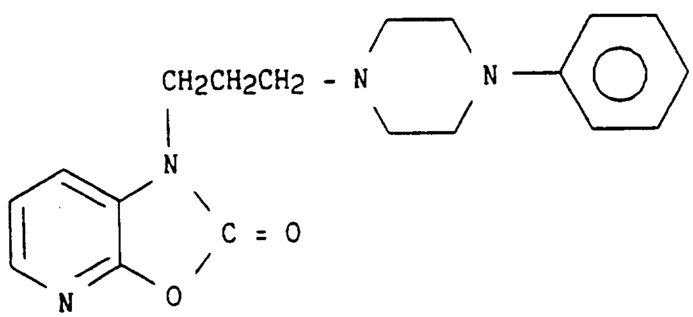

.

11. Process according to claim 1 for the preparation of the compound which is 1-[4-(4-phenylpiperazin-1-yl)but-1-yl]-[1H]-oxazolo[5,4-b]pyridin-2-one, the formula of which is shown below, and of its addition salts with a pharmaceutically acceptable acid

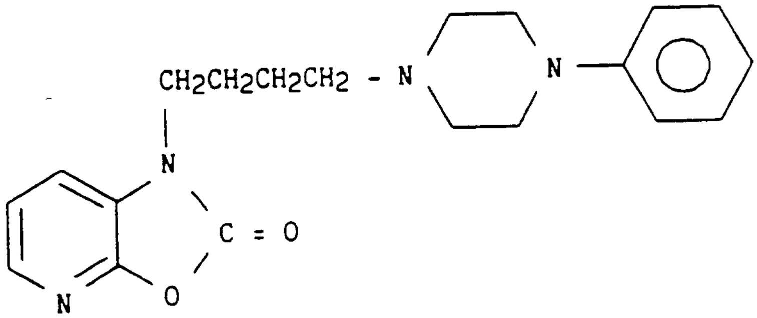

.

12. Process according to claim 1 for the preparation of the compound which is 1-[2-(4-(4-fluorophenyl)-piperazin-1-yl)ethyl]-[1H]-oxazolo[5,4-b]pyridin-2-one, the formula of which is shown below, and of its addition salts with a pharmaceutically acceptable acid

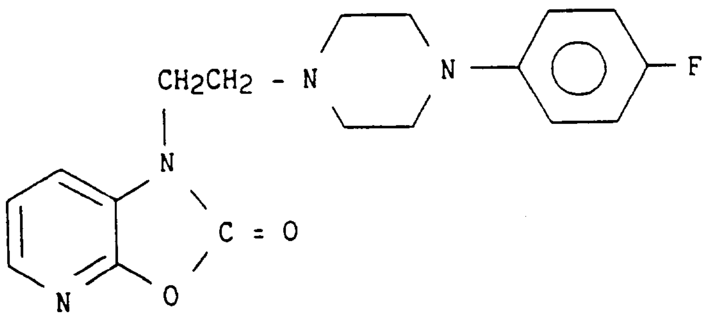

.

13. Process according to claim 1 for the preparation of the compound which is 1-[2-(pyrrolidin-1-yl)ethyl]-[1H]-oxazolo[5,4-b]pyridin-2-one, the formula of which is shown below, and of its addition salts with a pharmaceutically acceptable acid

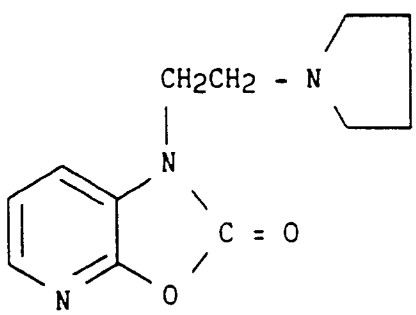

.

14. Process according to claim 1 for the preparation of the compound which is 1-[2-(4-phenylpiperidin-1-yl)ethyl]-[1H]-oxazolo[5,4-b]pyridin-2-one, the formula of which is shown below, and of its addition salts with a pharmaceutically acceptable acid

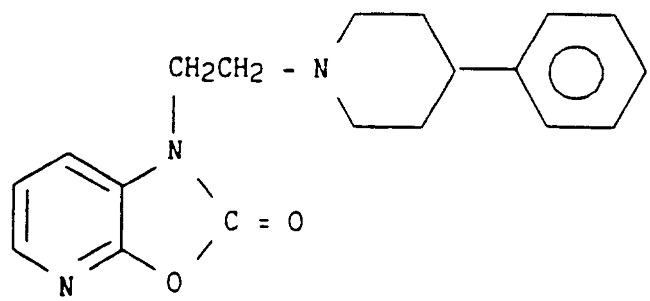

.

15. Process according to claim 1 for the preparation of the compound which is 1-[2-(4-(N,N-diethylaminocarbonyl)piperazin-1-yl)ethyl]-[1H]-oxazolo[5,4-b]pyridin-2-one, the formula of which is shown below, and of its addition salts with a pharmaceutically acceptable acid or base

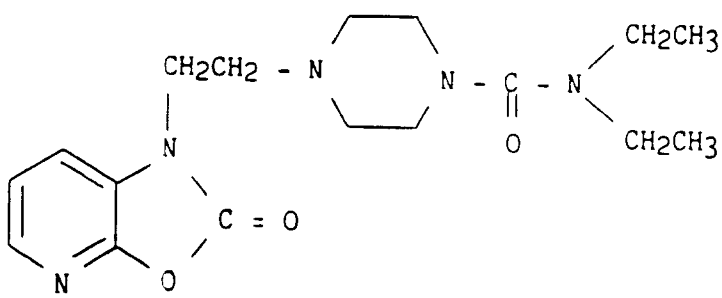

.

16. Process according to claim 1 for the preparation of the compound which is 3-[2-(4-phenylpiperazin-1-yl)ethyl-amino]-2-hydroxypyridine, the formula of which is shown below, and of its addition salts with a pharmaceutically acceptable acid or base

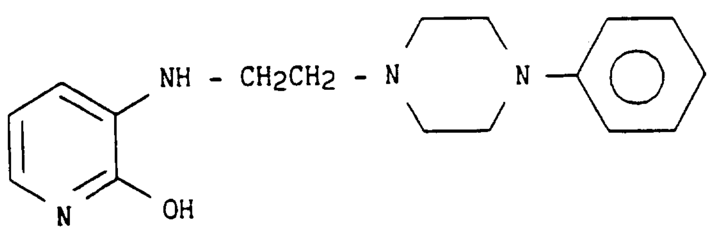

.

17. Process for the manufacture of pharmaceutical compositions containing as active ingredient at least one compound prepared according to claim 1, in combination with one or more inert, non-toxic, pharmaceutically acceptable excipients or carriers.

18. Process for the manufacture of pharmaceutical compositions according to claim 17 which can be used in the treatment of pain.

**Claims for the following Contracting State : ES**

**1.** Process for the preparation of new compounds of the general formula (I):

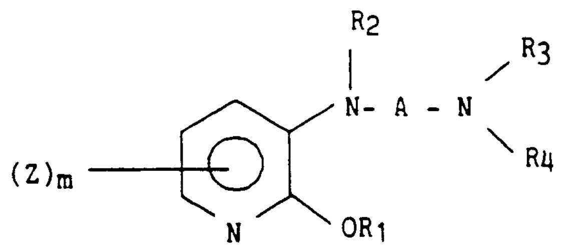

(I)

in which:

- $R_1$ and $R_2$ each represent a hydrogen atom or, with the oxygen and the nitrogen carrying them, form a chain -O-CO-N-, which corresponds to the oxazolo[5,4-b]pyridin-2-ones,
- Z represents a halogen atom, a linear or branched lower alkyl group containing from 1 to 6 carbon atoms, a linear or branched lower alkoxy group containing from 1 to 6 carbon atoms, or a trifluoromethyl group,
- m, which is an integer, may be 0, 1, 2 or 3,
- A is a linear or branched alkyl radical containing from 1 to 6 carbon atoms,
- $R_3$ and $R_4$, which may be the same or different, represent:
  - a hydrogen atom,
  - a linear or branched lower alkyl group containing from 1 to 6 carbon atoms,
  - a linear or branched lower alkenyl group containing from 1 to 6 carbon atoms,
  - an optionally substituted aryl or heteroaryl group,
  - an optionally substituted arylalkyl or heteroarylalkyl group, the alkyl chain of which contains from 1 to 3 carbon atoms, or
  - a mono- or bi-cyclic cycloalkyl group having from 3 to 10 carbon atoms,

  or:
- $R_3$ and $R_4$, with the nitrogen atom to which they are bonded, form a mono- or bi-cyclic, saturated or unsaturated, nitrogen-containing heterocyclic system that contains not more than 12 atoms, not including the hydrogen atoms, which may include from one to three hetero atoms selected from nitrogen, oxygen and sulphur, and that is optionally substituted by one or more:
  - hydroxy groups,
  - oxo groups,
  - linear or branched lower alkyl groups containing from 1 to 6 carbon atoms,
  - optionally substituted aryl groups,
  - optionally substituted arylalkyl or optionally substituted diarylalkyl groups, the alkyl chain of which contains from 1 to 3 carbon atoms,
  - -CO-$OR_5$ groups,
  - 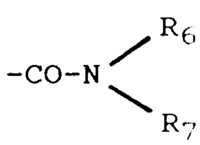

    groups,
- $R_5$ represents:
  - a hydrogen atom,
  - a linear or branched lower alkyl group containing from 1 to 6 carbon atoms,
  - an optionally substituted aryl group, or
  - an optionally substituted arylalkyl group, the alkyl chain of which contains from 1 to 3 carbon atoms,

- $R_6$ and $R_7$, which may be the same or different, have the same meaning as $R_5$,

it being understood that:

- "aryl group" denotes a mono- or bi-cyclic, unsaturated or aromatic group containing from 5 to 12 carbon atoms,
- "heteroaryl group" denotes a mono- or bi-cyclic, unsaturated or aromatic group that contains from 5 to 12 atoms, not including the hydrogen atoms, and that includes in its carbon skeleton one, two or three hetero atoms selected from nitrogen, oxygen and sulphur,
- the term "substituted" associated with the expressions aryl, arylalkyl, diarylalkyl, heteroaryl and heteroarylalkyl indicates that the aryl or heteroaryl ring, or rings, may be substituted by one or more groups linear or branched lower alkyl having from 1 to 6 carbon atoms, linear or branched lower alkoxy having from 1 to 6

carbon atoms, hydroxy, nitro, halogen, trifluoromethyl, and of their isomers, epimers and diastereoisomers, and also of their addition salts with a pharmaceutically acceptable acid and/or, when $R_1$ and $R_2$ each represent a hydrogen atom, their addition salts with a pharmaceutically acceptable base, which process is characterised in that:

A) either a 3-amino-2-pyridinone of the general formula (II):

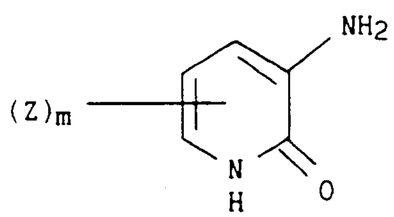

(II),

in which Z and m have the same meaning as in the compounds of the general formula (I), in solution at -78 °C (temperature of the acetone/solid carbon dioxide mixture) in an aprotic solvent or a mixture of aprotic solvents, is reacted with bis(trichloromethyl) carbonate (triphosgene) in the presence of a basic amine, such as, for example, triethylamine, in such a manner as to obtain a [1H]-oxazolo[5,4-b]pyridin-2-one of the general formula (III):

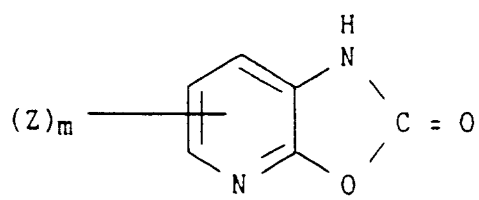

(III),

in which Z and m have the same meaning as in the compounds of the general formula (I), which is reacted with an alkali metal alcoholate or hydride in an aprotic organic medium in such a manner as to obtain the compound of the general formula (IV):

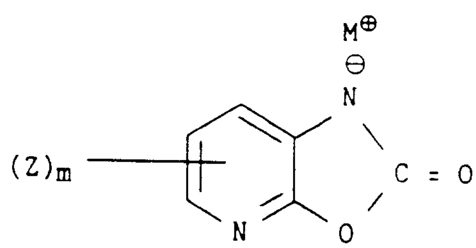

(IV),

in which Z and m have the same meaning as in the compounds of the general formula (I) and M represents an alkali metal, which is reacted:

a) either, in an organic medium and at a temperature of from room temperature to the reflux temperature of the chosen solvent (or mixture of solvents), with an electrophilic compound (preferably in excess) of the general formula (V):

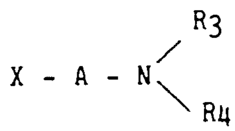

(V),

in which X represents a halogen atom and A, $R_3$ and $R_4$ have the same meaning as in the compounds of the general formula (I), in such a manner as to obtain, after cooling, extraction and, optionally, purification, the compounds of the general formula ($I_A$):

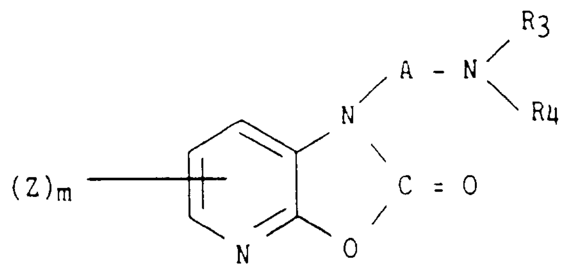

($I_A$)

in which A, $R_3$, $R_4$, Z and m have the same meaning as in the compounds of the general formula (I),

b) or, in an organic medium and at a temperature of from room temperature to the reflux temperature of the chosen solvent (or mixture of solvents), with an electrophilic compound (preferably in excess) of the general formula (VI):

X - A - X' (VI),

in which X and X', which may be the same or different, each represent a halogen atom and A has the same definition as in the compounds of the general formula (I), in such a manner as to obtain the halogenated compound of the general formula (VII):

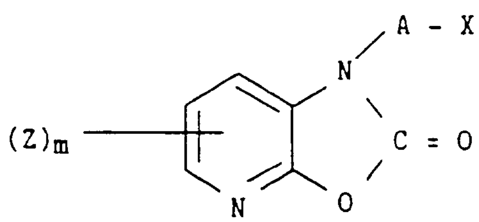

(VII),

in which X', A, Z and m have the same meaning as above, which, finally, is reacted with an amine (preferably in excess) of the general formula (VIII):

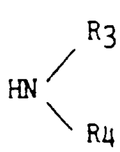

(VIII),

in which $R_3$ and $R_4$ have the same meaning as in the compounds of formula (I), in an organic medium, optionally in the presence of a basic amine, such as, for example, diisopropylamine, and at a temperature of from room temperature to the reflux temperature of the chosen solvent, in such a manner as to obtain, after cooling, extraction and, optionally, purification, the compounds of the general formula ($I_A$) such as defined above,

c) or, in the case of the compounds of general formula ($I_A$) in which A is a methylene chain $-CH_2-$, in an organic medium and at a temperature of from room temperature to the reflux temperature of the chosen solvent (or mixture of solvents), with chloromethyl phenyl sulphide in such a manner as to obtain the compounds of the general formula (IX):

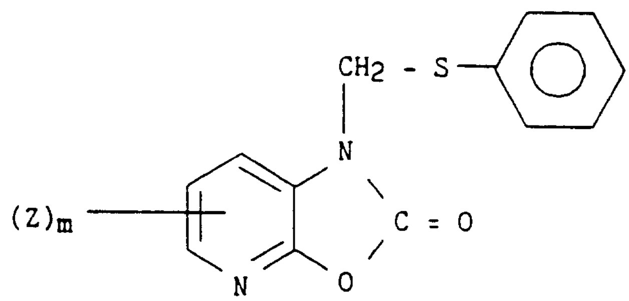

(IX),

in which Z and m have the same meaning as in the compounds of the general formula (I), which are then reacted, in an organic medium, with sulphuryl chloride to obtain, after purification, the chlorinated compound of the general formula (X):

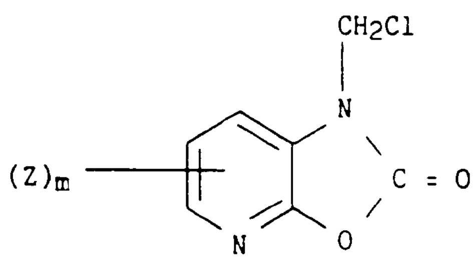

(X),

in which Z and m have the same meaning as in the compounds of the general formula (I), which is then reacted, as in the case of the compounds of the general formula (VII), with compounds of the general formula (VIII) in such a manner as to obtain the compounds of the general formula ($I_A$) such as defined above in which A is a methylene chain $-CH_2-$,

B) or, in the case of the compounds of the general formula ($I_A$) in which A is a methylene chain $-CH_2-$, a compound of the general formula (III) is condensed, in a lower aliphatic alcohol medium, with an amine of the general formula (VIII) in a slight excess and in an excess of formaldehyde, at a temperature of from room temperature to the reflux temperature of the reaction medium, it being possible for the resulting compounds of the general formula ($I_A$), after isolation, optionally to be purified by chromatography on a column of silica,

C) or, in the case of the compounds of the general formula (I) in which $R_1$ and $R_2$ each represent a hydrogen atom, the compounds ($I_A$) are treated with an alkaline agent such as, for example, sodium hydroxide in aqueous solution, at a temperature of from room temperature to the reflux temperature of the reaction medium, in such a manner as to obtain, after optional acidification and/or neutralisation of the reaction medium, the compounds of the general formula ($I_B$):

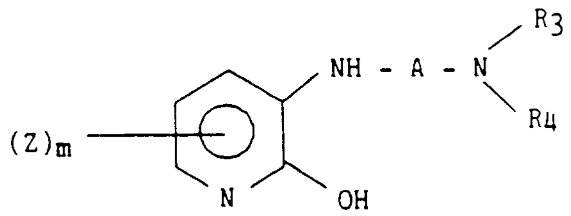

$(I_B)$,

which are a particular case of the compounds of the general formula (I) in which $R_1 = R_2 = H$ and A, $R_3$, $R_4$, Z and m have the same meaning as in the compounds of the general formula (I),
it being understood that the compounds of formulae ($I_A$) and ($I_B$) so obtained may, if desired, be separated into their isomers and/or converted into a salt with a pharmaceutically acceptable acid or base.

**2.** Process according to claim 1 for the preparation of compounds in which $R_1$ and $R_2$, with the oxygen and the nitrogen carrying them, form a chain -O-CO-N-, of the general formula ($I_A$):

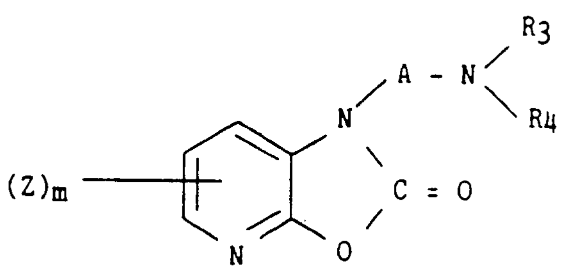

$(I_A)$,

Z, m, A, $R_3$ and $R_4$ having the same meaning as in claim 1, and of their isomers, epimers and diastereoisomers, and also of their addition salts with a pharmaceutically acceptable acid.

**3.** Process according to claim 1 for the preparation of compounds in which $R_1$ and $R_2$ each represent a hydrogen atom, of the general formula ($I_B$):

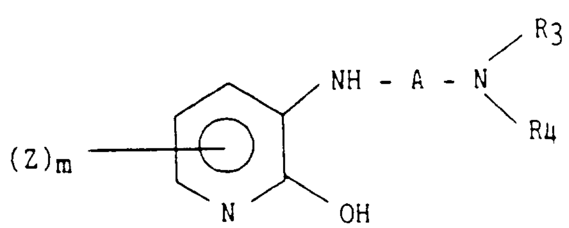

$(I_B)$,

Z, m, A, $R_3$ and $R_4$ having the same meaning as in claim 1, and of their isomers, epimers and diastereoisomers, and also of their addition salts with a pharmaceutically acceptable acid or base.

**4.** Process according to claim 1 for the preparation of compounds of the general formula (I) in which m = 0 and A is a linear alkyl radical having from 2 to 6 carbon atoms.

**5.** Process according to claim 1 for the preparation of the compound which is 1-[2-(4-phenylpiperazin-1-yl)ethyl]-[1H]-oxazolo[5,4-b]pyridin-2-one, the formula of which is shown below, and its addition salts with a pharmaceutically acceptable acid

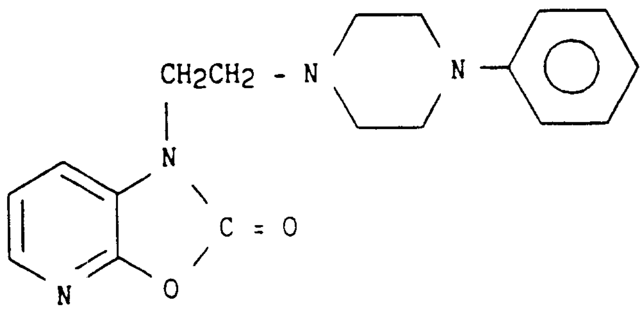

.

6. Process according to claim 1 for the preparation of the compound which is 1-[2-(4-(3-trifluoromethylphenyl)piperazin-1-yl)ethyl]-[1H]-oxazolo[5,4-b]pyridin-2-one, the formula of which is shown below, and of its addition salts with a pharmaceutically acceptable acid

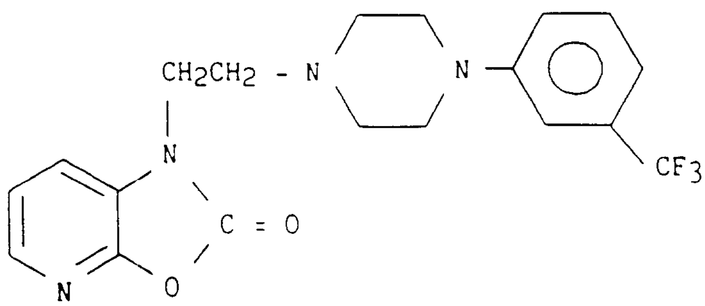

.

7. Process according to claim 1 for the preparation of the compound which is 1-[2-morpholinoethyl]-[1H]-oxazolo[5,4-b]pyridin-2-one, the formula of which is shown below, and of its addition salts with a pharmaceutically acceptable acid

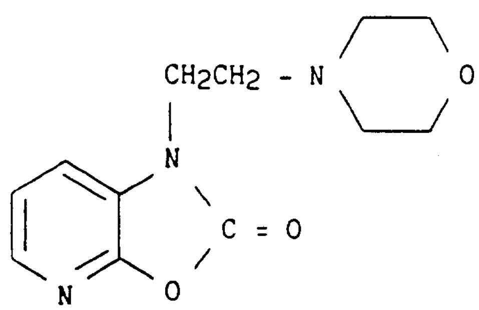

.

8. Process according to claim 1 for the preparation of the compound which is 1-[3-morpholinoprop-1-yl]-[1H]-oxazolo[5,4-b]pyridin-2-one, the formula of which is shown below, and of its addition salts with a pharmaceutically acceptable acid

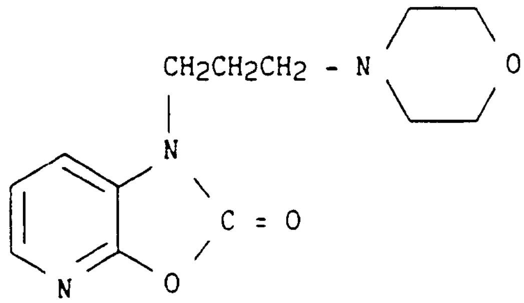

.

9. Process according to claim 1 for the preparation of the compound which is 1-[4-morpholinobut-1-yl]-[1H]-oxazolo[5,4-b]pyridin-2-one, the formula of which is shown below, and of its addition salts with a pharmaceutically acceptable acid

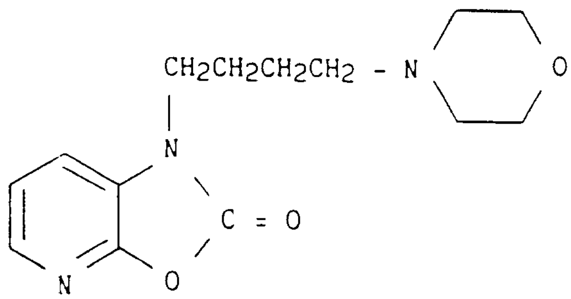

.

10. Process according to claim 1 for the preparation of the compound which is 1-[3-(4-phenylpiperazin-1-yl)prop-1-yl]-[1H]-oxazolo[5,4-b]pyridin-2-one, the formula of which is shown below, and of its addition salts with a pharmaceutically acceptable acid

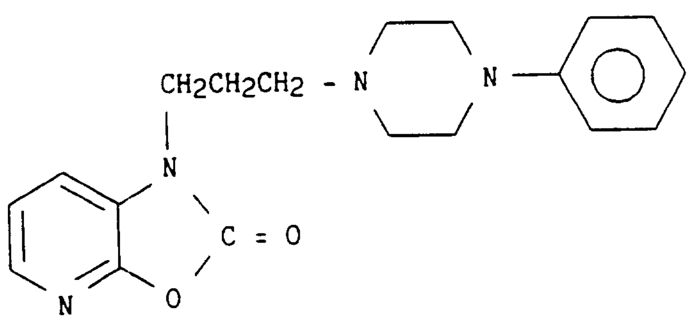

.

11. Process according to claim 1 for the preparation of the compound which is 1-[4-(4-phenylpiperazin-1-yl)but-1-yl]-[1H]-oxazolo[5,4-b]pyridin-2-one, the formula of which is shown below, and of its addition salts with a pharmaceutically acceptable acid

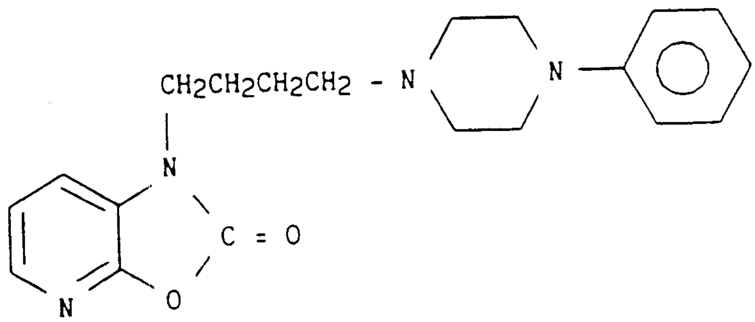

.

**12.** Process according to claim 1 for the preparation of the compound which is 1-[2-(4-(4-fluorophenyl)-piperazin-1-yl)ethyl]-[1H]-oxazolo[5,4-b]pyridin-2-one, the formula of which is shown below, and of its addition salts with a pharmaceutically acceptable acid

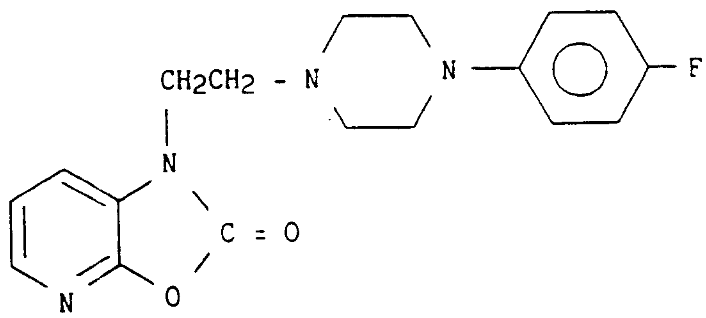

.

**13.** Process according to claim 1 for the preparation of the compound which is 1-[2-(pyrrolidin-1-yl)ethyl]-[1H]-oxazolo[5,4-b]pyridin-2-one, the formula of which is shown below, and of its addition salts with a pharmaceutically acceptable acid

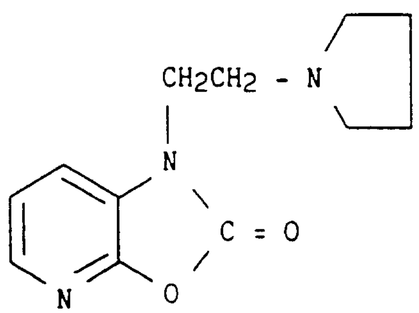

.

**14.** Process according to claim 1 for the preparation of the compound which is 1-[2-(4-phenylpiperidin-1-yl)ethyl]-[1H]-oxazolo[5,4-b]pyridin-2-one, the formula of which is shown below, and of its addition salts with a pharmaceutically acceptable acid

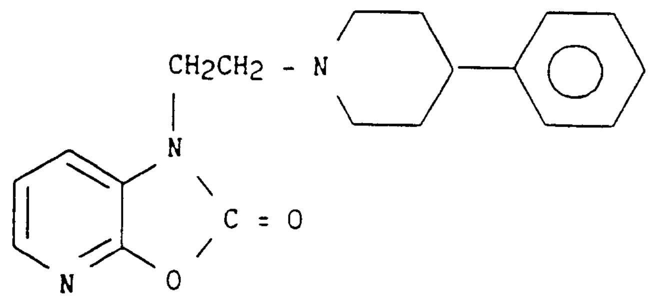

.

15. Process according to claim 1 for the preparation of the compound which is 1-[2-(4-(N,N-diethylaminocarbonyl)piperazin-1-yl)ethyl]-[1H]-oxazolo[5,4-b]pyridin-2-one, the formula of which is shown below, and of its addition salts with a pharmaceutically acceptable acid or base

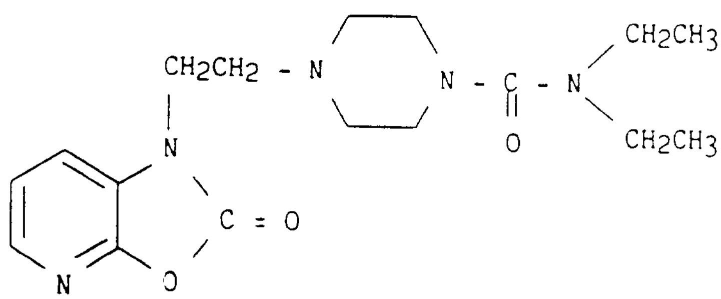

.

16. Process according to claim 1 for the preparation of the compound which is 3-[2-(4-phenylpiperazin-1-yl)ethyl-amino]-2-hydroxypyridine, the formula of which is shown below, and of its addition salts with a pharmaceutically acceptable acid or base

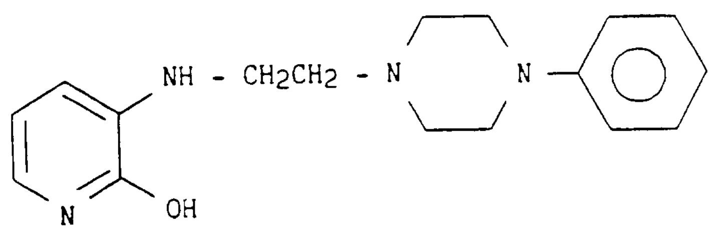

.

17. Process for the manufacture of pharmaceutical compositions containing as active ingredient at least one compound prepared according to claim 1, in combination with one or more inert, non-toxic, pharmaceutically acceptable excipients or carriers.

18. Process for the manufacture of pharmaceutical compositions according to claim 17 which can be used in the treatment of pain.

**Patentansprüche**

**Patentansprüche für folgende Vertragsstaaten : AT, BE, CH, DE, DK, FR, GB, IT, LI, LU, NL, SE**

**1.** Verbindungen der allgemeinen Formel (I):

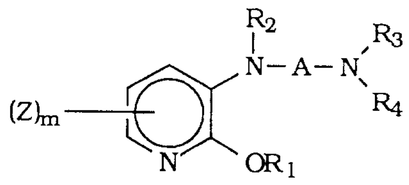

(I)

in der:

- $R_1$ und $R_2$ jeweils ein Wasserstoffatom oder gemeinsam mit dem Sauerstoff und dem Stickstoff, die sie tragen, eine Kette - O - CO - N -, was Oxazolo[5,4-b]pyridin-2-onen entspricht,
- Z ein Halogenatom, eine geradkettige oder verzweigte Niedrigalkylgruppe mit 1 bis 6 Kohlenstoffatomen, eine geradkettige oder verzweigte Niedrigalkoxygruppe mit 1 bis 6 Kohlenstoffatomen oder eine Trifluormethylgruppe,
- m eine ganze Zahl mit Werten von 0, 1, 2 oder 3,
- A eine geradkettige oder verzweigte Alkylgruppe mit 1 bis 6 Kohlenstoffatomen,
- $R_3$ und $R_4$, die gleichartig oder verschieden sein können, jeweils:
  - ein Wasserstoffatom,
  - eine geradkettige oder verzweigte Niedrigalkylgruppe mit 1 bis 6 Kohlenstoffatomen,
  - eine geradkettige oder verzweigte Niedrigalkenylgruppe mit 1 bis 6 Kohlenstoffatomen,
  - eine gegebenenfalls substituierte Aryl- oder Heteroarylgruppe,
  - eine gegebenenfalls substituierte Arylalkyl- oder Heteroarylalkylgruppe, deren Alkylkette 1 bis 3 Kohlenstoffatome aufweist,
  - eine mono- oder bicyclische Cycloalkylgruppe mit 3 bis 10 Kohlenstoffatomen,

  oder:

  $R_3$ und $R_4$ zusammen mit dem Stickstoffatom, an das sie gebunden sind, ein mono- oder bicyclisches, gesättigtes oder ungesättigtes stickstoffhaltiges heterocyclisches System mit maximal 12 Atomen ohne die Wasserstoffatome, darunter bis zu drei Heteroatomen ausgewählt aus Stickstoff, Sauerstoff oder Schwefel und das gegebenenfalls durch eine oder mehrere:
  - Hydroxylgruppen,
  - Oxogruppen,
  - geradkettige oder verzweigte Niedrigalkylgruppen mit 1 bis 6 Kohlenstoffatomen,
  - gegebenenfalls substituierte Arylgruppen,
  - gegebenenfalls substituierte Arylalkylgruppen oder gegebenenfalls substituierte Diarylalkylgruppen, deren Alkylkette 1 bis 3 Kohlenstoffatome aufweist,
  - Gruppen - CO-$OR_5$ und
  - Gruppen

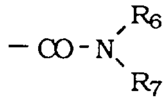

  substituiert sein können,
- $R_5$:
  - ein Wasserstoffatom,
  - eine geradkettige oder verzweigte Niedrigalkylgruppe mit 1 bis 6 Kohlenstoffatomen,
  - eine gegebenenfalls substituierte Arylgruppe oder
  - eine gegebenenfalls substituierte Aralkylgruppe, deren Alkylkette 1 bis 3 Kohlenstoffatome aufweist,

  bedeuten, und

  $R_6$ und $R_7$, die gleichartig oder verschieden sein können, die gleichen Bedeutungen besitzen

wie $R_5$,
- deren Isomere, Epimere und Diastereoisomere,
- deren Additionssalze mit einer pharmazeutisch annehmbaren Säure und/oder, wenn $R_1$ und $R_2$ jeweils ein Wasserstoffatom darstellen, deren Additionssalze mit einer pharmazeutisch annehmbaren Base,
- wobei unter einer Arylgruppe eine mono- oder bicyclische, ungesättigte oder aromatische Gruppe mit 5 bis 12 Kohlenstoffatomen zu verstehen ist,
- unter einer Heteroarylgruppe eine mono- oder bicyclische, ungesättigte oder aromatische Gruppe mit 5 bis 12 Atomen ohne die Wasserstoffatome zu verstehen ist, die in ihrem Kohlenstoffskelett eines, zwei oder drei Heteroatome ausgewählt aus Stickstoff, Sauerstoff oder Schwefel aufweist,
- und der mit den Bedeutungen Aryl, Arylalkyl, Diarylalkyl, Heteroaryl, Heteroarylalkyl verwendete Begriff "substituiert" bedeutet, daß der oder die Aryl- oder Heteroarylkerne durch eine oder mehrere geradkettige oder verzweigte Niedrigalkylgruppen mit 1 bis 6 Kohlenstoffatomen, geradkettige oder verzweigte Niedrigalkoxygruppen mit 1 bis 6 Kohlenstoffatomen, Hydroxylgruppen, Nitrogruppen, Halogenatome oder Trifluormethylgruppen substituiert sein können.

**2.** Verbindungen nach Anspruch 1, worin $R_1$ und $R_2$ gemeinsam mit dem Sauerstoff und dem Stickstoff, die sie tragen, eine Kette - O - CO - N - bilden, der allgemeinen Formel ($I_A$):

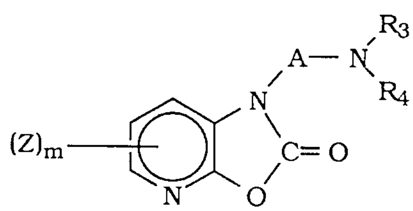

($I_A$)

worin Z, m, A, $R_3$ und $R_4$ die in Anspruch 1 angegebenen Bedeutungen besitzen, deren Isomere, Epimere, Diastereoisomere sowie deren Additionssalze mit einer pharmazeutisch annehmbaren Säure.

**3.** Verbindungen nach Anspruch 1, worin $R_1$ und $R_2$ jeweils ein Wasserstoffatom bedeuten, der allgemeinen Formel ($I_B$):

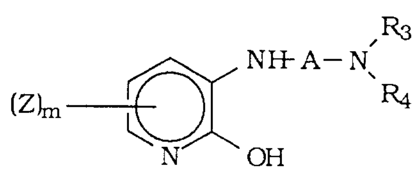

($I_B$)

worin Z, m, A, $R_3$ und $R_4$ die in Anspruch 1 angegebenen Bedeutungen besitzen, deren Isomere, Epimere, Diastereoisomere sowie deren Additionssalze mit einer pharmazeutisch annehmbaren Säure oder Base.

**4.** Verbindungen nach Anspruch 1, worin m den Wert 0 besitzt und A eine geradkettige Alkylgruppe mit 2 bis 6 Kohlenstoffatomen bedeutet.

**5.** Verbindung nach Anspruch 1, nämlich 1-[2-(4-Phenyl-piperazin-1-yl)-ethyl]-[1H]-oxazolo[5,4-b]pyridin-2-on der Formel

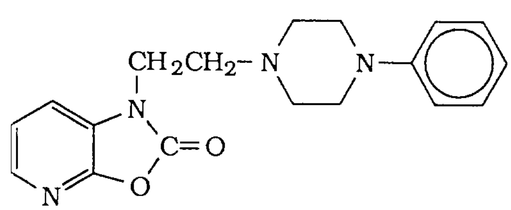

sowie deren Additionssalze mit einer pharmazeutisch annehmbaren Säure.

**6.** Verbindung nach Anspruch 1, nämlich 1-[2-(4-(3-Trifluormethyl-phenyl)piperazin-1-yl)-ethyl]-[1H]-oxazolo[5,4-b]pyridin-2-on der Formel

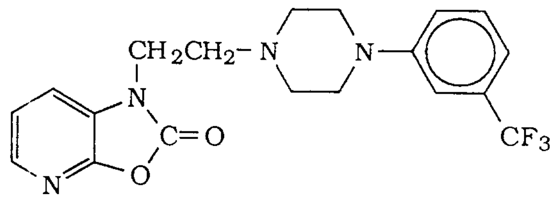

sowie deren Additionssalze mit einer pharmazeutisch annehmbaren Säure.

**7.** Verbindung nach Anspruch 1, nämlich 1-[2-Morpholino-ethyl]-[1H]-oxazolo[5,4-b]pyridin-2-on der Formel

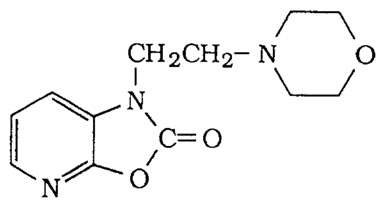

sowie deren Additionssalze mit einer pharmazeutisch annehmbaren Säure.

**8.** Verbindung nach Anspruch 1, nämlich 1-[3-Morpholino-prop-1-yl]-[1H]-oxazolo[5,4-b]pyridin-2-on der Formel

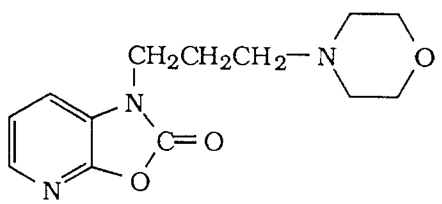

sowie deren Additionssalze mit einer pharmazeutisch annehmbaren Säure.

**9.** Verbindung nach Anspruch 1, nämlich 1-[4-Morpholino-but-1-yl]-[1H]-oxazolo[5,4-b]pyridin-2-on der Formel

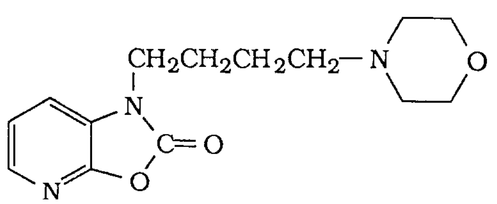

sowie deren Additionssalze mit einer pharmazeutisch annehmbaren Säure.

**10.** Verbindung nach Anspruch 1, nämlich 1-[3-(4-Phenyl-piperazin-1-yl)-prop-1-yl]-[1H]-oxazolo[5,4-b]pyridin-2-on der Formel

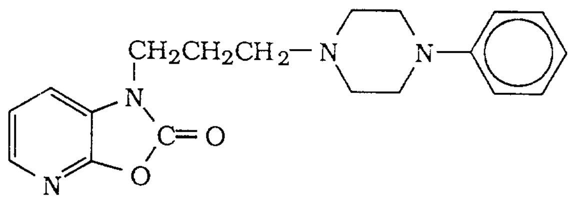

sowie deren Additionssalze mit einer pharmazeutisch annehmbaren Säure.

**11.** Verbindung nach Anspruch 1, nämlich 1-[4-(4-Phenyl-piperazin-1-yl)-but-1-yl]-[1H]-oxazolo[5,4-b]-pyridin-2-on der Formel

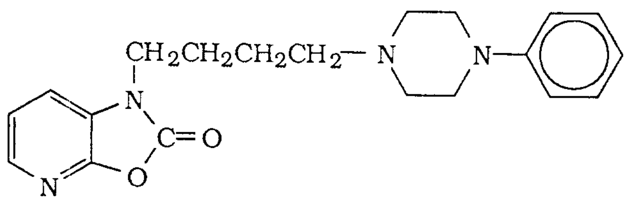

sowie deren Additionssalze mit einer pharmazeutisch annehmbaren Säure.

**12.** Verbindung nach Anspruch 1, nämlich 1-[2-(4-(4-Fluorphenyl)-piperazin-1-yl)-ethyl]-[1H]-oxazolo[5,4-b]-pyridin-2-on der Formel

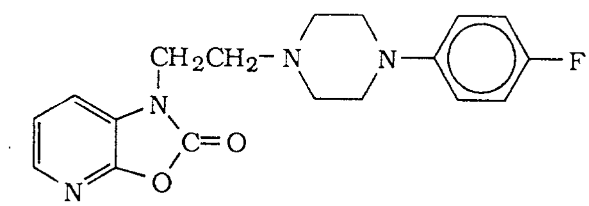

sowie deren Additionssalze mit einer pharmazeutisch annehmbaren Säure.

**13.** Verbindung nach Anspruch 1, nämlich 1-[2-(Pyrrolidin-1-yl)-ethyl]-[1H]-oxazolo[5,4-b]pyridin-2-on der Formel

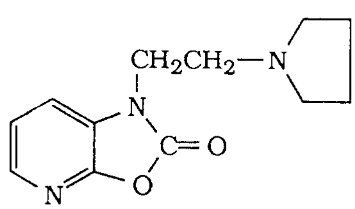

sowie deren Additionssalze mit einer pharmazeutisch annehmbaren Säure.

**14.** Verbindung nach Anspruch 1, nämlich 1-[2-(4-Phenyl-piperidin-1-yl)-ethyl]-[1H]-oxazolo[5,4-b]pyridin-2-on der Formel

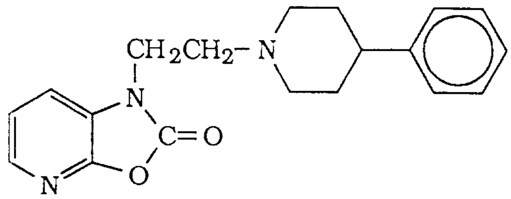

sowie deren Additionssalze mit einer pharmazeutisch annehmbaren Säure.

**15.** Verbindung nach Anspruch 1, nämlich 1-[2-(4-N,N-Diethylaminocarbonyl)-piperazin-1-yl)-ethyl]-[1H]-oxazolo[5,4-b]pyridin-2-on der Formel

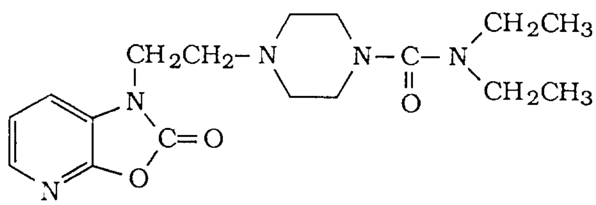

sowie deren Additionssalze mit einer pharmazeutisch annehmbaren Säure oder Base.

**16.** Verbindung nach Anspruch 1, nämlich 3-[2-(4-Phenyl-piperazin-1-yl)-ethylamino]-2-hydroxy-pyridin der Formel

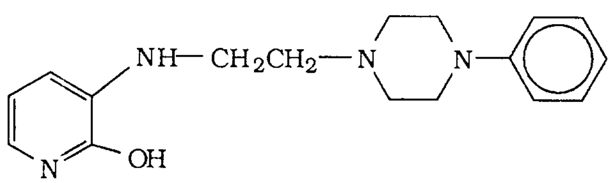

sowie deren Additionssalze mit einer pharmazeutisch annehmbaren Säure oder Base.

**17.** Verfahren zur Herstellung der Verbindungen der allgemeinen Formel ($I_A$) nach Anspruch 2, **dadurch gekennzeichnet**, daß man ein 3-Amino-2-pyridinon der allgemeinen Formel (II):

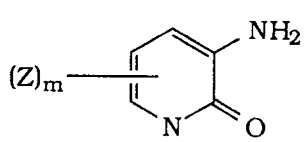

(II)

in der Z und m die bezuglich der Verbindungen der allgemeinen Formel (I) angegebenen Bedeutungen besitzen, in Lösung in einem aprotischen Lösungsmittel oder einer Mischung von aprotischen Lösungsmitteln bei -78 °C (Temperatur einer Mischung aus Aceton und Trockeneis) mit Bis(trichlormethyl)-carbonat (Triphosgen) in Gegenwart eines basischen Amins, wie beispielsweise Triethylamin, in der Weise umsetzt, daß man ein [1H]-Oxazolo[5,4-b]pyridin-2-on der allgemeinen Formel (III):

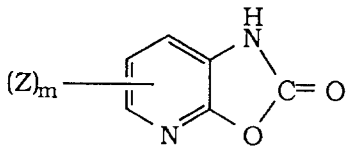

(III)

erhält,
worin Z und m die bezuglich der Verbindungen der allgemeinen Formel (I) angegebenen Bedeutungen besitzen, welches man mit einem Alkalimetallalkoholat oder Alkalimetallhydrid in aprotischem organischem Medium in der Weise umsetzt, daß man die Verbindung der allgemeinen Formel (IV):

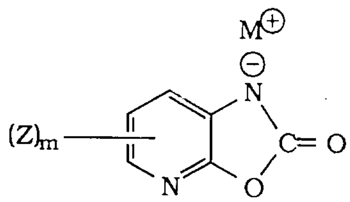

(IV)

in der Z und m die bezüglich der Verbindungen der allgemeinen Formel (I) angegebenen Bedeutungen besitzen und M ein Alkalimetall darstellt, erhält, welche Verbindung man in organischem Medium und bei einer Temperatur zwischen Raumtemperatur und der Rückflußtemperatur des ausgewählten Lösungsmittels (oder der Lösungsmittelmischung) mit einer (vorzugsweise im Überschuß eingesetzten) elektrophilen Verbindung der allgemeinen Formel (V):

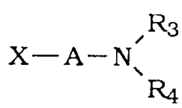

(V)

in der X ein Halogenatom darstellt und A, $R_3$ und $R_4$ die bezüglich der Verbindungen der allgemeinen Formel (I) angegebenen Bedeutungen besitzen, in der Weise umsetzt, daß man nach der Abkühlung, der Extraktion und der eventuellen Reinigung die Verbindungen der allgemeinen Formel ($I_A$):

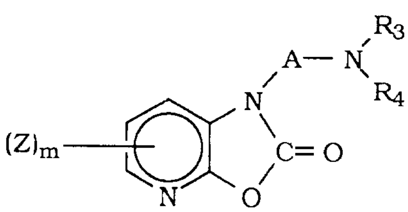

($I_A$)

wie sie in Anspruch 1 definiert ist, erhält, wobei es sich versteht, daß die in dieser Weise erhaltenen Verbindungen der allgemeinen Formel ($I_A$) gewünschtenfalls in ihre Isomeren aufgetrennt und/oder mit einer pharmazeutisch annehmbaren Säure in ihre Salze überführt werden können.

**18.** Verfahren zur Herstellung der Verbindungen der allgemeinen Formel ($I_A$) nach Anspruch 2, **dadurch gekennzeichnet**, daß man eine Verbindung der allgemeinen Formel (IV):

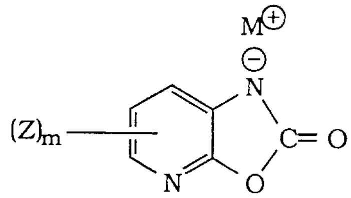

(IV)

in der Z und m die bezüglich der Verbindungen der allgemeinen Formel (I) angegebenen Bedeutungen besitzen und M ein Alkalimetall darstellt, in organischem Medium und bei einer Temperatur zwischen der Raumtemperatur und der Rückflußtemperatur des ausgewählten Lösungsmittels (oder der Lösungsmittelmischung) mit einer (vorzugsweise im Überschuß eingesetzten) elektrophilen Verbindung der allgemeinen Formel (VI):

X - A - X' (VI)

in der X und X', die gleichartig oder verschieden sein können, jeweils ein Halogenatom darstellen und A die bezüglich der Verbindungen der allgemeinen Formel (I) angegebenen Bedeutungen besitzt, in der Weise umsetzt, daß man die Halogenverbindung der allgemeinen Formel (VII):

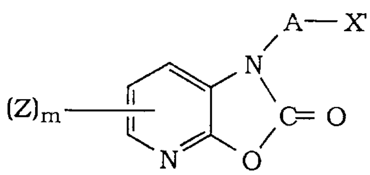

(VII)

in der X', A, Z und m die oben angegebenen Bedeutungen besitzen, erhält, welche man schließlich mit einem (vorzugsweise im Überschuß eingesetzten) Amin der allgemeinen Formel (VIII):

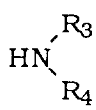

(VIII)

in der $R_3$ und $R_4$ die bezüglich der Verbindungen der allgemeinen Formel (I) angegebenen Bedeutungen besitzen, in organischem Medium und gegebenenfalls in Gegenwart eines basischen Amins, wie beispielsweise Diisopropylamin, und bei einer Temperatur zwischen der Raumtemperatur und der Rückflußtemperatur des ausgewählten Lösungsmittels in der Weise umsetzt, daß man nach dem Abkühlen, der Extraktion und der eventuellen Reinigung die Verbindungen der allgemeinen Formel ($I_A$) erhält, wie sie in Anspruch 2 definiert sind, wobei es sich versteht, daß die in dieser Weise erhaltenen Verbindungen der Formel ($I_A$) gewünschtenfalls in ihre Isomeren aufgetrennt und/oder mit einer pharmazeutisch annehmbaren Säure in ihre Salze umgewandelt werden können.

**19.** Verfahren zur Herstellung der Verbindungen der allgemeinen Formel ($I_A$) nach Anspruch 2, worin A eine Methylenkette - $CH_2$ - darstellt, **dadurch gekennzeichnet**, daß man eine Verbindung der allgemeinen Formel (IV):

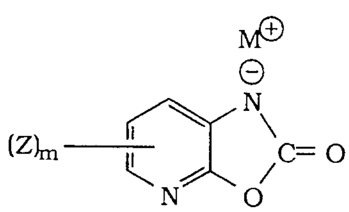

(IV)

in der Z und m die bezüglich der Verbindungen der allgemeinen Formel (I) angegebenen Bedeutungen besitzen und M ein Alkalimetall darstellt, in organischem Medium und bei einer Temperatur zwischen Raumtemperatur und der Rückflußtemperatur des ausgewählten Lösungsmittels (oder der Lösungsmittelmischung) mit Chlormethylphenylsulfid in der Weise umsetzt, daß man die Verbindungen der allgemeinen Formel (IX):

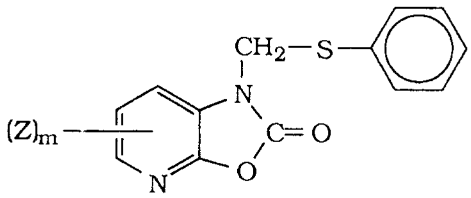

(IX)

in der Z und m die bezüglich der Verbindungen der allgemeinen Formel (I) angegebenen Bedeutungen besitzt, erhält, die man anschließend in organischem Medium mit Sulfurylchlorid umsetzt, so daß man nach der Reinigung die Chlorverbindung der allgemeinen Formel (X):

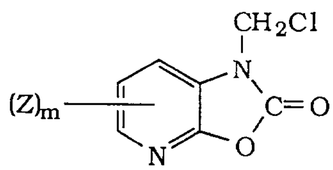

(X)

in der Z und m die bezüglich der Verbindungen der allgemeinen Formel (I) angegebenen Bedeutungen besitzen, erhält, welche man anschließend wie in Anspruch 18 angegeben mit den Verbindungen der allgemeinen Formel (VIII) in der Weise umsetzt, daß man die Verbindungen der allgemeinen Formel ($I_A$) erhält, wie sie in Anspruch 2 definiert ist, worin A eine Methylenkette - $CH_2$ - darstellt, wobei es sich versteht, daß die in dieser Weise erhaltenen Verbindungen der Formel ($I_A$) gewünschtenfalls in ihre Isomeren aufgetrennt und/oder mit einer pharmazeutisch annehmbaren Säure in ihre Salze überführt werden können.

**20.** Verfahren zur Herstellung der Verbindungen der allgemeinen Formel ($I_A$) nach Anspruch 2, worin A eine Methylenkette - $CH_2$ - darstellt, **dadurch gekennzeichnet**, daß man eine Verbindung der allgemeinen Formel (III), wie sie in Anspruch 17 definiert ist, in einem niedrigmolekularen aliphatischen Alkohol als Medium mit einem Amin der allgemeinen Formel (VIII), wie sie in Anspruch 18 definiert ist, in schwachem Überschuß und in Gegenwart eines Überschusses Formaldehyd bei einer Temperatur zwischen Raumtemperatur und der Rückflußtemperatur des Reaktionsmediums umsetzt, so daß man nach dem Abkühlen, dem Isolieren und der eventuellen Reinigung durch Säulenchromatographie über Siliciumdioxid die Verbindungen der allgemeinen Formel ($I_A$), wie sie in Anspruch 2 definiert ist, worin A eine Methylenkette - $CH_2$ - bedeutet, erhält, wobei es sich versteht, daß die in dieser Weise erhaltenen Verbindungen der Formel ($I_A$) gewünschtenfalls in ihre optischen Isomeren aufgetrennt und/oder mit einer pharmazeutisch annehmbaren Säure in ihre Salze umgewandelt werden können.

**21.** Verfahren zur Herstellung der Verbindungen der allgemeinen Formel ($I_B$) nach Anspruch 3, **dadurch gekennzeichnet**, daß man die Verbindungen der allgemeinen Formel ($I_A$) mit einem alkalischen Mittel, wie beispielsweise Natriumhydroxid, in wäßriger Lösung bei einer Temperatur zwischen Raumtemperatur und der Rückflußtemperatur des Reaktionsmediums umsetzt, so daß man nach der eventuellen Ansäuerung und/oder Neutralisation des Reaktionsmediums die Verbindungen der allgemeinen Formel ($I_B$) erhält:

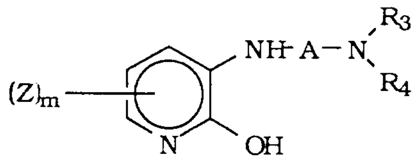

($I_B$)

einem Sonderfall der Derivate der allgemeinen Formel (I), worin $R_1$ = $R_2$ = H bedeuten und A, $R_3$, $R_4$, Z und m die bezüglich der Verbindungen der allgemeinen Formel (I) angegebenen Bedeutungen besitzen.

**22.** Verbindungen der allgemeinen Formel (VII)

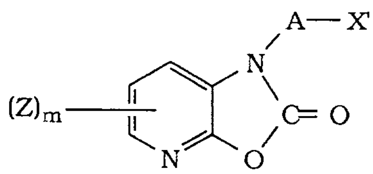

(VII)

worin X' ein Halogenatom, A eine geradkettige oder verzweigte Alkylgruppe mit 1 bis 6 Kohlenstoffatomen, Z ein Halogenatom, eine Niedrigalkyl- oder Niedrigalkoxygruppe mit 1 bis 6 Kohlenstoffatomen oder eine Trifluormethylgruppe und m eine ganze Zahl mit einem Wert zwischen 0 und 3 einschließlich bedeuten, als Synthesezwischenprodukte für die Herstellung der Verbindungen der allgemeinen Formel (I) nach Anspruch 1.

**23.** Pharmazeutische Zubereitungen enthaltend als Wirkstoffmindestens eine Verbindung nach Anspruch 1 in Kombination mit einem oder mehreren inerten, nichttoxischen, pharmazeutisch annehmbaren Trägermaterialien oder Bindemitteln.

**24.** Pharmazeutische Zubereitungen nach Anspruch 23 für die Behandlung von Schmerzen.

**Patentansprüche für folgenden Vertragsstaat : GR**

**1.** Verfahren zur Herstellung der neuen Verbindungen der allgemeinen Formel (I):

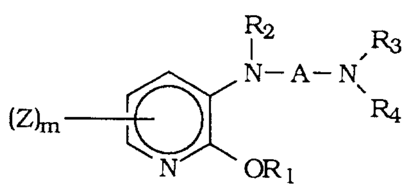

(I)

in der:

- $R_1$ und $R_2$ jeweils ein Wasserstoffatom oder gemeinsam mit dem Sauerstoff und dem Stickstoff, die sie tragen, eine Kette - O - CO - N -, was Oxazolo[5,4-b]pyridin-2-onen entspricht,
- Z ein Halogenatom, eine geradkettige oder verzweigte Niedrigalkylgruppe mit 1 bis 6 Kohlenstoffatomen, eine geradkettige oder verzweigte Niedrigalkoxygruppe mit 1 bis 6 Kohlenstoffatomen oder eine Trifluormethylgruppe,
- m eine ganze Zahl mit Werten von 0, 1, 2 oder 3,
- A eine geradkettige oder verzweigte Alkylgruppe mit 1 bis 6 Kohlenstoffatomen,
- $R_3$ und $R_4$, die gleichartig oder verschieden sein können, jeweils:
  - ein Wasserstoffatom,
  - eine geradkettige oder verzweigte Niedrigalkylgruppe mit 1 bis 6 Kohlenstoffatomen,
  - eine geradkettige oder verzweigte Niedrigalkenylgruppe mit 1 bis 6 Kohlenstoffatomen,

- eine gegebenenfalls substituierte Aryl- oder Heteroarylgruppe,
- eine gegebenenfalls substituierte Arylalkyl- oder Heteroarylalkylgruppe, deren Alkylkette 1 bis 3 Kohlenstoffatome aufweist,
- eine mono- oder bicyclische Cycloalkylgruppe mit 3 bis 10 Kohlenstoffatomen,

oder:

$R_3$ und $R_4$ zusammen mit dem Stickstoffatom, an das sie gebunden sind, ein mono- oder bicyclisches, gesättigtes oder ungesättigtes stickstoffhaltiges heterocyclisches System mit maximal 12 Atomen ohne die Wasserstoffatome, darunter bis zu drei Heteroatomen ausgewählt aus Stickstoff, Sauerstoff oder Schwefel und das gegebenenfalls durch eine oder mehrere:

- Hydroxylgruppen,
- Oxogruppen,
- geradkettige oder verzweigte Niedrigalkylgruppen mit 1 bis 6 Kohlenstoffatomen,
- gegebenenfalls substituierte Arylgruppen,
- gegebenenfalls substituierte Arylalkylgruppen oder gegebenenfalls substituierte Diarylalkylgruppen, deren Alkylkette 1 bis 3 Kohlenstoffatome aufweist,
- Gruppen - $CO\text{-}OR_5$ und
- Gruppen

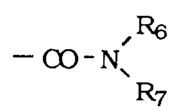

substituiert sein können,

- $R_5$:
  - ein Wasserstoffatom,
  - eine geradkettige oder verzweigte Niedrigalkylgruppe mit 1 bis 6 Kohlenstoffatomen,
  - eine gegebenenfalls substituierte Arylgruppe oder
  - eine gegebenenfalls substituierte Aralkylgruppe, deren Alkylkette 1 bis 3 Kohlenstoffatome aufweist,

  bedeuten, und

  $R_6$ und $R_7$, die gleichartig oder verschieden sein können, die gleichen Bedeutungen besitzen wie $R_5$,
- wobei unter einer Arylgruppe eine mono- oder bicyclische, ungesättigte oder aromatische Gruppe mit 5 bis 12 Kohlenstoffatomen zu verstehen ist,
- unter einer Heteroarylgruppe eine mono- oder bicyclische, ungesättigte oder aromatische Gruppe mit 5 bis 12 Atomen ohne die Wasserstoffatome zu verstehen ist, die in ihrem Kohlenstoffskelett eines, zwei oder drei Heteroatome ausgewählt aus Stickstoff, Sauerstoff oder Schwefel aufweist,
- und der mit den Bedeutungen Aryl, Arylalkyl, Diarylalkyl, Heteroaryl, Heteroarylalkyl verwendete Begriff "substituiert" bedeutet, daß der oder die Aryl- oder Heteroarylkerne durch eine oder mehrere geradkettige oder verzweigte Niedrigalkylgruppen mit 1 bis 6 Kohlenstoffatomen, geradkettige oder verzweigte Niedrigalkoxygruppen mit 1 bis 6 Kohlenstoffatomen, Hydroxylgruppen, Nitrogruppen, Halogenatome oder Trifluormethylgruppen substituiert sein können,

sowie von deren Isomeren, Epimeren, Diastereoisomeren und von deren Additionssalzen mit einer pharmazeutisch annehmbaren Säure und/oder, wenn $R_1$ und $R_2$ jeweils ein Wasserstoffatom bedeuten, von deren Additionssalzen mit einer pharmazeutisch annehmbaren Base,

**dadurch gekennzeichnet**, daß man

A) entweder ein 3-Amino-2-pyridinon der allgemeinen Formel (II):

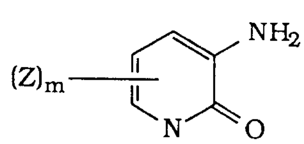
(II)

in der Z und m die bezüglich der Verbindungen der allgemeinen Formel (I) angegebenen Bedeutungen besitzen, in Lösung in einem aprotischen Lösungsmittel oder einer Mischung von aprotischen

Lösungsmitteln bei -78 °C (Temperatur der Mischung aus Aceton und Trockeneis) mit Bis-(trichlormethyl)-carbonat (Triphosgen) in Gegenwart eines basischen Amins, wie beispielsweise Triethylamin, in der Weise umsetzt, daß man ein [1H]-Oxazolo[5,4-b]pyridin-2-on der allgemeinen Formel (III):

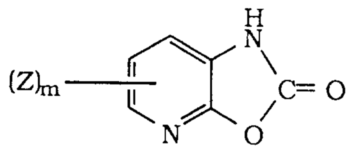 (III)

in der Z und m die bezüglich der Verbindungen der allgemeinen Formel (I) angegebenen Bedeutungen besitzen, erhält, welches man mit einem Alkalimetallalkoholat oder Alkalimetallhydrid in aprotischem organischem Medium in der Weise umsetzt, daß man die Verbindung der allgemeinen Formel (IV):

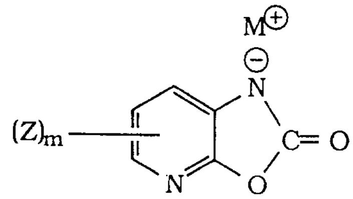 (IV)

in der Z und m die bezüglich der Verbindungen der allgemeinen Formel (I) angegebenen Bedeutungen besitzen und M ein Alkalimetall darstellt, erhält, welche man:

a) entweder in organischem Medium und bei einer Temperatur zwischen Raumtemperatur und der Rückflußtemperatur des ausgewählten Lösungsmittels (oder Lösungsmittelmischung) mit einer (vorzugsweise im Überschuß eingesetzten) elektrophilen Verbindung der allgemeinen Formel (V):

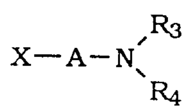 (V)

in der X ein Halogenatom darstellt und A, $R_3$ und $R_4$ die bezüglich der Verbindungen der allgemeinen Formel (I) angegebenen Bedeutungen besitzen, in der Weise umsetzt, daß man nach dem Abkühlen, der Extraktion und der eventuellen Reinigung die Verbindungen der allgemeinen Formel ($I_A$):

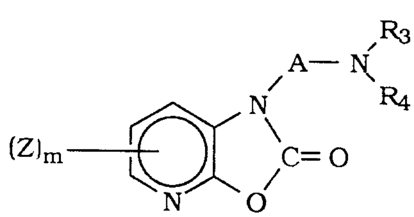 ($I_A$)

in der A, $R_3$, $R_4$, Z und m die bezüglich der Verbindungen der allgemeinen Formel (I) angegebenen Bedeutungen besitzen, erhält,

b) oder in organischem Medium bei einer Temperatur zwischen Raumtemperatur und der Rückflußtemperatur des ausgewählten Lösungsmittels (oder der Lösungsmittelmischung) mit einer (vorzugsweise im Überschuß eingesetzten) elektrophilen Verbindung der allgemeinen Formel (VI):

X - A - X'      (VI)

in der X und X', die gleichartig oder verschieden sein können, jeweils ein Halogenatom darstellen und A die bezüglich der Verbindungen der allgemeinen Formel (I) angegebenen Bedeutungen besitzt, in der Weise umsetzt, daß man die Halogenverbindung der allgemeinen Formel (VII):

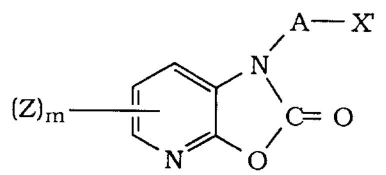

(VII)

in der X', A, Z und m die oben angegebenen Bedeutungen besitzen, erhält, die man schließlich mit einem (vorzugsweise im Überschuß eingesetzten) Amin der allgemeinen Formel (VIII):

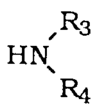

(VIII)

in der $R_3$ und $R_4$ die bezüglich der Verbindungen der allgemeinen Formel (I) angegebenen Bedeutungen besitzen, in organischem Medium, gegebenenfalls in Gegenwart eines basischen Mittels, wie Diisopropylamin, und bei einer Temperatur zwischen Raumtemperatur und der Rückflußtemperatur des ausgewählten Lösungsmittels in der Weise umsetzt, daß man nach dem Abkühlen, der Extraktion und der eventuellen Reinigung die Verbindungen der allgemeinen Formel ($I_A$), wie sie oben definiert worden ist, erhält,

c) oder im Fall der Verbindungen der allgemeinen Formel ($I_A$), worin A eine Methylenkette - $CH_2$ - darstellt, in organischem Medium und bei einer Temperatur zwischen Raumtemperatur und der Rückflußtemperatur des ausgewählten Lösungsmittels (oder der Lösungsmittelmischung) mit Chlormethylphenylsulfid in der Weise umsetzt, daß man die Verbindungen der allgemeinen Formel (IX):

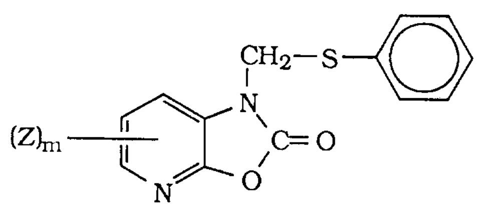

(IX)

worin Z und m die bezüglich der Verbindungen der allgemeinen Formel (I) angegebenen Bedeutungen besitzt, erhält, welche man anschließend in organischem Medium mit Sulfurylchlorid umsetzt, so daß man nach der Reinigung die Chlorverbindung der allgemeinen Formel (X):

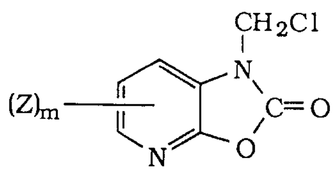

(X)

in der Z und m die bezüglich der Verbindungen der allgemeinen Formel (I) angegebenen Bedeutungen besitzen, erhält, welche man anschließend wie im Fall der Verbindungen der

allgemeinen Formel (VII) mit den Verbindungen der allgemeinen Formel (VIII) in der Weise umsetzt, daß man die Verbindungen der allgemeinen Formel ($I_A$) erhält, wie sie oben definiert worden ist und worin A eine Methylenkette - $CH_2$ - darstellt, erhält,

B) oder für den Fall der Verbindungen der allgemeinen Formel ($I_A$), worin A eine Methylenkette - $CH_2$ - darstellt, eine Verbindung der allgemeinen Formel (III) in einem niedrigmolekularen aliphatischen Alkohol als Medium mit einem in schwachem Überschuß eingesetzten Amin der allgemeinen Formel (VIII) und einem Überschuß von Formaldehyd bei einer Temperatur zwischen Raumtempertur und der Rückflußtemperatur des Reaktionsmediums umsetzt, wobei die erhaltenen Verbindungen der allgemeinen Formel ($I_A$) gegebenenfalls nach der Isolierung säulenchromatographisch über Siliciumdioxid gereinigt werden können,

C) oder im Fall der Verbindungen der allgemeinen Formel (I), worin $R_1$ und $R_2$ jeweils ein Wasserstoffatom darstellen, die Verbindungen ($I_A$) in wäßriger Lösung bei einer Temperatur zwischen Raumtemperatur und der Rückflußtemperatur des Reaktionsmediums mit einem alkalischen Mittel, wie beispielsweise Natriumhydroxid, behandelt, so daß man nach der eventuellen Ansäuerung und/oder Neutralisation des Reaktionsmediums die Verbindungen der allgemeinen Formel ($I_B$):

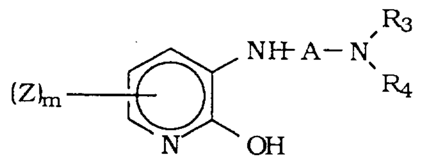

($I_B$)

einem Sonderfall der Verbindungen der allgemeinen Formel (I), worin $R_1$ = $R_2$ = H darstellen und A, $R_3$, $R_4$, Z und m die bezüglich der Derivate der allgemeinen Formel (I) angegebenen Bedeutungen besitzen, erhält,

wobei es sich versteht, daß die in dieser Weise erhaltenen Verbindungen der Formel ($I_A$) und ($I_B$) gewünschtenfalls in ihre Isomeren aufgetrennt und/oder mit einer pharmazeutisch annehmbaren Säure oder Base in ihre Salze überführt werden können.

**2.** Verfahren nach Anspruch 1 zur Herstellung von Verbindungen, worin $R_1$ und $R_2$ zusammen mit dem Sauerstoff und dem Stickstoff, die sie tragen, eine Kette - O - CO - N - bilden, der allgemeinen Formel ($I_A$):

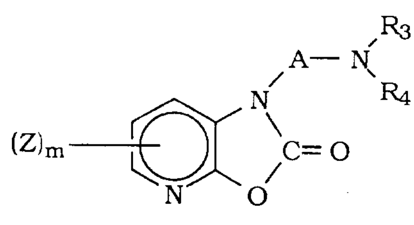

($I_A$)

worin Z, m, A, $R_3$ und $R_4$ die in Anspruch 1 angegebenen Bedeutungen besitzen, sowie von deren Isomeren, Epimeren, Diastereoisomeren und von ihren Additionssalzen mit einer pharmazeutisch annehmbaren Säure.

**3.** Verfahren nach Anspruch 1 zur Herstellung der Verbindungen, worin $R_1$ und $R_2$ jeweils ein Wasserstoffatom bedeuten, der allgemeinen Formel ($I_B$):

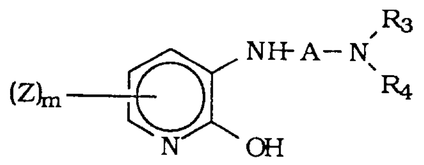

($I_B$)

worin Z, m, A, $R_3$ und $R_4$ die in Anspruch 1 angegebenen Bedeutungen besitzen, sowie von deren Isomeren, Epimeren, Diastereoisomeren und von deren Additionssalzen mit einer pharmazeutisch annehmbaren Säure oder Base.

**4.** Vefahren nach Anspruch 1 zur Herstellung der Verbindungen der allgemeinen Formel (I), worin m = 0 und A eine geradkettige Alkylgruppe mit 2 bis 6 Kohlenstoffatomen bedeuten.

**5.** Verfahren nach Anspruch 1 zur Herstellung von 1-[2-(4-Phenyl-piperazin-1-yl)-ethyl]-[1H]-oxazolo[5,4-b]-pyridin-2-on der Formel

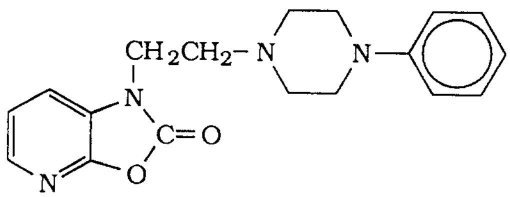

sowie von deren Additionssalzen mit einer pharmazeutisch annehmbaren Säure.

**6.** Verfahren nach Anspruch 1 zur Herstellung von 1-[2-(4-(3-Trifluormethylphenyl)-piperazin-1-yl)-ethyl]-[1H]-oxazolo[5,4-b]pyridin-2-on der Formel

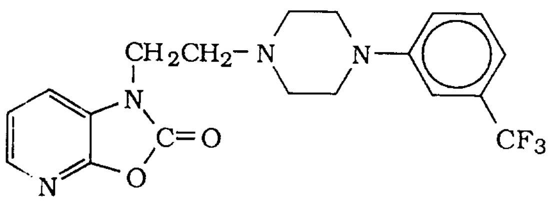

sowie von deren Additionssalzen mit einer pharmazeutisch annehmbaren Säure.

**7.** Verfahren nach Anspruch 1 zur Herstellung von 1-[2-Morpholino-ethyl]-[1H]-oxazolo[5,4-b]pyridin-2-on der Formel

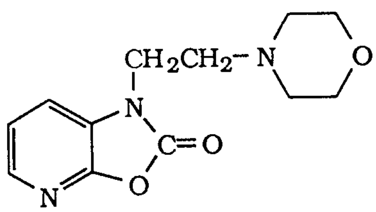

sowie von deren Additionssalzen mit einer pharmazeutisch annehmbaren Säure.

**8.** Verfahren nach Anspruch 1 zur Herstellung von 1-[3-Morpholino-prop-1-yl]-[1H]-oxazolo[5,4-b]pyridin-2-on der Formel

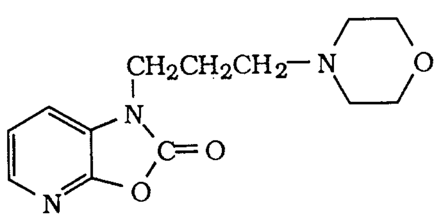

sowie von deren Additionssalzen mit einer pharmazeutisch annehmbaren Säure.

**9.** Verfahren nach Anspruch 1 zur Herstellung von 1-[4-Morpholino-but-1-yl]-[1H]-oxazolo[5,4-b]pyridin-2-on der Formel

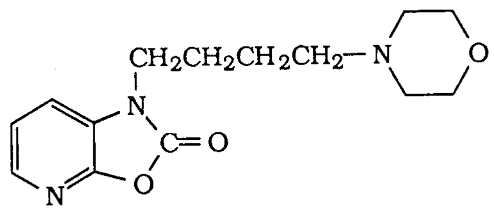

sowie von deren Additionssalzen mit einer pharmazeutisch annehmbaren Säure.

**10.** Verfahren nach Anspruch 1 zur Herstellung von 1-[3-(4-Phenyl-piperazin-1-yl)-prop-1-yl]-[1H]-oxazolo-[5,4-b]pyridin-2-on der Formel

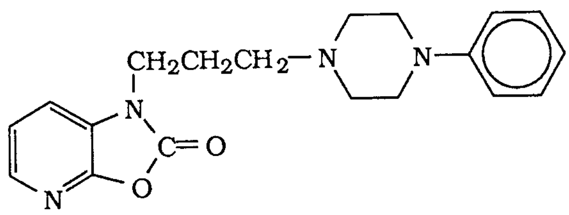

sowie von deren Additionssalzen mit einer pharmazeutisch annehmbaren Säure.

**11.** Verfahren nach Anspruch 1 zur Herstellung von 1-[4-(4-Phenyl-piperazin-1-yl)-but-1-yl]-[1H]-oxazolo-[5,4-b]pyridin-2-on der Formel

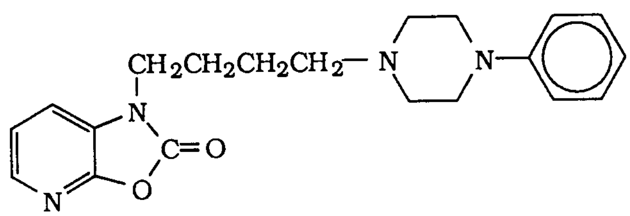

sowie von deren Additionssalzen mit einer pharmazeutisch annehmbaren Säure.

**12.** Verfahren nach Anspruch 1 zur Herstellung von 1-[2-(4-(4-Fluorphenyl)-piperazin-1-yl)-ethyl]-[1H]-oxazolo[5,4-b]pyridin-2-on der Formel

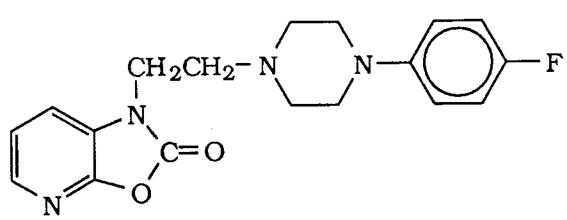

sowie von deren Additionssalzen mit einer pharmazeutisch annehmbaren Säure.

**13.** Verfahren nach Anspruch 1 zur Herstellung von 1-[2-(Pyrrolidin-1-yl)-ethyl]-[1H]-oxazolo[5,4-b]pyridin-2-on der Formel

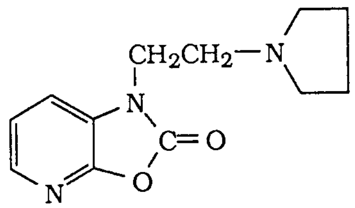

sowie von deren Additionssalzen mit einer pharmazeutisch annehmbaren Säure.

**14.** Verfahren nach Anspruch 1 zur Herstellung von 1-[2-(4-Phenyl-piperidin-1-yl)-ethyl]-[1H]-oxazolo[5,4-b]-pyridin-2-on der Formel

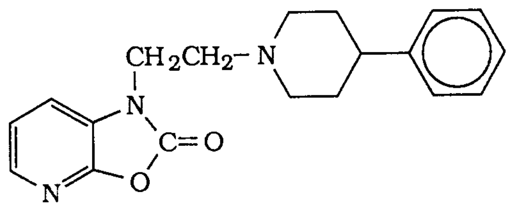

sowie von deren Additionssalzen mit einer pharmazeutisch annehmbaren Säure.

**15.** Verfahren nach Anspruch 1 zur Herstellung von 1-[2-(4-N,N-Diethylaminocarbonyl)-piperazin-1-yl)-ethyl]-[1H]-oxazolo[5,4-b]pyridin-2-on der Formel

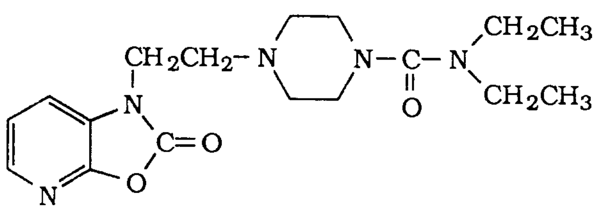

sowie von deren Additionssalzen mit einer pharmazeutisch annehmbaren Säure oder Base.

**16.** Verfahren nach Anspruch 1 zur Herstellung von 3-[2-(4-Phenyl-piperazin-1-yl)-ethylamino]-2-hydroxy-pyridin der Formel

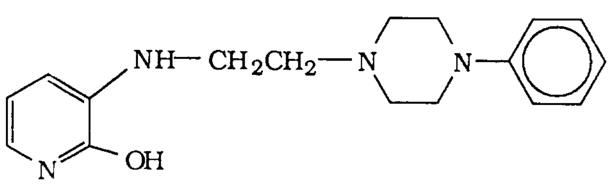

sowie von deren Additionssalzen mit einer pharmazeutisch annehmbaren Säure oder Base.

**17.** Verfahren zur Herstellung von pharmazeutischen Zubereitungen enthaltend als Wirkstoff mindestens eine Verbindung hergestellt nach Anspruch 1 in Kombination mit einem oder mehreren inerten, nichttoxischen, pharmazeutisch annehmbaren Trägermaterialien oder Bindemitteln.

**18.** Verfahren zur Herstellung der pharmazeutischen Zubereitungen nach Anspruch 17, die zur Behandlung von Schmerzen geeignet sind.

**Patentansprüche für folgenden Vertragsstaat : ES**

1. Verfahren zur Herstellung der neuen Verbindungen der allgemeinen Formel (I):

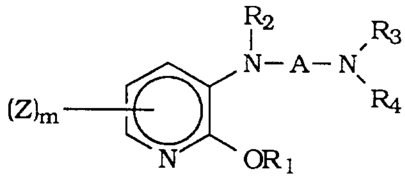

(I)

in der:

- $R_1$ und $R_2$ jeweils ein Wasserstoffatom oder gemeinsam mit dem Sauerstoff und dem Stickstoff, die sie tragen, eine Kette - O - CO - N -, was Oxazolo[5,4-b]pyridin-2-onen entspricht,
- Z ein Halogenatom, eine geradkettige oder verzweigte Niedrigalkylgruppe mit 1 bis 6 Kohlenstoffatomen, eine geradkettige oder verzweigte Niedrigalkoxygruppe mit 1 bis 6 Kohlenstoffatomen oder eine Trifluormethylgruppe,
- m eine ganze Zahl mit Werten von 0, 1, 2 oder 3,
- A eine geradkettige oder verzweigte Alkylgruppe mit 1 bis 6 Kohlenstoffatomen,
- $R_3$ und $R_4$, die gleichartig oder verschieden sein können, jeweils:
  - ein Wasserstoffatom,
  - eine geradkettige oder verzweigte Niedrigalkylgruppe mit 1 bis 6 Kohlenstoffatomen,
  - eine geradkettige oder verzweigte Niedrigalkenylgruppe mit 1 bis 6 Kohlenstoffatomen,
  - eine gegebenenfalls substituierte Aryl- oder Heteroarylgruppe,
  - eine gegebenenfalls substituierte Arylalkyl- oder Heteroarylalkylgruppe, deren Alkylkette 1 bis 3 Kohlenstoffatome aufweist,
  - eine mono- oder bicyclische Cycloalkylgruppe mit 3 bis 10 Kohlenstoffatomen,

  oder:

  $R_3$ und $R_4$ zusammen mit dem Stickstoffatom, an das sie gebunden sind, ein mono- oder bicyclisches, gesättigtes oder ungesättigtes stickstoffhaitiges heterocyclisches System mit maximal 12 Atomen ohne die Wasserstoffatome, darunter bis zu drei Heteroatomen ausgewählt aus Stickstoff, Sauerstoff oder Schwefel und das gegebenenfalls durch eine oder mehrere:
  - Hydroxylgruppen,
  - Oxogruppen,
  - geradkettige oder verzweigte Niedrigalkylgruppen mit 1 bis 6 Kohlenstoffatomen,
  - gegebenenfalls substituierte Arylgruppen,
  - gegebenenfalls substituierte Arylalkylgruppen oder gegebenenfalls substituierte Diarylalkylgruppen, deren Alkylkette 1 bis 3 Kohlenstoffatome aufweist,
  - Gruppen - CO-$OR_5$ und
  - Gruppen

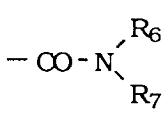

  substituiert sein können,
- $R_5$:
  - ein Wasserstoffatom,
  - eine geradkettige oder verzweigte Niedrigalkylgruppe mit 1 bis 6 Kohlenstoffatomen,
  - eine gegebenenfalls substituierte Arylgruppe oder
  - eine gegebenenfalls substituierte Aralkylgruppe, deren Alkylkette 1 bis 3 Kohlenstoffatome aufweist,

  bedeuten, und

  $R_6$ und $R_7$, die gleichartig oder verschieden sein können, die gleichen Bedeutungen besitzen wie $R_5$,

- wobei unter einer Arylgruppe eine mono- oder bicyclische, ungesättigte oder aromatische Gruppe mit 5 bis 12 Kohlenstoffatomen zu verstehen ist,
- unter einer Heteroarylgruppe eine mono- oder bicyclische, ungesättigte oder aromatische Gruppe mit 5 bis 12 Atomen ohne die Wasserstoffatome zu verstehen ist, die in ihrem Kohlenstoffskelett eines, zwei oder drei Heteroatome ausgewählt aus Stickstoff, Sauerstoff oder Schwefel aufweist,
- und der mit den Bedeutungen Aryl, Arylalkyl, Diarylalkyl, Heteroaryl, Heteroarylalkyl verwendete Begriff "substituiert" bedeutet, daß der oder die Aryl- oder Heteroarylkerne durch eine oder mehrere geradkettige oder verzweigte Niedrigalkylgruppen mit 1 bis 6 Kohlenstoffatomen, geradkettige oder verzweigte Niedrigalkoxygruppen mit 1 bis 6 Kohlenstoffatomen, Hydroxylgruppen, Nitrogruppen, Halogenatome oder Trifluormethylgruppen substituiert sein können,

sowie von deren Isomeren, Epimeren, Diastereoisomeren und von deren Additionssalzen mit einer pharmazeutisch annehmbaren Säure und/oder, wenn $R_1$ und $R_2$ jeweils ein Wasserstoffatom bedeuten, von deren Additionssalzen mit einer pharmazeutisch annehmbaren Base,

**dadurch gekennzeichnet**, daß man

A) entweder ein 3-Amino-2-pyridinon der allgemeinen Formel (II):

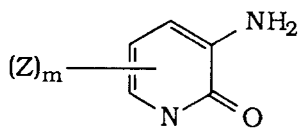

(II)

in der Z und m die bezüglich der Verbindungen der allgemeinen Formel (I) angegebenen Bedeutungen besitzen, in Lösung in einem aprotischen Lösungsmittel oder einer Mischung von aprotischen Lösungsmitteln bei -78 °C (Temperatur der Mischung aus Aceton und Trockeneis) mit Bis-(trichlormethyl)-carbonat (Triphosgen) in Gegenwart eines basischen Amins, wie beispielsweise Triethylamin, in der Weise umsetzt, daß man ein [1H]-Oxazolo[5,4-b]pyridin-2-on der allgemeinen Formel (III):

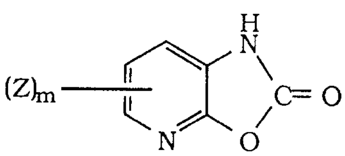

(III)

in der Z und m die bezuglich der Verbindungen der allgemeinen Formel (I) angegebenen Bedeutungen besitzen, erhält, welches man mit einem Alkalimetallalkoholat oder Alkalimetallhydrid in aprotischem organischem Medium in der Weise umsetzt, daß man die Verbindung der allgemeinen Formel (IV):

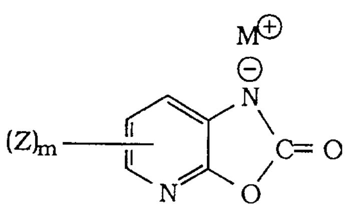

(IV)

in der Z und m die bezüglich der Verbindungen der allgemeinen Formel (I) angegebenen Bedeutungen besitzen und M ein Alkalimetall darstellt, erhält, welche man:

a) entweder in organischem Medium und bei einer Temperatur zwischen Raumtemperatur und der Rückflußtemperatur des ausgewählten Lösungsmittels (oder Lösungsmittelmischung) mit einer (vorzugsweise im Überschuß eingesetzten) elektrophilen Verbindung der allgemeinen Formel (V):

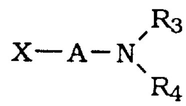

(V)

in der X ein Halogenatom darstellt und A, $R_3$ und $R_4$ die bezüglich der Verbindungen der allgemeinen Formel (I) angegebenen Bedeutungen besitzen, in der Weise umsetzt, daß man nach dem Abkühlen, der Extraktion und der eventuellen Reinigung die Verbindungen der allgemeinen Formel ($I_A$):

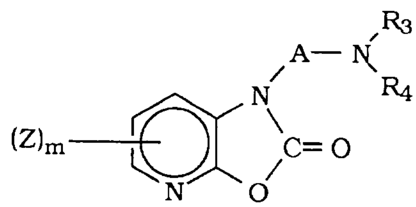

($I_A$)

in der A, $R_3$, $R_4$, Z und m die bezüglich der Verbindungen der allgemeinen Formel (I) angegebenen Bedeutungen besitzen, erhält,

b) oder in organischem Medium bei einer Temperatur zwischen Raumtemperatur und der Rückflußtemperatur des ausgewählten Lösungsmittels (oder der Lösungsmittelmischung) mit einer (vorzugsweise im Überschuß eingesetzten) elektrophilen Verbindung der allgemeinen Formel (VI):

X - A - X' (VI)

in der X und X', die gleichartig oder verschieden sein können, jeweils ein Halogenatom darstellen und A die bezüglich der Verbindungen der allgemeinen Formel (I) angegebenen Bedeutungen besitzt, in der Weise umsetzt, daß man die Halogenverbindung der allgemeinen Formel (VII):

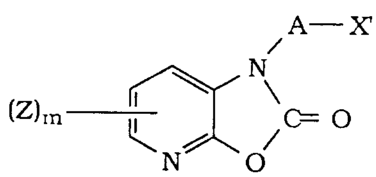

(VII)

in der X', A, Z und m die oben angegebenen Bedeutungen besitzen, erhält, die man schließlich mit einem (vorzugsweise im Überschuß eingesetzten) Amin der allgemeinen Formel (VIII):

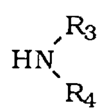

(VIII)

in der $R_3$ und $R_4$ die bezüglich der Verbindungen der allgemeinen Formel (I) angegebenen Bedeutungen besitzen, in organischem Medium, gegebenenfalls in Gegenwart eines basischen Mittels, wie Diisopropylamin, und bei einer Temperatur zwischen Raumtemperatur und der Rückflußtemperatur des ausgewählten Lösungsmittels in der Weise umsetzt, daß man nach dem Abkühlen, der Extraktion und der eventuellen Reinigung die Verbindungen der allgemeinen Formel ($I_A$), wie sie oben definiert worden ist, erhält,

c) oder im Fall der Verbindungen der allgemeinen Formel ($I_A$), worin A eine Methylenkette - $CH_2$ - darstellt, in organischem Medium und bei einer Temperatur zwischen Raumtemperatur und der Rückflußtemperatur des ausgewählten Lösungsmittels (oder der Lösungsmittelmischung) mit Chlormethylphenylsulfid in der Weise umsetzt, daß man die Verbindungen der allgemeinen

Formel (IX):

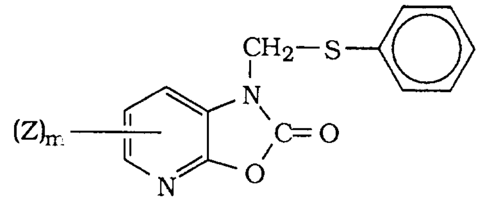

(IX)

worin Z und m die bezüglich der Verbindungen der allgemeinen Formel (I) angegebenen Bedeutungen besitzt, erhält, welche man anschließend in organischem Medium mit Sulfurylchlorid umsetzt, so daß man nach der Reinigung die Chlorverbindung der allgemeinen Formel (X):

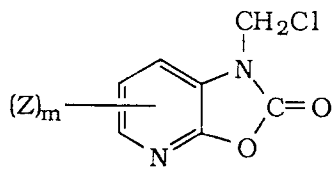

(X)

in der Z und m die bezüglich der Verbindungen der allgemeinen Formel (I) angegebenen Bedeutungen besitzen, erhält, welche man anschließend wie im Fall der Verbindungen der allgemeinen Formel (VII) mit den Verbindungen der allgemeinen Formel (VIII) in der Weise umsetzt, daß man die Verbindungen der allgemeinen Formel ($I_A$) erhält, wie sie oben definiert worden ist und worin A eine Methylenkette - $CH_2$ - darstellt, erhält,

B) oder für den Fall der Verbindungen der allgemeinen Formel ($I_A$), worin A eine Methylenkette - $CH_2$ - darstellt, eine Verbindung der allgemeinen Formel (III) in einem niedrigmolekularen aliphatischen Alkohol als Medium mit einem in schwachem Überschuß eingesetzten Amin der allgemeinen Formel (VIII) und einem Überschuß von Formaldehyd bei einer Temperatur zwischen Raumtemperatur und der Rückflußtemperatur des Reaktionsmediums umsetzt, wobei die erhaltenen Verbindungen der allgemeinen Formel ($I_A$) gegebenenfalls nach der Isolierung säulenchromatographisch über Siliciumdioxid gereinigt werden können,

C) oder im Fall der Verbindungen der allgemeinen Formel (I), worin $R_1$ und $R_2$ jeweils ein Wasserstoffatom darstellen, die Verbindungen ($I_A$) in wäßriger Lösung bei einer Temperatur zwischen Raumtemperatur und der Rückflußtemperatur des Reaktionsmediums mit einem alkalischen Mittel, wie beispielsweise Natriumhydroxid, behandelt, so daß man nach der eventuellen Ansäuerung und/oder Neutralisation des Reaktionsmediums die Verbindungen der allgemeinen Formel ($I_B$):

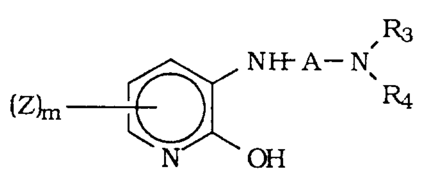

($I_B$)

einem Sonderfall der Verbindungen der allgemeinen Formel (I), worin $R_1$ = $R_2$ = H darstellen und A, $R_3$, $R_4$, Z und m die bezüglich der Derivate der allgemeinen Formel (I) angegebenen Bedeutungen besitzen, erhält, wobei es sich versteht, daß die in dieser Weise erhaltenen Verbindungen der Formel ($I_A$) und ($I_B$) gewünschtenfalls in ihre Isomeren aufgetrennt und/oder mit einer pharmazeutisch annehmbaren Säure oder Base in ihre Salze überführt werden können.

**2.** Verfahren nach Anspruch 1 zur Herstellung von Verbindungen, worin $R_1$ und $R_2$ zusammen mit dem Sauerstoff und dem Stickstoff, die sie tragen, eine Kette - O - CO - N - bilden, der allgemeinen Formel ($I_A$):

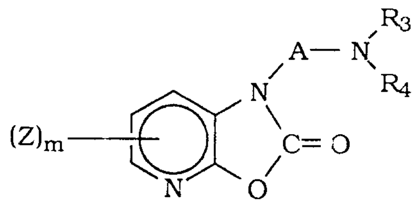

($I_A$)

worin Z, m, A, $R_3$ und $R_4$ die in Anspruch 1 angegebenen Bedeutungen besitzen, sowie von deren Isomeren, Epimeren, Diastereoisomeren und von ihren Additionssalzen mit einer pharmazeutisch annehmbaren Säure.

3. Verfahren nach Anspruch 1 zur Herstellung der Verbindungen, worin $R_1$ und $R_2$ jeweils ein Wasserstoffatom bedeuten, der allgemeinen Formel ($I_B$):

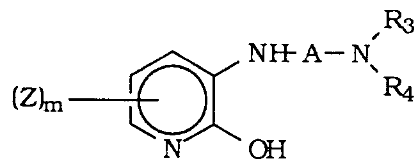

($I_B$)

worin Z, m, A, $R_3$ und $R_4$ die in Anspruch 1 angegebenen Bedeutungen besitzen, sowie von deren Isomeren, Epimeren, Diastereoisomeren und von deren Additionssalzen mit einer pharmazeutisch annehmbaren Säure oder Base.

4. Vefahren nach Anspruch 1 zur Herstellung der Verbindungen der allgemeinen Formel (I), worin m = 0 und A eine geradkettige Alkylgruppe mit 2 bis 6 Kohlenstoffatomen bedeuten.

5. Verfahren nach Anspruch 1 zur Herstellung von 1-[2-(4-Phenyl-piperazin-1-yl)-ethyl]-[1H]-oxazolo[5,4-b]-pyridin-2-on der Formel

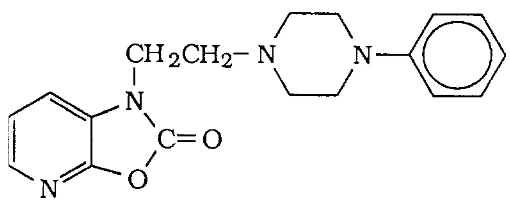

sowie von deren Additionssalzen mit einer pharmazeutisch annehmbaren Säure.

6. Verfahren nach Anspruch 1 zur Herstellung von 1-[2-(4-(3-Trifluormethylphenyl)-piperazin-1-yl)-ethyl]-[1H]-oxazolo[5,4-b]pyridin-2-on der Formel

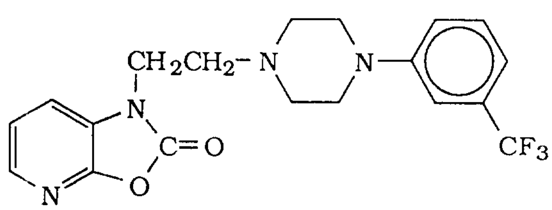

sowie von deren Additionssalzen mit einer pharmazeutisch annehmbaren Säure.

7. Verfahren nach Anspruch 1 zur Herstellung von 1-[2-Morpholino-ethyl]-[1H]-oxazolo[5,4-b]pyridin-2-on der Formel

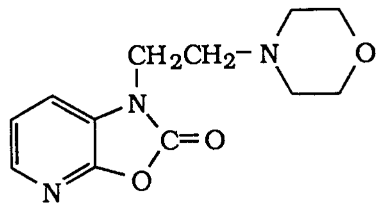

sowie von deren Additionssalzen mit einer pharmazeutisch annehmbaren Säure.

8. Verfahren nach Anspruch 1 zur Herstellung von 1-[3-Morpholino-prop-1-yl]-[1H]-oxazolo[5,4-b]pyridin-2-on der Formel

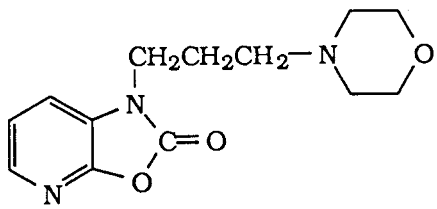

sowie von deren Additionssalzen mit einer pharmazeutisch annehmbaren Säure.

9. Verfahren nach Anspruch 1 zur Herstellung von 1-[4-Morpholino-but-1-yl]-[1H]-oxazolo[5,4-b]pyridin-2-on der Formel

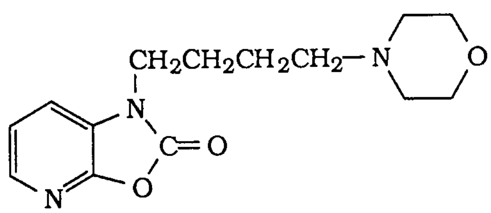

sowie von deren Additionssalzen mit einer pharmazeutisch annehmbaren Säure.

10. Verfahren nach Anspruch 1 zur Herstellung von 1-[3-(4-Phenyl-piperazin-1-yl)-prop-1-yl]-[1H]-oxazolo-[5,4-b]pyridin-2-on der Formel

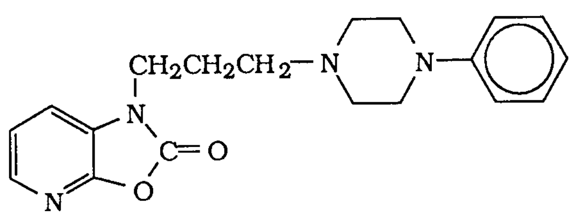

sowie von deren Additionssalzen mit einer pharmazeutisch annehmbaren Säure.

11. Verfahren nach Anspruch 1 zur Herstellung von 1-[4-(4-Phenyl-piperazin-1-yl)-but-1-yl]-[1H]-oxazolo-[5,4-b]pyridin-2-on der Formel

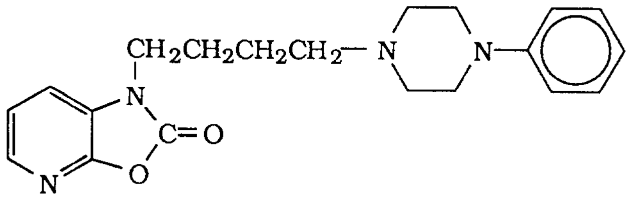

sowie von deren Additionssalzen mit einer pharmazeutisch annehmbaren Säure.

**12.** Verfahren nach Anspruch 1 zur Herstellung von 1-[2-(4-(4-Fluorphenyl)-piperazin-1-yl)-ethyl]-[1H]-oxazolo[5,4-b]pyridin-2-on der Formel

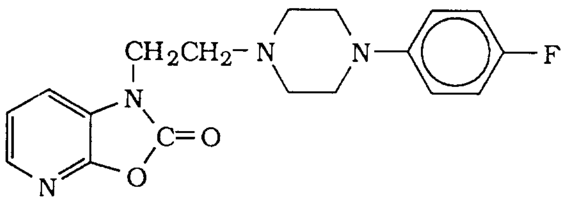

sowie von deren Additionssalzen mit einer pharmazeutisch annehmbaren Säure.

**13.** Verfahren nach Anspruch 1 zur Herstellung von 1-[2-(Pyrrolidin-1-yl)-ethyl)-[1H]-oxazolo[5,4-b]pyridin-2-on der Formel

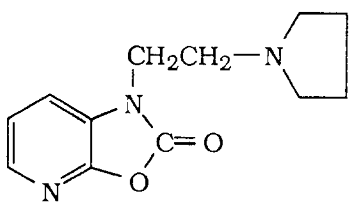

sowie von deren Additionssalzen mit einer pharmazeutisch annehmbaren Säure.

**14.** Verfahren nach Anspruch 1 zur Herstellung von 1-[2-(4-Phenyl-piperidin-1-yl)-ethyl[-[1H]-oxazolo[5,4-b]-pyridin-2-on der Formel

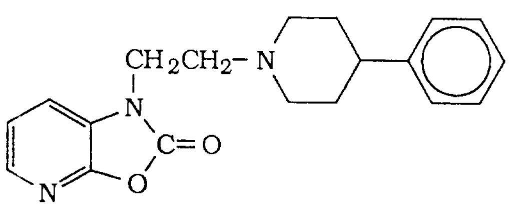

sowie von deren Additionssalzen mit einer pharmazeutisch annehmbaren Säure.

**15.** Verfahren nach Anspruch 1 zur Herstellung von 1-[2-(4-N,N-Diethylaminocarbonyl)-piperazin-1-yl)-ethyl]-[1H]-oxazolo[5,4-b]pyridin-2-on der Formel

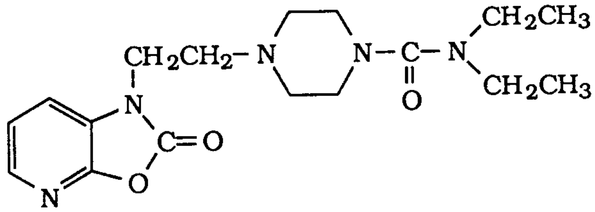

sowie von deren Additionssalzen mit einer pharmazeutisch annehmbaren Säure oder Base.

**16.** Verfahren nach Anspruch 1 zur Herstellung von 3-[2-(4-Phenyl-piperazin-1-yl)-ethylamino]-2-hydroxy-pyridin der Formel

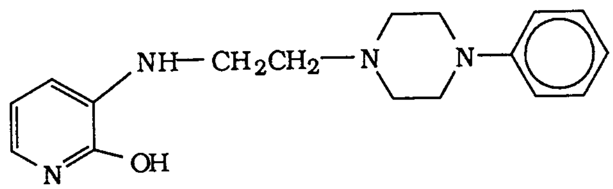

sowie von deren Additionssalzen mit einer pharmazeutisch annehmbaren Säure oder Base.

**17.** Verfahren zur Herstellung von pharmazeutischen Zubereitungen enthaltend als Wirkstoff mindestens eine Verbindung hergestellt nach Anspruch 1 in Kombination mit einem oder mehreren inerten, nichttoxischen, pharmazeutisch annehmbaren Trägermateriallen oder Bindemitteln.

**18.** Verfahren zur Herstellung der pharmazeutischen Zubereitungen nach Anspruch 17, die zur Behandlung von Schmerzen geeignet sind.